# EUROPEAN PATENT SPECIFICATION

(11) **EP 4 547 789 B1**
(45) Date of publication and mention of the grant of the patent: **17.06.2026**
(21) Application number: 23744193.6
(22) Date of filing: 30.06.2023
(51) Int. Cl.: C10G 3/00, C10G 45/62, C10G 47/02, C10G 55/06, C10G 47/36, C10G 65/12, C10G 45/72, B01J 8/00, C10G 2/00, C10G 45/64

(54) **A PROCESS FOR PRODUCING A LIQUID TRANSPORTATION FUEL COMPONENT**
VERFAHREN ZUR HERSTELLUNG EINER KOMPONENTE FÜR FLÜSSIGEN TRANSPORTKRAFTSTOFF
PROCÉDÉ DE PRODUCTION D'UN COMPOSANT DE CARBURANT DE TRANSPORT LIQUIDE

(30) Priority: 30.06.2022 FI 20225609; 02.12.2022 FI 20226071
(43) Date of publication of application: 07.05.2025
(73) Proprietor: Neste Oyj, 02150 Espoo (FI)
(72) Inventor: LAMMINPÄÄ, Kaisa, 06101 Porvoo (FI); AALTO, Pekka, 06101 Porvoo (FI); SIPPOLA, Väinö, 06101 Porvoo (FI); SUNTIO, Ville, 06101 Porvoo (FI); TOPPINEN, Sami, 06101 Porvoo (FI); KHAN, Yaseen, 06101 Porvoo (FI)
(74) Representative: Espatent Oy
(86) International application number: PCT/FI2023/050417
(87) International publication number: WO 2024/003467

(56) References cited:
- US-A1- 2002 116 079
- US-A1- 2010 000 908
- US-B2- 8 039 682
- US-B2- 8 324 438

## Description

### TECHNICAL FIELD

The present disclosure generally relates to processes for producing renewable fuel components. The disclosure relates particularly, though not exclusively, to processes for producing at least one or more liquid transportation fuel component(s), preferably at least an aviation fuel component. Also, a computer program usable for performing the processes is disclosed.

### BACKGROUND

This section illustrates useful background information without admission of any technique described herein representative of the state of the art.

There is an ongoing need to reduce greenhouse gas emissions and/or carbon footprint in transportation, especially aviation. Accordingly, interest towards renewable aviation fuels and aviation fuel components is and has been growing.

Catalytic processes for producing aviation fuel components from renewable raw materials have been proposed. However, the yield of aviation fuel components (compared to other fuel components) has been relatively low in said processes due to unavoidable catalyst deactivation. Also, there is a need to reduce quality decrease of renewable aviation fuel components towards end of run. Additionally, there is an increasing demand to produce fuel components from a wider range of renewable raw materials, including raw material qualities having elevated content of impurities, without compromising the catalyst lifetime. US2010/000908 relates to the manufacture of hydrocarbon components suitable as aviation fuels or jet fuels and as blending stocks for aviation fuels.

### SUMMARY

It is an aim to solve or alleviate at least some of the problems related to prior art. An aim is to improve quality of aviation fuel components obtainable from renewable sources. A further aim is to enable increasing the yield of an aviation fuel component. Another aim is to prolong run-time or hydrotreatment catalyst lifetime in processes for producing renewable liquid transportation fuel components. Yet further, an aim is to enable use of a wider range of renewable raw materials in processes for producing renewable liquid transportation fuel components, particularly aviation fuel component.

The appended claims define the scope of protection. Any examples and technical descriptions of apparatuses, products, systems, and/or methods in the description and/or drawings not covered by the claims are presented as examples useful for understanding the invention.

According to a first aspect of the invention, there is provided a process for producing at least one liquid transportation fuel component, the process comprising:
providing a hydrocarbon feed comprising nitrogen impurities;
   i) subjecting a reactor A feed comprising the hydrocarbon feed to hydroprocessing in a reactor A in the presence of a hydrotreatment catalyst A to obtain a hydroprocessing effluent A, and separating from the hydroprocessing effluent A at least compounds gaseous at NTP to obtain a degassed hydroprocessing effluent A;
   ii) subjecting a reactor B feed comprising the degassed hydroprocessing effluent A to hydroprocessing in a reactor B in the presence of a hydrotreatment catalyst B to obtain a hydroprocessing effluent B;
   iii) feeding the hydroprocessing effluent B, optionally after separating from the hydroprocessing effluent B at least compounds gaseous at NTP, to fractionation, and recovering from the fractionation at least one or more liquid transportation fuel component(s); and
monitoring parameters indicative of deactivation of the hydrotreatment catalyst A to receive values;
comparing the received values with predetermined values; and
when the received values reach the predetermined values, switching from i), ii), iii) to:
   I) subjecting a reactor B feed comprising the hydrocarbon feed to hydroprocessing in the reactor B in the presence of the hydrotreatment catalyst B to obtain a hydroprocessing effluent B, and separating from the hydroprocessing effluent B at least compounds gaseous at NTP to obtain a degassed hydroprocessing effluent B;
   II) subjecting a reactor A feed comprising the degassed hydroprocessing effluent B to hydroprocessing in the reactor A in the presence of the hydrotreatment catalyst A to obtain a hydroprocessing effluent A;
   III) feeding the hydroprocessing effluent A, optionally after separating from the hydroprocessing effluent A at least compounds gaseous at NTP, to fractionation, and recovering from the fractionation at least one or more liquid transportation fuel component(s).

According to a second aspect of the invention, there is provided a process for producing at least one liquid transportation fuel component, the process comprising:
providing a hydrocarbon feed comprising nitrogen impurities;
   i) subjecting a reactor A feed comprising the hydrocarbon feed to hydroprocessing in a reactor A in the presence of a hydrotreatment catalyst A to obtain a hydroprocessing effluent A, subjecting the hydroprocessing effluent A to fractionation and separating from the fractionation at least a recycle stream having a T5 temperature (5 vol-% recovered, EN ISO 3405-2019) of 270 °C or higher, and optionally comprising C16 n-paraffins;
   ii) subjecting a reactor B feed comprising the recycle stream to hydroprocessing in a reactor B in the presence of a hydrotreatment catalyst B to obtain a hydroprocessing effluent B;
   iii) feeding the hydroprocessing effluent B to the fractionation as a co-feed with the hydroprocessing effluent A, and recovering from the fractionation at least one or more liquid transportation fuel component(s); and
monitoring parameters indicative of deactivation of the hydrotreatment catalyst A to receive values;
comparing the received values with predetermined values; and
when the received values reach the predetermined values, switching from i), ii), iii) to:
   I) subjecting a reactor B feed comprising the hydrocarbon feed to hydroprocessing in the reactor B in the presence of the hydrotreatment catalyst B to obtain a hydroprocessing effluent B, subjecting the hydroprocessing effluent B to fractionation and separating from the fractionation at least a recycle stream having a T5 temperature (5 vol-% recovered, EN ISO 3405-2019) of 270 °C or higher, and optionally comprising C16 n-paraffins;
   II) subjecting a reactor A feed comprising the recycle stream to hydroprocessing in the reactor A in the presence of the hydrotreatment catalyst A to obtain a hydroprocessing effluent A;
   III) feeding the hydroprocessing effluent A to the fractionation as a co-feed with the hydroprocessing effluent B, and recovering from the fractionation at least one or more liquid transportation fuel component(s).

According to a third aspect of the invention, there is provided a process for producing at least one liquid transportation fuel component, the process comprising:
i) providing a reactor A feed comprising a hydrocarbon feed comprising nitrogen impurities, wherein the reactor A feed comprises at least 0.4 w-ppm nitrogen, expressed as elemental nitrogen (ASTM D4629-17), of the total weight of the reactor A feed, or at least 0.6 w-ppm, or at least 1.0 w-ppm, or even at least 1.5 w-ppm or at least 2.0 w-ppm nitrogen;
ii) subjecting the reactor A feed to hydroprocessing in a reactor A in the presence of a hydrotreatment catalyst A to obtain a hydroprocessing effluent A, and optionally separating from the hydroprocessing effluent A at least compounds gaseous at NTP to obtain a degassed hydroprocessing effluent A;
iii) feeding the hydroprocessing effluent A or the degassed hydroprocessing effluent A to fractionation, and recovering from the fractionation at least one or more liquid transportation fuel component(s); and

monitoring parameters indicative of deactivation of the hydrotreatment catalyst A to receive values;
comparing the received values with predetermined values; and
when the received values reach the predetermined values, switching from i), ii), iii) to:
   I) providing a reactor A feed comprising a hydrocarbon feed, wherein the reactor A feed comprises essentially no nitrogen impurities;
   II) subjecting the reactor A feed to hydroprocessing in the reactor A in the presence of the hydrotreatment catalyst A to obtain a hydroprocessing effluent A, and optionally separating from the hydroprocessing effluent A at least compounds gaseous at NTP to obtain a degassed hydroprocessing effluent A;
   III) feeding the hydroprocessing effluent A or the degassed hydroprocessing effluent A to fractionation, and recovering from the fractionation at least one or more liquid transportation fuel component(s).

In the present disclosure, reference to a/the process, to the present process, or to any features thereof, should be understood to concern any of the processes according to the first, second and/or third example aspects, unless it is specifically indicated to concern only certain example aspect(s), or unless it is evident from the context to concern only certain example aspect(s).

In certain preferred embodiments, the hydrocarbon feed comprises at least 0.4 w-ppm nitrogen impurities, expressed as elemental nitrogen (ASTM D4629-17), of the total weight of the hydrocarbon feed, or at least 0.6 w-ppm, or at least 1.0 w-ppm, or even at least 1.5 w-ppm or at least 2.0 w-ppm nitrogen impurities.

In certain embodiments, in steps iii) and III) at least one or more of an aviation fuel component, a diesel fuel component, a gasoline fuel component, and/or a marine fuel component is recovered from the fractionation, preferably at least an aviation fuel component, more preferably at least an aviation fuel component and a diesel fuel component or at least an aviation fuel component and a gasoline fuel component.

In certain preferred embodiments, in steps iii) and III) at least an aviation fuel component having density at 15 °C within a range from 730 to 772 kg/m³ (EN ISO 12185-1996), T10 temperature at most 205 °C (EN ISO 3405-2019), final boiling point at most 300 °C (EN ISO 3405-2019), flash point at least 38 °C (IP 170-2013, Abel closed-cup method), and freezing point at most -40 °C (IP 529-2016) is recovered from the fractionation.

The present process is particularly useful for producing liquid transportation fuel components from a broader range of feeds, including more impure and optionally also heavier feeds, particularly feeds comprising elevated content of nitrogen impurities, compared to conventional processes for producing fuel components by hydrodeoxygenation (HDO) and hydroisomerisation (HI) of renewable fats and oils. Further, the present process may provide prolonged process run-time or extend the hydrotreatment catalyst lifetime, compared to the above-referred conventional processes. The present process is particularly suitable for producing aviation fuel component. The present process enables obtaining higher yield of aviation fuel component. Also, the present process enables producing aviation fuel component with improved quality that is usable in aviation fuels in elevated amounts, or even as such when suitably additized, compared to aviation fuel components produced by the above-referred conventional processes from same feed.

According to a fourth aspect of the invention, there is provided a computer program product comprising instructions which, when executed by a processor of a control apparatus in a system for producing at least one liquid transportation fuel component, cause in a process according to the first, the second, or the third aspect of the invention, the control apparatus to compare the received values with predetermined values, and when the received values reach the predetermined values to switch from i), ii), iii) to I), II), III), and optionally to switch from I), II), III) back to i), ii), iii). The computer program product may cause a control apparatus to perform of the process according to the first, the second, or the third example aspect at least the steps of comparing the received values with predetermined values; and switching from i), ii), iii) to I), II), III), and optionally switching from I), II), III) back to i), ii), iii).

The computer program product according to the fourth aspect of the invention enables the process according to the first, second and/or third example aspects to be operated more precisely, monitoring a higher number of different parameters, comparing the obtained values to huge datasets, using the obtained values in complex models, adjusting, and/or switching the process quicker based on the comparison, improving or adjusting the yields and/or quality of the recovered liquid transportation fuel component(s) depending on the need, and enhancing safety of the present process, just to name a few advantages.

Different non-binding example aspects and embodiments have been illustrated in the foregoing. The embodiments in the foregoing are used merely to explain selected aspects or steps that may be utilised in different implementations. Some embodiments may be presented only with reference to certain example aspects.

### BRIEF DESCRIPTION OF THE FIGURES

Some example embodiments will be described with reference to the accompanying figures, in which:
Fig. 1 schematically shows a process according to an embodiment of the first aspect of the invention before switching from i), ii), iii) to I), II), III) i.e. running according to the steps i), ii), iii) (first mode);
Fig. 2 schematically shows a process according to an embodiment of the first aspect of the invention after having switched from i), ii), iii) to I), II), III) i.e. running according to the steps I), II), III) (second mode);
Fig. 3 schematically shows a process according to an embodiment of the second aspect of the invention before switching from i), ii), iii) to I), II), III) i.e. running according to the steps i), ii), iii) (first mode);
Fig. 4 schematically shows a process according to an embodiment of the second aspect of the invention after having switched from i), ii), iii) to I), II), III) i.e. running according to the steps I), II), III) (second mode); and
Fig. 5 schematically shows a process according to an embodiment of the third aspect of the invention.

### DETAILED DESCRIPTION

In the following description, like reference signs denote like elements or steps.

All standards referred to herein are the latest revisions available at the filing date, unless otherwise mentioned.

Unless otherwise stated, regarding distillation characteristics, such as initial boiling points (IBP), final boiling points (FBP), T5 temperature (5 vol-% recovered), T95 temperature (95 vol-% recovered), and boiling ranges, reference is made to EN ISO 3405-2019. IBP is the temperature at the instant the first drop of condensate falls from the lower end of the condenser tube, and FBP is the maximum thermometer reading obtained during the test, usually occurring after the evaporation of all liquid from the bottom of the flask. For boiling point distribution, reference may also be made to GC-based method (simdis) ASTM D2887-19e1, or for gasoline range hydrocarbons to ASTM D7096-19.

As used in the context of this disclosure, aviation fuel component refers to hydrocarbon compositions suitable for use in fuel compositions meeting standard specifications for aviation fuels, such as specifications laid down in ASTM D7566-21. Typically, such aviation fuel components boil, i.e. have IBP and FBP, within a range from about 100 °C to about 300 °C, such as within a range from about 150 °C to about 300 °C, as determined according to EN ISO 3405-2019.

As used in the context of this disclosure, diesel fuel component refers to hydrocarbon compositions suitable for use in fuel compositions meeting standard specifications for diesel fuels, such as specifications laid down in EN 590:2022 or in EN 15940:2016 + A1:2018 + AC:2019. Typically, such diesel fuel components boil, i.e. have IBP and FBP, within a range from about 160 °C to about 380 °C, as determined according to EN ISO 3405-2019.

As used in the context of this disclosure, gasoline fuel component or naphtha refers to hydrocarbon components suitable for use in fuel compositions meeting standard specifications for gasoline fuels, such as specifications laid down in EN 228-2012 + A1-2017. Typically, such gasoline fuel components boil, i.e. have IBP and FBP, within a range from about 25 °C to about 210 °C, as determined according to EN ISO 3405-2019.

As used in the context of this disclosure, marine fuel component refers to hydrocarbon components suitable for use in fuel compositions meeting standard specifications for marine fuels, such as specifications laid down in ISO 8217-2017. Typically, such marine fuel components boil, i.e. have IBP and FBP, within a range from about 180 °C to about 600 °C, such as from about 180 °C to about 400 °C, as determined according to EN ISO 3405-2019.

As used herein hydrocarbons refer to compounds consisting of carbon and hydrogen. Hydrocarbons of particular interest in the present context comprise paraffins, n-paraffins, i-paraffins, monobranched i-paraffins, multiple-branched i-paraffins, olefins, naphthenes, and aromatics. Oxygenated hydrocarbons refer herein to hydrocarbons comprising covalently bound oxygen.

As used herein paraffins refer to non-cyclic alkanes, i.e. non-cyclic, open chain saturated hydrocarbons that are linear (normal paraffins, n-paraffins) or branched (isoparaffins, i-paraffins). In other words, paraffins refer herein to n-paraffins and/or i-paraffins.

In the context of the present disclosure, i-paraffins refer to branched open chain alkanes, i.e. non-cyclic, open chain saturated hydrocarbons having one or more alkyl side chains. Herein, i-paraffins having one alkyl side chain or branch are referred to as monobranched i-paraffins and i-paraffins having two or more alkyl side chains or branches are herein referred to as multiple-branched i-paraffins. In other words, i-paraffins refer herein to monobranched i-paraffins and/or multiple-branched i-paraffins. The alkyl side chain(s) of i-paraffins may for example be C1-C9 alkyl side chain(s), preferably methyl side chain(s). The amounts of monobranched and multiple-branched i-paraffins may be given separately. The term "i-paraffins" refers to sum amount of any monobranched i-paraffins and any multiple-branched i-paraffins, if present, indicating the total amount of any i-paraffins present regardless the number of branches. Correspondingly, "paraffins" refers to sum amount of any n-paraffins, any mono-branched, and any multiple-branched i-paraffins, if present.

In the context of the present disclosure, olefins refer to unsaturated, linear, branched, or cyclic hydrocarbons, excluding aromatic compounds. In other words, olefins refer to hydrocarbons having at least one unsaturated bond, excluding unsaturated bonds in aromatic rings.

As used herein, cyclic hydrocarbons refer to all hydrocarbons containing cyclic structure(s), including cyclic olefins, naphthenes, and aromatics. Naphthenes refer herein to cycloalkanes i.e. saturated hydrocarbons containing at least one cyclic structure, with or without side chains. As naphthenes are saturated compounds, they are compounds without aromatic ring structure(s) present. Aromatics refer herein to hydrocarbons containing at least one aromatic ring structure, i.e. cyclic structure having delocalized, alternating π bonds all the way around said cyclic structure.

In the context of the present disclosure, for compositions boiling at 36 °C or higher (at standard atmospheric pressure), contents of n-paraffins, i-paraffins, monobranched i-paraffins, various multiple-branched isoparaffins, naphthenes, and aromatics, are expressed as weight % (wt-%) relative to the degassed weight of the feed, stream, effluent, product, component or sample in question or, when so defined, as weight-% (wt-%) relative to the (total) weight of paraffins or (total) weight of i-paraffins of the feed, stream, effluent, product, component, or sample in question. Said contents can be determined by GCxGC-FID/GCxGC-MS method, preferably conducted as follows: GCxGC (2D GC) method was run as generally disclosed in UOP 990-2011 and by Nousiainen M. in the experimental section of his Master's Thesis Comprehensive two-dimensional gas chromatography with mass spectrometric and flame ionization detectors in petroleum chemistry, University of Helsinki, August 2017, with the following modifications. The GCxGC was run in reverse mode, using a semipolar column (Rxi17Sil) first and a nonpolar column (Rxi5Sil) thereafter, followed by FID detector, using run parameters: carrier gas helium 31.7 cm/sec (column flow at 40 °C 1.60 ml/min); split ratio 1:350; injector 280 °C; Column T program 40 °C (0 min) - 5 °C/min - 250 °C (0 min) - 10 °C/min - 300 °C (5 min), run time 52 min; modulation period 10 sec; detector 300 °C with H₂ 40 ml/min and air 400 ml/min; makeup flow helium 30 ml/min; sampling rate 250 Hz and injection size 0.2 microliters. Individual compounds were identified using GCxGC-MS, with MS-parameters: ion source 230 °C; interface 300 °C; scan range 25 - 500 amu; event time (sec) 0.05; scan speed 20000. Commercial tools (Shimadzu's LabSolutions, Zoex's GC Image) were used for data processing including identification of the detected compounds or hydrocarbon groups, and for determining their mass concentrations by application of response factors relative to n-heptane to the volumes of detected peaks followed by normalization to 100 wt-%. Olefins were lumped with naphthenes and heteroatomic species with aromatics, unless separately reported. The limit of quantitation for individual compounds of this method is 0.1 wt-%.

In the context of the present disclosure, various characteristics of the feeds, streams, effluents, products, components, or samples are determined according to the standard methods referred to or disclosed herein, as properly prepared. For example, cloud point is determined according to ASTM D 5771-17 from a degassed feed, stream, effluent, product, component, or sample.

Typically, the various hydrocarbon feeds, streams, effluents, and fuel components as recovered products or fractions thereof, referred to herein, may comprise in addition to hydrocarbons, also varying trace amounts of e.g. heteroatom-containing hydrocarbons and inorganic compounds as impurities, and the oxygenated hydrocarbon feed varying amounts of e.g. other heteroatom-containing hydrocarbons and inorganic compounds as impurities. Generally, the level of impurities in the main process streams is highest in the first parts of the process and decreases subsequently being negligible or even below detection limit in the recovered liquid transportation fuel component(s).

As used herein, the term hydroprocessing refers to a treatment in the presence of added molecular hydrogen and a solid hydrotreatment catalyst. Typically, the solid hydrotreatment catalyst is selective to the desired reaction(s) at the prevailing reaction conditions. The term hydroprocessing includes herein treatments consuming hydrogen, but also treatments not involving substantial hydrogen consumption. Examples of hydroprocessing include hydroisomerisation, hydrocracking, hydrodearomatisation, and hydropolishing, and may involve various reactions such as isomerization, cracking, (de)hydrogenation, desulfurization, denitrogenation, deoxygenation etc, depending on the feed constituents and impurity species, selected catalyst and reaction conditions.

Isomerisation converts at least a certain amount of n-paraffins to i-paraffins, especially to mono-branched i-paraffins. By (further) raising isomerization degree, for example by increasing severity of hydroisomerisation as described hereinafter, more n-paraffins can be converted to i-paraffins, and mono-branched i-paraffins can be converted to multiple-branched i-paraffins, such as di-branched, and/or tri-branched i-paraffins, even i-paraffins comprising more than three branches. As used herein, and particularly in the context of embodiments wherein hydroprocessing in reactor A and/or B is hydrocracking, degree of effective cracking refers to cracking that yields non-gaseous (NTP) cracking products, and especially as expressed herein as the ratio of the C8 to C14 hydrocarbon content in the effluent of the respective reactor to the C8 to C14 hydrocarbon content in the respective reactor feed.

As used herein, wherever the reaction steps are defined to take place in "reactors", such as reactor A and/or reactor B, said expression is used for illustrative purposes mainly. A person skilled in the art contemplates that any "reactor" is in practice implemented as a reactor system that may consist of one or more reactors. Whether the reactors are actually arranged in a single reactor or several reactors is a matter of engineering, and may be influenced by practical issues such as maximum height of the facility at the site, reactor diameter, regulatory and maintenance issues at the site, wind conditions at the site, and/or available equipment. Analogously, the "fractionation" may take place in a fractionation system, typically comprising e.g. separation and distillation units, which may be arranged according to conventional engineering practice in the field.

Catalyst characteristics, such as total number of acid sites, refers in the context of this disclosure to the catalyst characteristics in their ready-to-use state, in the beginning of the present process.

As used herein, the term catalyst deactivation refers to decreased activity of the catalyst (reflected by an increased amount of unconverted feed in the reactor effluent) and/or decreased selectivity of the catalyst (reflected by decreased amount of desired reaction products in the reactor effluent) at a given time point (tₙ), compared to the activity and/or selectivity of the catalyst in the beginning of the process (t₀). As used herein, the term catalyst deactivation is not limited to any specific deactivation type or mechanism, although the catalyst deactivation observed in the present process is generally believed to be attributed to poisoning and fouling phenomena, and encompasses both reversible and irreversible deactivation. However, as the present processes use hydrocarbon feeds comprising at least nitrogen impurities, the term catalyst deactivation encompasses herein at least reversible deactivation.

In the context of this disclosure, feed(s) to reactors, particularly to reactor A and/or reactor B, are defined so that H₂ possibly fed to the respective reactor, for example H₂ fed to the hydroprocessing, is excluded from the definition of the feed(s).

As used herein, the term renewable refers to compounds or compositions that are obtainable, derivable, or originating from plants and/or animals, including compounds or compositions obtainable, derivable, or originating from fungi and/or algae, in full or in part. As used herein, renewable compounds or compositions may comprise gene manipulated compounds or compositions. Renewable feeds, components, compounds, or compositions may also be referred to as biological feeds, components, compounds, or compositions, or as biogenic feeds, components, compounds, or compositions.

As used herein, the term fossil refers to compounds or compositions that are obtainable, derivable, or originating from naturally occurring non-renewable compositions, such as crude oil, petroleum oil/gas, shale oil/gas, natural gas, or coal deposits, and the like, and combinations thereof, including any hydrocarbon-rich deposits that can be utilised from ground/underground sources.

The term circular refers to recycled material typically originating from non-renewable sources. For example, the term circular may refer to recycled material originating from waste plastics. Said renewable, circular, and fossil compounds or compositions are considered differing from one another based on their origin and impact on environmental issues. Therefore, they may be treated differently under legislation and regulatory framework. Typically, renewable, circular, and fossil compounds or compositions are differentiated based on their origin and information thereof provided by the producer.

Chemically the renewable or fossil origin of any organic compounds, including hydrocarbons, can be determined by suitable method for analysing the content of carbon from renewable sources e.g. DIN 51637 (2014), ASTM D6866 (2020) or EN 16640 (2017). Said methods are based on the fact that carbon atoms of renewable or biological origin comprise a higher number of unstable radiocarbon (¹⁴C) atoms compared to carbon atoms of fossil origin. Therefore, it is possible to distinguish between carbon compounds derived from renewable or biological sources or raw material and carbon compounds derived from non-renewable or fossil sources or raw material by analysing the ratio of ¹²C and ¹⁴C isotopes. Thus, a particular ratio of said isotopes can be used as a "tag" to identify a renewable carbon compound and differentiate it from non-renewable carbon compounds. The isotope ratio does not change in the course of chemical reactions. Therefore, the isotope ratio can be used for identifying renewable compounds, components, and compositions, and distinguishing them from non-renewable, fossil materials in reactor feeds, reactor effluents, separated product fractions, and various blends thereof. Numerically, the biogenic carbon content can be expressed as the amount of biogenic carbon in the material as a weight percent of the total carbon (TC) in the material (in accordance with ASTM D6866 (2020) or EN 16640 (2017)). In the present context, the term renewable preferably refers to a material having a biogenic carbon content of more than 50 wt-%, especially more than 60 wt-% or more than 70 wt-%, preferably more than 80 wt-%, more preferably more than 90 wt-% or more than 95 wt-%, even more preferably about 100 wt-%, based on the total weight of carbon in the material (EN 16640 (2017)).

According to the first aspect of the invention, herein is provided a process for producing at least one liquid transportation fuel component, the process comprising:
providing a hydrocarbon feed comprising nitrogen impurities;
i) subjecting a reactor A feed comprising the hydrocarbon feed to hydroprocessing in a reactor A in the presence of a hydrotreatment catalyst A to obtain a hydroprocessing effluent A, and separating from the hydroprocessing effluent A at least compounds gaseous at NTP to obtain a degassed hydroprocessing effluent A;
ii) subjecting a reactor B feed comprising the degassed hydroprocessing effluent A to hydroprocessing in a reactor B in the presence of a hydrotreatment catalyst B to obtain a hydroprocessing effluent B,;
iii) feeding the hydroprocessing effluent B, optionally after separating from the hydroprocessing effluent B at least compounds gaseous at NTP, to fractionation, and recovering from the fractionation at least one or more liquid transportation fuel component(s); and

monitoring parameters indicative of deactivation of the hydrotreatment catalyst A to receive values;
comparing the received values with predetermined values; and
when the received values reach the predetermined values, switching from i), ii), iii) to:
   I) subjecting a reactor B feed comprising the hydrocarbon feed to hydroprocessing in the reactor B in the presence of the hydrotreatment catalyst B to obtain a hydroprocessing effluent B, and separating from the hydroprocessing effluent B at least compounds gaseous at NTP to obtain a degassed hydroprocessing effluent B;
   II) subjecting a reactor A feed comprising the degassed hydroprocessing effluent B to hydroprocessing in the reactor A in the presence of the hydrotreatment catalyst A to obtain a hydroprocessing effluent A;
   III) feeding the hydroprocessing effluent A, optionally after separating from the hydroprocessing effluent A at least compounds gaseous at NTP, to fractionation, and recovering from the fractionation at least one or more liquid transportation fuel component(s).

According to the second aspect of the invention, herein is provided a process for producing at least one liquid transportation fuel component, the process comprising:
providing a hydrocarbon feed comprising nitrogen impurities;
i) subjecting a reactor A feed comprising the hydrocarbon feed to hydroprocessing in a reactor A in the presence of a hydrotreatment catalyst A to obtain a hydroprocessing effluent A, subjecting the hydroprocessing effluent A to fractionation and separating from the fractionation at least a recycle stream having a T5 temperature (5 vol-% recovered, EN ISO 3405-2019) of 270 °C or higher, and optionally comprising C16 n-paraffins;
ii) subjecting a reactor B feed comprising the recycle stream to hydroprocessing in a reactor B in the presence of a hydrotreatment catalyst B to obtain a hydroprocessing effluent B;
iii) feeding the hydroprocessing effluent B to the fractionation as a co-feed with the hydroprocessing effluent A, and recovering from the fractionation at least one or more liquid transportation fuel component(s); and

monitoring parameters indicative of deactivation of the hydrotreatment catalyst A to receive values;
comparing the received values with predetermined values; and
when the received values reach the predetermined values, switching from i), ii), iii) to:
   I) subjecting a reactor B feed comprising the hydrocarbon feed to hydroprocessing in the reactor B in the presence of the hydrotreatment catalyst B to obtain a hydroprocessing effluent B, subjecting the hydroprocessing effluent B to fractionation and separating from the fractionation at least a recycle stream having a T5 temperature (5 vol-% recovered, EN ISO 3405-2019) of 270 °C or higher, and optionally comprising C16 n-paraffins;
   II) subjecting a reactor A feed comprising the recycle stream to hydroprocessing in the reactor A in the presence of the hydrotreatment catalyst A to obtain a hydroprocessing effluent A;
   III) feeding the hydroprocessing effluent A to the fractionation as a co-feed with the hydroprocessing effluent B, and recovering from the fractionation at least one or more liquid transportation fuel component(s).

According to the third aspect of the invention, herein is provided a process for producing at least one liquid transportation fuel component, the process comprising:
i) providing a reactor A feed comprising a hydrocarbon feed comprising nitrogen impurities, wherein the reactor A feed comprises at least 0.4 w-ppm nitrogen, expressed as elemental nitrogen (ASTM D4629-17), of the total weight of the reactor A feed, or at least 0.6 w-ppm, or at least 1.0 w-ppm, or even at least 1.5 w-ppm or at least 2.0 w-ppm nitrogen;
ii) subjecting the reactor A feed to hydroprocessing in a reactor A in the presence of a hydrotreatment catalyst A to obtain a hydroprocessing effluent A, and optionally separating from the hydroprocessing effluent A at least compounds gaseous at NTP to obtain a degassed hydroprocessing effluent A;
iii) feeding the hydroprocessing effluent A or the degassed hydroprocessing effluent A to fractionation, and recovering from the fractionation at least one or more liquid transportation fuel component(s); and
   monitoring parameters indicative of deactivation of the hydrotreatment catalyst A to receive values;
   comparing the received values with predetermined values; and
   when the received values reach the predetermined values, switching from i), ii), iii) to:
      I) providing a reactor A feed comprising a hydrocarbon feed, wherein the reactor A feed comprises essentially no nitrogen impurities;
      II) subjecting the reactor A feed to hydroprocessing in the reactor A in the presence of the hydrotreatment catalyst A to obtain a hydroprocessing effluent A, and optionally separating from the hydroprocessing effluent A at least compounds gaseous at NTP to obtain a degassed hydroprocessing effluent A;
      III) feeding the hydroprocessing effluent A or the degassed hydroprocessing effluent A to fractionation, and recovering from the fractionation at least one or more liquid transportation fuel component(s).

Preferably, the present process according to the first, second and third aspects is a continuous process.

The process as operated according to steps i), ii), iii), may be referred to as the initial/first mode of the process/operation, and the process as operated according to steps I), II), III), may be referred to as the switched/second mode of the process/operation.

During continued operation of catalytic hydroprocessing, the hydrotreatment catalyst(s) deactivate gradually for example due to impurities in process streams and coking. Catalyst deactivation may be of reversible or irreversible nature. A typical example of reversible deactivation is the deactivation of acid sites of a catalyst by nitrogen impurities in the feed. Although the effects can be severe, they are temporary and may generally be eliminated within hours to days, or sometimes within weeks, after the nitrogen source is removed from the feed.

Nitrogen is a typical impurity e.g. in hydrocarbon feeds obtained by subjecting an oxygenated hydrocarbon feed to catalytic hydrodeoxygenation (HDO). This may be due to slippage of nitrogen in the oxygenated hydrocarbon feed through the catalytic HDO step, whether caused by particularly high nitrogen content in the feed and/or reduced activity and efficiency of the (HDO) catalyst to cleave heteroatoms, which may occur particularly in the end-of-run. Other examples of reversibly bound impurities may include some carbonaceous impurities such as coke, particularly soft coke.

In the first mode of operation, the hydrotreatment catalyst A is subjected to most of the impurities, in many embodiments to all of the impurities, in the hydrocarbon feed. Switching the process from i), ii), iii) to I), II), III), i.e. from the first mode of the process to the second mode of the process, results in feeding to the hydroprocessing reactor A a stream that is purer (contains less nitrogen impurities) than the hydrocarbon feed fed thereto in the first mode. As the deactivation of the hydrotreatment catalyst A occurring in the first mode of the present process is caused at least by the nitrogen impurities present in the hydrocarbon feed, the purer stream fed to the hydroprocessing reactor A in the second mode helps to flush the hydroteatment catalyst A and liberate reversibly bound impurities therefrom, thereby reversing the deactivation at least to some extent. In other words, in this way reversibly bound nitrogen can be flushed from the hydrotreatment catalyst A and its deactivation reversed at least in part. Nitrogen is liberated from the hydrotreatment catalyst A as NH₃, which is then separated from the hydroprocessing effluent A by degassing or fractionation. Liberating nitrogen from the hydrotreatment catalyst A may be enhanced by operating reactor A with elevated pressure and/or temperature.

In the process according to the first and the second aspects of the invention, in the first mode of operation the hydrotreatment catalyst B in reactor B is protected by the cleaning effect of the hydrotreatment catalyst A in reactor A located upstream, so that hydrotreatment catalyst B is expected to deactivate slower than the hydrotreatment catalyst A in reactor A. The cleaning effect of catalyst A includes conversion of impurities to compounds gaseous at NTP, particularly conversion of nitrogen impurities to NH₃, and to a lesser extent binding or deposition of impurities on the hydrotreatment catalyst A. Separating or removing compounds gaseous at NTP from the hydrotreatment effluent A removes converted impurities from the process streams and hence from the reactor B feed (and vice versa in the second mode of operation). In the first aspect, the purer stream fed to the reactor A in the second mode of operation is achieved by feeding the hydrocarbon feed to reactor B and the hydrotreatment effluent B, after separation of compounds gaseous at NTP, to reactor A, i.e. in a sense changing the order of reactors A and B in the process so that the hydrotreatment catalyst A in reactor A is protected by the cleaning effect of the hydrotreatment catalyst B in reactor B now located upstream. In the second aspect, the purer stream fed to the reactor A in the second mode is achieved similarly by, in a sense, changing the order of reactors A and B so that the hydrocarbon feed is fed to reactor B and the recycle stream, comprising a fraction of the hydrotreatment effluent B, is fed to reactor A. Hence, typically in the first mode of operation, reactor A is upstream of reactor B, and in the second mode of operation reactor B is upstream of reactor A.

In the third aspect, the purer stream fed to the reactor A in the second mode of operation is achieved by providing a reactor A feed essentially without nitrogen impurities, for example by providing a purer hydrocarbon feed (hydrocarbon feed with less or essentially no nitrogen impurities) and/or by diluting the hydrocarbon feed.

In the present disclosure the aim is to improve the overall process efficiency by prolonging run-time of the process, or extending hydrotreatment catalyst lifetime, and/or enabling higher impurity content in the process feed. In this way the raw materials may be subjected to less laborious and/or severe pre-treatment methods, and sourced with far higher flexibility regarding quality. Furthermore, embodiments using hydrocarbon feeds obtained by subjecting an oxygenated hydrocarbon feed to HDO have as an additional or alternative aim running the catalytic HDO longer and hence allowing the HDO catalyst to deactivate more than conventionally, before changing the HDO catalyst.

Hence, in certain preferred embodiments of the process according to the first and the second aspects, and in certain preferred embodiments of the first mode of the process according to the third aspect, the hydrocarbon feed comprises nitrogen impurities and comprises at least 0.4 w-ppm nitrogen, expressed as elemental nitrogen (ASTM D4629-17), of the total weight of the hydrocarbon feed, or at least 0.6 w-ppm, or at least 1.0 w-ppm, or even at least 1.5 w-ppm or at least 2.0 w-ppm nitrogen. The present process is particularly suitable for processing hydrocarbon feeds with a high or increased nitrogen content. Preferably, the hydrocarbon feed comprises at most 40.0 w-ppm nitrogen, expressed as elemental nitrogen (ASTM D4629-17). An increased amount of nitrogen in the hydrocarbon feed typically expedites catalyst deactivation. As mentioned above, deactivation of acid sites of a catalyst by nitrogen impurities is reversible deactivation which may be reversed at least to some extent by switching of the mode of the present process.

When running the process according to the third aspect in the second mode, the reactor A feed comprises essentially no nitrogen impurities, i.e. a reactor A feed essentially without nitrogen impurities is provided. Preferably this means that the reactor A feed comprises in the second mode at most 0.3 w-ppm, more preferably less than 0.3 w-ppm nitrogen expressed as elemental nitrogen (ASTM D4629-17), based on the total weight of the reactor A feed. This is in the process according to the third aspect preferably achieved by a hydrocarbon feed comprising at most 0.3 w-ppm, more preferably less than 0.3 w-ppm nitrogen expressed as elemental nitrogen (ASTM D4629-17), based on the total weight of the hydrocarbon feed.

The at least one or more liquid transportation fuel component(s) recovered from the fractionation may be one or more of an aviation fuel component, a diesel fuel component, a gasoline fuel component, and/or a marine fuel component. Preferably, at least an aviation fuel component is recovered, more preferably at least an aviation fuel component and a diesel fuel component, or at least an aviation fuel component and a gasoline fuel component are recovered.

With the present process it is possible to obtain higher yield of liquid transportation fuel components, particularly aviation fuel component, throughout the run-time of the process or lifetime of hydrotreatment catalyst A, without a need to reduce the capacity of the hydroprocessing in reactor A, and/or to improve quality of the aviation fuel component, compared for example to conventional processes for producing fuel components by hydrodeoxygenation (HDO) and hydroisomerisation (HI) of renewable fats and oils.

With the present process it may be possible to produce, even towards the end of run, an aviation fuel component having excellent cold properties and hence usable in aviation fuels with high blending ratio or even as such, i.e. unblended, when suitably additised. Also, the present process enables extending the hydrotreatment catalyst lifetime and enables utilisation of a broader range of feeds, including more impure and even heavier feeds, compared to conventional processes for producing fuel components by HDO and HI of renewable fats and oils.

Furthermore, the present process enables flexible adjustment of product selectivity towards the various liquid transportation fuel components, such as gasoline fuel component, aviation fuel component, diesel fuel component, and/or marine fuel component based on e.g. market dynamics, even towards the end of run.

The present process is highly flexible and involves various possibilities to adjust the yield and/or quality of the recovered liquid transportation fuel component(s), and to extend process run-time or the lifetime of the hydrotreatment catalyst, particularly the lifetime of the hydrotreatment catalyst A. The various possibilities of the present process to compensate deactivation of the hydrotreatment catalyst enable use of hydrocarbon feeds with higher impurity content compared to conventional processes for producing liquid fuel components by HDO and HI of renewable fats and oils. Additionally, when using hydrocarbon feeds obtained by subjecting an oxygenated hydrocarbon feed to catalytic hydrodeoxygenation (HDO), the present process may tolerate slippage of some nitrogen through the HDO step, as may occur particularly towards the end of HDO catalyst lifetime, and consequently the HDO may be run longer, compared for example to a conventional process where HDO is followed by HI.

The present process comprises monitoring parameters indicative of deactivation of the hydrotreatment catalyst A to receive values; comparing the received values with predetermined values; and when the received values reach or equal the predetermined values switching from i), ii), iii) to I), II), III), i.e. switching from the first mode of the process to the second mode of the process. Preferably, the received values are compared with corresponding predetermined values. Switching from i), ii), iii) to I), II), III) enables compensating hydrotreatment catalyst deactivation (especially deactivation of hydrotreatment catalyst A) allowing good yield and quality of the recovered at least one liquid transportation fuel component for a longer period before a need to change or regenerate the hydrotreatment catalyst A. Also, switching from i), ii), iii) to I), II), III) results in feeding to the hydroprocessing reactor A a stream that is purer than the hydrocarbon feed fed thereto in the first mode which flushes the hydrotreatment catalyst A at least to some extent liberating reversibly bound impurities therefrom, i.e. causes in a sense regeneration of the hydrotreatment catalyst A at least to some extent. Initially, in the beginning of a process run, typically the activity of the hydrotreatment catalyst A is good or sufficient and the received values from the monitoring do not equal the (corresponding) predetermined values. However, at some point during the process run the deactivation of the hydrotreatment catalyst A has typically reached a point when the received values from the monitoring reaches the (corresponding) predetermined values, which predetermined values have been identified to represent insufficient or near-insufficient performance of the hydrotreatment catalyst A, for example in terms of volumes (yields), and/or compositional, and/or physico-chemical characteristics of the recovered liquid transportation fuel component(s). At that point, when the received values from the monitoring reaches the (corresponding) predetermined values, switching from i), ii), iii) to I), II), III) is performed typically in order to restore, and preferably even exceed, the targeted minimum for the process performance. By switching from i), ii), iii) to I), II), III) the process may be operated longer producing the desired liquid transportation fuel component(s) in desired quantity and/or with desired quality. Optionally, the present process may comprise when running according to I), II), III) monitoring parameters indicative of reversal of the deactivation of the hydrotreatment catalyst A to receive values, comparing the received values with predetermined values, and optionally when the received values reach the predetermined values, switching from I), II), III) back to i), ii), iii). For example, the process may be switched back from I), II), III) to i), ii), iii) when sufficient reversal of deactivation of hydrotreatment catalyst A has been achieved.

None of reactors A and/or B is typically by-passed when running the present process. Typically, a process run of the present process, comprising at least one switching from i), ii), iii) to I), II), III), is performed without replacing the hydrotreatment catalyst A and/or hydrotreatment catalyst B, or without by-passing reactor A and/or B to regenerate a catalyst therein. In other words, reactors A and B typically remain on-stream, i.e. in fluid communication with each other throughout the process run, including during the switching and/or just before it. Similarly, in the third aspect, feeding reactor feed to reactor A is typically not interrupted during the process run.

In certain embodiments, the present process may include in addition to switching from the first mode to the second mode, adjusting one or more operating conditions of reactor A and/or reactor B to reach, maintain, or even exceed targeted minimum for the process performance. In such embodiments, switching from the first mode to the second mode may be performed at some point after operating conditions of reactor A and/or reactor B have been adjusted, at one or more occasions. Operating conditions may be adjusted to their feasible extreme(s) and then the switching is performed, or alternatively, operating conditions may be adjusted to some extent and the switching is performed already before extremes are reached. Said adjusting of one or more operating conditions may be based on same or different parameters than the switching from i), ii), iii) to I), II), III). Preferably, one or more operating conditions of reactor A and/or reactor B, particularly reactor A, are first adjusted to compensate for catalyst deactivation, and when compensating for catalyst deactivation by merely adjusting operating conditions in reactor A and/or reactor B is no longer feasible (or no longer desired), the switching from i), ii), iii) to I), II), III) is performed. In certain embodiments, after the switching from i), ii), iii) to I), II), III) operating conditions of reactor A may be relieved, when the switching is substantially completed, and thus the targeted yield and/or quality of the recovered fuel component(s) may be achieved for example at lower temperature and/or higher WHSV in reactor A, than just before switching. In these embodiments, after switching, it may be beneficial to increase the temperature in reactor B to compensate for the typically increased WHSV therein. In the course of the second mode of operation, one or more operating conditions of reactor A and/or reactor B may (again) be adjusted to compensate for further catalyst deactivation, particularly deactivation of the hydrotreatment catalyst A in reactor A.

When the process has been operated long enough and switched between the modes, at least from the first to the second mode, and operating conditions in reactor A and/or reactor B have optionally been adjusted to their feasible extreme(s), the targeted performance of the process, for example in terms of yields, and/or compositional, and/or physico-chemical characteristics of the recovered liquid transportation fuel component(s), will no longer be reached due to extent of catalyst deactivation. Then typically the catalyst(s) is changed to fresh one(s) or regenerated, this typically denoting in the present process end of a process run, before optionally starting to operate the process in the first mode (starting a new process run), i.e. running sequence i), ii), iii).

In certain embodiments, the process does not comprise, i.e. is performed without, adjusting operating conditions to compensate for catalyst deactivation, and deactivation of the catalyst in reactor A is compensated for by switching from i), ii), iii) to I), II), III), and optionally back from I), II) III) to i), ii), iii). In such embodiments, the total feasible run-time of the process may be shorter than in embodiments wherein, in addition to switching between the modes, also operating conditions of at least reactor A are adjusted.

In certain preferred embodiments, the process comprises when running I), II), III), i.e. running the process in the second mode, monitoring parameters indicative of reversal of the deactivation of the hydrotreatment catalyst A to receive values, wherein the parameters indicative of reversal of the deactivation of the hydrotreatment catalyst A preferably comprise at least two or more parameters selected from items a. to i. as defined later; comparing the received values with (corresponding) predetermined values; and when the received values reach the (corresponding) predetermined values, switching from I), II), III) back to i), ii), iii). When monitoring parameters indicative of reversal of the deactivation of the hydrotreatment catalyst A, the predetermined values may be different from the predetermined values when monitoring deactivation of the hydrotreatment catalyst A, although the parameters indicative of the deactivation of the hydrotreatment catalyst A and reversal of the deactivation of the hydrotreatment catalyst A may be the same.

These variants of the present process may comprise, after switching from I), II), III) back to i), ii), iii), adjusting operating conditions in reactor A and/or reactor B to compensate for the deactivation of the catalyst. Again, operating conditions may be adjusted to their feasible extreme(s) and then switched from i), ii), iii) to I), II), III) or alternatively, operating conditions may be adjusted to some extent and switching from i), ii), iii) to I), II), III) performed already before extremes are reached.

An example sequence applying these variants comprises running the process in the first mode, and adjusting operating conditions of reactor A and/or reactor B at most to feasible extremes; thereafter switching to the second mode and adjusting operating conditions of reactor A and/or reactor B at most to feasible extremes; then switching back, i.e. from I), II), III) to i), ii), iii), and running until targeted performance of the process is no longer met despite of adjusting operation conditions, and then switching from i), ii), iii) to I), II), III) and running until targeted performance of the process no longer met despite of adjusting operation conditions, which leads to regeneration or change of the catalyst(s).

The first and the second mode of operation, in combination with appropriate temperatures and other operating conditions in reactor A and reactor B (where applicable), provide a possibility to adjust the process flexibly in order to meet and even optimise the targeted yields and/or qualities of various recovered components. In the present process according to the third aspect, the operating conditions of reactor A may be adjusted as described in the foregoing.

In certain preferred embodiments, when running i), ii), iii), a portion of the hydrocarbon feed is fed as part of the reactor B feed to the hydroprocessing in reactor B; and/or, when running I), II), III), a portion of the hydrocarbon feed is fed as part of the reactor A feed to the hydroprocessing in reactor A. In these embodiments said portions may be obtained e.g. by simply splitting the hydrocarbon feed between reactor A and reactor B, using e.g. a fixed or preferably gradually adjusted ratio. These embodiments provide further flexibility for operating the present process, and possibility to adjust the process even more precisely in order to meet targeted yields and qualities of more than one recovered component at a time. In this way it may be possible to avoid situations where targeted yield and quality of one recovered fuel component are met but at the expense of producing over-quality of another recovered fuel component (e.g. producing a diesel fuel component having unnecessarily low cloud point). Splitting the hydrocarbon feed may also be beneficial when switching the process from i), ii), iii) to I), II), III), or from I), II), III) to i), ii), iii), providing a smooth transition between the modes.

In these embodiments, the amount of the portion of the hydrocarbon feed fed to reactor B in the first mode or to reactor A in the second mode may vary but is generally just a minor portion (outside of the switching). For example, the portion of the hydrocarbon feed optionally fed to reactor B in the first mode or to reactor A in the second mode may be less than 50 wt-%, preferably less than 30 wt-%, more preferably less than 10 wt-% of the total weight of the hydrocarbon feed.

In certain preferred embodiments of the process according to the first aspect, the switching comprises, when running i), ii), iii), feeding a gradually decreasing portion of the hydrocarbon feed and a gradually increasing portion of the degassed hydroprocessing effluent B as part of the reactor A feed to reactor A and at the same time a gradually increasing portion of the hydrocarbon feed and a gradually decreasing portion of the degassed hydroprocessing effluent A as part of the reactor B feed to reactor B, until the process is run according to I), II), III). In certain preferred embodiments of the process according to the second aspect, the switching comprises, when running i), ii), iii), feeding a gradually decreasing portion of the hydrocarbon feed and a gradually increasing portion of the recycle stream as part of the reactor A feed to reactor A and at the same time a gradually increasing portion of the hydrocarbon feed and a gradually decreasing portion of the recycle stream as part of the reactor B feed to reactor B, until the process is run according to I), II, III).

In the present process parameters indicative of deactivation of the hydrotreatment catalyst A are monitored to receive values, the comparison of which to predetermined values may be used, in addition to initiation of any switching, to select appropriate adjustments for operating conditions in reactor A and optionally in further process units.

The parameters indicative of deactivation of the hydrotreatment catalyst A may be selected freely, but preferably at least two of the monitored parameters belong to one or more of the following items a, b, c, d, e, f, g, h, and/or i. For example, two of the parameters may be selected from one of the items and optionally more parameters from the other items, or one of the parameters may be selected from one of the items and at least one more parameter from the other items. Hence, in certain preferred embodiments, the parameters indicative of deactivation of the hydrotreatment catalyst A comprise at least two or more of:
a. content of nitrogen impurities in the reactor A feed or in the hydrocarbon feed (assessed as elemental nitrogen), and optionally content of at least one or more additional impurity selected from S, O, P, Si, CI, Fe, alkali metals, alkaline earth metals, and/or coke-forming compounds in the reactor A feed or in the hydrocarbon feed; All of said species or impurities are known catalyst deactivators, particularly deactivators of non-sulphided bifunctional hydrotreatment catalysts comprising noble metal(s). Also, said species or impurities are commonly present in varying amounts in feeds derived from vegetable oils, animal fats, microbial oils, thermally and/or enzymatically liquefied organic waste and residues as well as in feeds derived from Fischer-Tropsch process, and may hence in certain embodiments be carried-over in varying amounts to and be present in the hydrocarbon feed. Typically, the elemental impurities are not present as such in the hydrocarbon feed, but contents thereof indicate presence of compounds comprising them. Elemental impurities and coke-forming compounds are determined by standard laboratory analyses. Increased content of any of these impurities may lead to increased catalyst deactivation.
b. content of NH₃ and optionally content of H₂S in the gaseous phase of the hydroprocessing effluent A; Typically, increased content of e.g. NH₃ in the gaseous phase of the hydroprocessing effluent indicates increased catalyst exposure to nitrogen impurities, and may thus be a sign of increased catalyst deactivation. Similarly, increased content of H₂S in the gaseous phase of the hydroprocessing effluent indicates increased catalyst exposure to sulphur and may be a sign of increased catalyst deactivation.
c. physico-chemical characteristic(s) of the degassed hydroprocessing effluent A, preferably at least one or more of a cloud point, freezing point, pour point, cold filter plugging point, kinematic viscosity, density, and/or a distillation characteristic; Increase in any of cloud point, freezing point, pour point, cold filter plugging point, kinematic viscosity, and/or density may indicate increased catalyst deactivation. Distillation characteristics, wherein an increase may be indicative of catalyst deactivation, comprise T5, T50, FBP etc. As to further distillation characteristics, a shift of boiling point distribution to higher boiling compounds, may be indicative of catalyst deactivation.
d. compositional characteristic(s) of the degassed hydroprocessing effluent A, preferably at least one or more of content of isoparaffins, content of C8-C14 hydrocarbons, content of multiple-branched isoparaffins, and/or content of C1-C4 hydrocarbons in the degassed hydroprocessing effluent A; Decrease in content of isoparaffins, multiple-branched isoparaffins, and/or C8-C14 hydrocarbons may indicate increased catalyst deactivation. Content of C1-C4 hydrocarbons may decrease along with increased catalyst deactivation. However, increasing operating temperature to compensate catalyst deactivation may lead to increase in C1-C4 hydrocarbon content.
e. yield of at least one or more of the recovered liquid transportation fuel component(s) and/or the separated recycle stream, preferably yield of an aviation fuel component; Increased yield of higher boiling hydrocarbons and increased volume of the recovered recycle stream may indicate increased catalyst deactivation.
f. physico-chemical characteristic(s) of at least one or more of the recovered liquid transportation fuel component(s) and/or of the separated recycle stream, preferably at least one or more of a cloud point, freezing point, pour point, cold filter plugging point, kinematic viscosity, density, research octane number (RON), cetane number, and/or a distillation characteristic; A decrease in cetane may indicate increased catalyst deactivation. Among distillation characteristics, an increase e.g. in T5, T50, T95, FBP etc. may be indicative of catalyst deactivation. As to further distillation characteristics, a shift of boiling point distribution to higher boiling compounds, may be indicative of catalyst deactivation. To some extent the physico-chemical characteristics may be indicative of, or correlate with, the content of isoparaffins or multiple-branched isoparaffins in the recovered liquid transportation fuel component(s) and/or the separated recycle stream.
g. compositional characteristic(s) of at least one or more of the recovered liquid transportation fuel component(s) and/or the recycle stream, preferably content of isoparaffins and/or content of multiple-branched isoparaffins in one or more of the recovered liquid transportation fuel component(s) and/or of the separated recycle stream; Decrease in content of isoparaffins, multiple-branched isoparaffins, and C8-C14 hydrocarbons may indicate increased catalyst deactivation. To some extent the content of isoparaffins or multiple-branched isoparaffins can be indicative of, or correlate with, the physico-chemical characteristics of the recovered liquid transportation fuel component(s) and/or the separated recycle stream.
h. temperature difference over the reactor A, or over one or more catalyst bed therein; Said temperature differences are relatively easy to monitor, and a decrease in temperature difference may indicate increased catalyst deactivation.
   and/or
   i. operating condition(s) in the reactor A selected from temperature, pressure, weight hourly space velocity (WHSV), H₂ to reactor A feed ratio, and/or H₂ partial pressure at the inlet of the reactor A. For example, temperature and/or WHSV and/or pressure may already have needed adjusting during continued operation of the process to compensate for the hydrotreatment catalyst deactivation, so increased temperature, decreased WHSV, and/or increased pressure may indicate increased catalyst deactivation. Conveniently the temperature in the reactor is as measured at the reactor inlet.

Active sites of the hydrotreatment catalyst may be occupied, and catalyst pores blocked by the impurities and coke, so that the sites are not available for catalysis anymore. For example, coke is caused by coke-forming compounds, such as olefins, aromatics and naphthenes, and coke formation may be enhanced by further impurities, when present in the hydrocarbon feed or reactor feed(s). For impurities causing reversible catalyst deactivation it may be sufficient to monitor the total content of such impurity or impurities in the feed, as that may give indication of expected catalyst deactivation. For some other impurities, especially those causing irreversible catalyst deactivation, one may need to monitor the content of one or more of such impurities in the hydrocarbon feed, and calculate as the received value the cumulative amount thereof the hydrotreatment catalyst has encountered at a given time point from the beginning of the run, and to compare the received value to a value that has been predetermined for example based on historical data or a model based on historical data to reflect too high hydrotreatment catalyst deactivation so that meeting the target yield and/or quality of the desired fuel component(s) can no longer be foreseen.

In certain preferred embodiments, the process comprises monitoring at least two, or at least three of the above-mentioned parameters indicative of deactivation of the hydrotreatment catalyst A. For example, when the hydroprocessing in reactor A is HI, good cold properties (e.g. cloud point, pour point, subzero viscosity) of the degassed hydroprocessing effluent A may be achieved due to increased amount of shorter carbon chains caused by increased cracking taking place in reactor A, instead of sufficient degree of isomerization. Thus, it may be beneficial to monitor at least two or three or even more parameters in order to obtain better understanding of the status of the HI catalyst deactivation. For example, one or more operating condition(s) in the reactor A, and one or more physico-chemical characteristic(s) and/or yields of the recovered liquid transportation fuel component(s) are conveniently monitored, and give enhanced understanding on the deactivation status of the hydrotreatment catalyst A. In certain exemplary embodiments, the monitored parameters indicative of deactivation of the hydrotreatment catalyst A comprise temperature, and optionally also WHSV, in reactor A, and for a recovered diesel fuel component yield, at least one or more distillation characteristics, and/or cloud point, and/or for a recovered aviation fuel component yield, at least one or more distillation characteristics, and/or freezing point.

Monitoring parameters indicative of hydrotreatment catalyst deactivation, or parameters indicative of reversal of hydrotreatment catalyst deactivation, may be performed continuously, repeatedly, continually, periodically, intermittently, discontinuously, or be one-time monitoring. The monitoring of parameters indicative of hydrotreatment catalyst deactivation, or parameters indicative of reversal of hydrotreatment catalyst deactivation, may be performed on-line, for example using a sensor or sensors in any of the process streams, feed tanks, and/or product tanks, including slip-stream arrangements, or off-line, based on samples taken from any of the process streams, feed tanks, and/or product tanks. The frequency and way of monitoring one or more parameters may be performed independently from other monitored parameters, i.e. certain parameters may for example be monitored continuously on-line, while others may for example be monitored periodically off-line.

It is well known to a skilled person how to select the hydroprocessing conditions, taking into account the selected hydrotreatment catalyst, composition of the reactor feed, and targeted hydroprocessing type such as hydroisomerisation, hydrocracking, hydrodearomatisation, and/or hydropolishing. To a certain extent, catalyst age or catalyst deactivation may also be taken into account when selecting or adjusting operating conditions in reactor A and B (where applicable). This applies similarly for the operating conditions in reactor A of the third aspect. During continued operation, the hydrotreatment catalyst(s) deactivate gradually, for example due to impurities in process streams and coking. As the catalyst(s) deactivate, catalyst activity is reduced and selectivity affected, and typically at some point, desired properties of the hydroprocessing effluents and/or the recovered liquid transportation fuel component(s) are no longer reached. When that happens, at least one or more operating conditions of reactor A and/or reactor B, including for example temperature, pressure, WHSV, and/or H₂ partial pressure at the inlet of reactor A and/or reactor B, may be adjusted, for example within the operating condition ranges specified in the following, to compensate for catalyst deactivation so that desired properties of the hydroprocessing effluents, and/or the recovered liquid transportation fuel component(s), may be reached again. Typically, this means increasing the temperature and/or decreasing the WHSV and/or adjusting the H₂ to hydrocarbon feed or reactor feed ratio and/or increasing the pressure. However, there are limits beyond which e.g. the temperature cannot be increased and/or WHSV decreased without compromising yield and/or quality of the recovered liquid transportation fuel component(s). For example, when operating temperature is increased to a high enough value, thermal cracking side reactions are increased, increasing the formation of gaseous hydrocarbons, and hence decreasing yield of liquid products. Accordingly, it is usually beneficial to start operating the reactors in the beginning of the run at a low temperature, using for example lowest feasible temperature in the specified range, as that provides the widest window for increasing the temperature upon gradual catalyst deactivation. Also, the hydrotreatment catalyst B deactivates gradually during continued operation in the first mode of the process according to the first and the second aspects, although typically at lower pace compared to the hydrotreatment catalyst A which is subjected to higher impurity levels. During continued operation in the second mode of the process according to the first and the second aspects, it is the hydrotreatment catalyst B that is subjected to higher impurity levels and the hydrotreatment catalyst A typically deactivates at lower pace compared to the hydrotreatment catalyst B. So similar considerations apply to adjusting operating conditions of reactor B.

In certain preferred embodiments, the present process comprises, when running i), ii), iii), feeding a portion of the hydrocarbon feed as part of the reactor B feed to the hydroprocessing in reactor B; and/or, when running I), II), III), feeding a portion of the hydrocarbon feed as part of the reactor A feed to hydroprocessing A in reactor A; and/or, when running i), ii), iii) or I), II), III), separating from the fractionation a side cut and feeding the side cut to reactor A and/or B as part of the respective reactor feed. In addition to the possibility to adjust the operating conditions in reactor A and/or in reactor B, these embodiments provide further enhanced flexibility for operating the process, including possibility to adjust the process e.g. according to the fluctuations in the characteristics of the hydrocarbon feed, according to the demand and/or quality of the recovered one or more liquid transportation fuel components, and/or according to the changing activity and/or selectivity of the hydrotreatment catalysts throughout their lifetime.

The desired properties of the hydroprocessing effluents, the recovered liquid transportation fuel component(s), and/or the separated recycle stream include compositional and/or physico-chemical characteristics, for example at least one or more of wt-% isoparaffins, wt-% multiple-branched isoparaffins, wt-% of hydrocarbons within a certain carbon number range, cloud point, freezing point, pour point, cold filter plugging point, kinematic viscosity, density, and/or a distillation characteristic. The properties of the hydroprocessing effluents, the recovered liquid transportation fuel component(s), and/or the separated recycle stream may change in the course of operating the process due to deactivation of the hydrotreatment catalysts, but also due to other reasons such as change in the hydrocarbon feed composition. Thus, one or more parameters indicative of deactivation of the hydrotreatment catalysts, and/or indicative of properties of the hydrocarbon feed, may be monitored to receive one or more values and the received values may be compared to predetermined adjustment values. Predetermined adjustment values are typically closer to the initial process settings than the predetermined values used for triggering the switching from the first mode to the second mode.

Hence, according to certain embodiments, the process comprises monitoring at least one or more parameters indicative of deactivation of the hydrotreatment catalyst A to receive at least one or more values; and/or monitoring at least one or more parameters indicative of deactivation of the hydrotreatment catalyst B to receive at least one or more values; and/or monitoring at least one or more parameters indicative of the properties of the hydrocarbon feed to receive at least one or more values; comparing the received values to predetermined adjustment values, and based on said comparison, adjusting at least one or more operating conditions in reactor A and/or in reactor B, and/or adjusting composition of the reactor A feed, and/or of the reactor B feed, preferably adjusting at least one or more of temperature, pressure, and/or weight hourly space velocity (WHSV) in reactor A and/or reactor B, H₂ to reactor A feed and/or reactor B feed ratio, and/or H₂ partial pressure at the inlet of reactor A and/ reactor B, the split-ratio of the hydrocarbon feed between reactor A and reactor B, and/or the weight-ratio in the reactor A feed and/or reactor B feed of a side cut optionally separated from the fractionation, more preferably increasing the temperature, and/or the pressure in reactor A and/or reactor B, and/or decreasing the WHSV in reactor A and/or reactor B, and/or adjusting the split-ratio of the hydrocarbon feed between reactor A and reactor B.

The adjustment of operating conditions to obtain or maintain desired properties of the hydroprocessing effluents, the recovered liquid transportation fuel component(s), and/or the separated recycle stream may be done for example based on historical process data, a model, e.g. a theoretical model or a model based on historical process data, and/or one or more parameters relating to, or indicative of, properties of one or more of the hydroprocessing effluent A, the hydroprocessing effluent B, the recovered liquid transportation fuel component(s), and/or the separated recycle stream. For example, the adjusting may be based on historical process data from earlier processes run using similar feed and similar qualitative and quantitative expectations for the recovered liquid transportation fuel components, for example using a model and/or operating condition profile based on historical process data.

The hydrocarbon feed used in the present process comprises nitrogen impurities, with the exception of the hydrocarbon feed used in the second mode of the process according to certain embodiments of the third aspect.

The hydrocarbon feed used in the second mode of the process according to the third aspect is preferably a hydrocarbon feed essentially without nitrogen impurities (comprising essentially no nitrogen impurities). Alternatively, the hydrocarbon feed used in the second mode of the process according to the third aspect may comprise nitrogen impurities and the low nitrogen content of the reactor A feed in the second mode of the process according to the third aspect is achieved by diluting the hydrocarbon feed for example by a side cut or recycle stream from the fractionation. Preferably, in the process according to the third aspect, the reactor A feed essentially consists in the first mode of the hydrocarbon feed comprising nitrogen impurities and in the second mode of a hydrocarbon feed comprising essentially no nitrogen impurities.

Other than nitrogen impurities, the hydrocarbon feed used in the second mode of the process according to the third aspect is a hydrocarbon feed as described herein.

Nitrogen impurities are known to deactivate acid sites of catalysts in a reversible way. Other than nitrogen and possible other impurities, the hydrocarbon feed may comprise paraffins, naphthenes, aromatics and/or olefins. Typically, the hydrocarbon feed comprises at least 90 wt-%, preferably at least 95 wt-%, more preferably at least 98 wt-%, even more preferably at least 99 wt-% hydrocarbons of the total weight of the hydrocarbon feed. Preferably, the hydrocarbon feed comprises oxygen at most 1.0 wt-%, preferably at most 0.8 wt-%, more preferably at most 0.5 wt-%, expressed as elemental oxygen (ASTM D5622-2017), of the total weight of the hydrocarbon feed.ln certain preferred embodiments, the hydrocarbon feed comprises at least 60 wt-% paraffins of the total weight of the hydrocarbon feed, of which paraffins at most 30 wt-% are isoparaffins. This means that when the hydrocarbon feed comprises 60 wt-% paraffins of the total weight of the hydrocarbon feed, then at most 30 wt-% of the total weight of paraffins in the hydrocarbon feed i.e. at most 18 wt-% of the total weight of the hydrocarbon feed, are isoparaffins. The hydrocarbon feed may be a highly paraffinic hydrocarbon feeds. The hydrocarbon feed may comprise at least 60 wt-%, preferably at least 70 wt-%, more preferably at least 80 wt-%, even more preferably at least 90 wt-% paraffins of the total weight of the hydrocarbon feed. The hydrocarbon feed of the present disclosure may comprise even at least 95 wt-% paraffins of the total weight of the hydrocarbon feed or consist essentially of paraffins. The hydrocarbon feed of the present disclosure may contain olefins, preferably less than 5 wt-%, more preferably less than 1 wt-%, based on the total weight of the hydrocarbon feed, as well as aromatics and/or naphthenes, preferably minor amounts thereof.

An advantage of using a highly paraffinic hydrocarbon feed in the present process is that paraffins are hydroprocessed, such as isomerised or hydrocracked, relatively easily and at milder conditions compared to e.g. cyclic hydrocarbons.

Of the paraffins in the hydrocarbon feed of the present disclosure, preferably at most 30 wt-%, further preferably at most 25 wt-%, more preferably at most 20 wt-%, and even more preferably at most 15 wt-% are isoparaffins. For example, of the paraffins in the hydrocarbon feed from 1 wt-% to 30 wt-%, or from 1 wt-% to 20 wt-%, or from 2 wt-% to 30 wt-%, or from 2 wt-% to 20 wt-% may be isoparaffins.

A high weight-ratio (wt-%:wt-%) of n-paraffins to isoparaffins in the hydrocarbon feed may contribute for example to limiting cracking side-reactions during HI as the hydroprocessing in the reactor to which the hydrocarbon feed is fed, i.e. reactor A or reactor B, depending on the mode and aspect according to which the process is ran, as n-paraffins are less prone to crack compared to isoparaffins of same carbon number. However, the presence of a certain amount of isoparaffins in the hydrocarbon feed may still be beneficial. Compared to otherwise similar feeds, but without isoparaffin content, hydrocarbon feeds containing a certain amount of isoparaffins may yield liquid transportation fuel component(s) with a higher content of multiple-branched isoparaffins.

Preferably, the hydrocarbon feed of the present disclosure comprises at least 70 wt-%, preferably at least 80 wt-%, more preferably at least 90 wt-%, even more preferably at least 95 wt-% hydrocarbons having a carbon number within the range from C12 to C30, of the total weight of the hydrocarbon feed. In certain particularly preferred embodiments, the hydrocarbon feed comprises at least 70 wt-%, preferably at least 80 wt-%, more preferably at least 90 wt-%, even more preferably at least 95 wt-% hydrocarbons having a carbon number within the range from C14 to C22, of the total weight of the hydrocarbon feed. Hydrocarbon feeds having a high content of C12 to C30 hydrocarbons are preferred as they allow good yields of two or more liquid transportation fuel components of different kinds. C14 to C22 hydrocarbons are particularly preferred for the same reason, and also because they are readily available for example from conventional HDO of vegetable oils, animal fats and/or microbial oils, comprising fatty acids.

To increase the yield of aviation fuel component and/or diesel fuel component, it is particularly preferred that the hydrocarbon feed comprises at least 50 wt-%, preferably at least 60 wt-%, more preferably at least 70 wt-%, even more preferably at least 80 wt-% or at least 90 wt-% C16+ paraffins (paraffins having a carbon number of at least C16) of the total weight of paraffins in the hydrocarbon feed. In certain embodiments, the hydrocarbon feed comprises at least 50 wt-% C17+ paraffins (paraffins having a carbon number of at least C17) of the total weight of paraffins in the hydrocarbon feed. This kind of compositions are attainable by suitably selecting heavier hydrocarbon feeds, particularly by suitably selecting feedstocks, such as oxygenated hydrocarbons, for producing them. Examples of suitable heavier oxygenated hydrocarbon feeds include oil from energy crops such as Brassica species, algal oils, crude tall oil (CTO), tall oil fatty acids (TOFA), tall oil pitch (TOP), and/or lignocellulosics-originating feeds. C17+ paraffins are also obtainable by Fischer-Tropsch conversion of syngas.

The hydrocarbon feed may comprise any suitable hydrocarbon composition or combinations thereof. Preferably, in the present process the step of providing the hydrocarbon feed comprises subjecting a hydrotreatment feed to a catalytic hydrotreatment, preferably subjecting an oxygenated hydrocarbon feed to catalytic hydrodeoxygenation, to obtain a hydrotreatment effluent, wherein the hydrotreatment feed preferably comprises at least one or more of vegetable oil(s), animal fat(s), microbial oil(s), thermally liquefied organic waste and residue(s), and/or enzymatically liquefied organic waste and residue(s), and/or subjecting a syngas to a Fischer-Tropsch (FT) conversion to obtain a FT effluent; subjecting the hydrotreatment effluent and/or the FT effluent to a gas-liquid separation, and optionally to a hydrocarbon feed fractionation to provide the hydrocarbon feed. By gas-liquid separation is meant removing at least compounds that are gaseous at NTP. Examples of thermally liquefied organic waste and residues and/or enzymatically liquefied organic waste and residues include thermally and/or enzymatically liquefied biomass waste and residues, municipal solid waste and/or waste plastics.

While e.g. **FT** effluents of fossil origin are readily available (in addition to **FT** effluents of renewable origin), preferably the hydrocarbon feed of the present disclosure is at least partially renewable, i.e. comprises biogenic components. In certain preferred embodiments, the biogenic carbon content of the hydrocarbon feed is at least 50 wt-%, preferably at least 70 wt-%, more preferably at least 90 wt-%, further preferably at least 95 wt-%, or even about 100 wt-%, based on the total weight of carbon (TC) of the hydrocarbon feed, preferably as determined according to EN 16640 (2017).

In certain embodiments, the hydrocarbon feed comprises or consists essentially of a degassed Fischer-Tropsch effluent, or a fraction thereof. Fischer-Tropsch process involves a catalytic conversion of syngas comprising carbon monoxide and hydrogen, typically to a substantially Gaussian distribution of hydrocarbon chains, mainly n-paraffins, having a wide carbon chain length distribution, such as from C2 to C100+, typically from about C5 to about C50. FT effluents are typically highly paraffinic comprising mainly n-paraffins. The catalytic FT conversion of syngas may result in a minor degree of isomerisation, the FT effluent typically comprising about 10 wt-% or less isoparaffins of the total weight of the FT effluent. The syngas used in the FT process may be produced from renewable materials, natural gas, coal, or a combination thereof.

Preferably, the hydrocarbon feed of the present disclosure comprises or consists essentially of a degassed hydrotreatment effluent, or a fraction thereof, particularly as obtained by catalytic hydrotreatment of a hydrotreatment feed comprising at least one or more of vegetable oil(s), animal fat(s), microbial oil(s), thermally liquefied organic waste and residue(s), and/or enzymatically liquefied organic waste and residue(s), more preferably at least one or more of vegetable oil(s), animal fat(s), and/or microbial oil(s), to obtain a hydrotreatment effluent, followed by subjecting the hydrotreatment effluent to a gas-liquid separation, and optionally to a hydrocarbon feed fractionation to provide the degassed hydrotreatment effluent, or a fraction thereof. Nitrogen impurities are commonly present in varying amounts in hydrotreatment feeds comprising vegetable oil(s), animal fat(s), microbial oil(s), thermally liquefied organic waste and residue(s), and/or enzymatically liquefied organic waste and residue(s), and may be carried-over in varying amounts to and be present in the degassed hydrotreatment effluent, or a fraction thereof.

By the term "catalytic hydrotreatment", is meant a catalytic process of treating organic material by means of molecular hydrogen. Preferably, catalytic hydrotreatment removes oxygen from organic oxygen compounds as water, i.e. hydrodeoxygenation (HDO), sulphur from organic sulphur compounds as dihydrogen sulphide (H₂S), i.e. hydrodesulphurisation (HDS), nitrogen from organic nitrogen compounds as ammonia (NH₃), i.e. hydrodenitrogenation (HDN), halogens, for example chlorine from organic chloride compounds as hydrochloric acid (HCI), i.e. hydrodechlorination (HDCI), and/or metals by hydrodemetallization, and/or hydrogenates olefinic bonds if present in the hydrotreatment feed. Depending on the composition of the hydrotreatment feed, different reactions may occur and/or prevail.

Preferably, the hydrocarbon feed of the present disclosure comprises or consists essentially of a degassed hydrodeoxygenation (HDO) effluent, or a fraction thereof, particularly as obtained by catalytic HDO of an oxygenated hydrocarbon feed comprising at least one or more of vegetable oil(s), animal fat(s), and/or microbial oil(s), to obtain a HDO effluent, followed by subjecting the HDO effluent to a gas-liquid separation, and optionally to a hydrocarbon feed fractionation to provide the degassed HDO effluent, or a fraction thereof. Nitrogen impurities are commonly present in varying amounts in oxygenated hydrocarbon feeds comprising vegetable oil(s), animal fat(s), and/or microbial oil(s) and may be carried-over in varying amounts to and be present in the degassed HDO effluent, or a fraction thereof.

In certain embodiments, providing the hydrocarbon feed comprises subjecting an oxygenated hydrocarbon feed, preferably in a hydrodeoxygenation (HDO) reactor, to hydrodeoxygenation (HDO) in the presence of a hydrodeoxygenation catalyst to obtain a hydrodeoxygenation effluent, and subjecting the hydrodeoxygenation effluent to gas-liquid separation and optionally to hydrocarbon feed fractionation to obtain as the hydrocarbon feed the degassed hydrodeoxygenation effluent or a fraction thereof, wherein the hydrodeoxygenation is preferably conducted at a temperature within a range from 200 °C to 500 °C, a pressure within a range from 1 MPa to 20 MPa, a H₂ partial pressure at the inlet of the reactor within a range from 1 MPa to 20 MPa, a weight hourly space velocity within a range from 0.1 to 10 kg oxygenated hydrocarbon feed per kg catalyst per hour, and a H₂ to oxygenated hydrocarbon feed ratio within a range from 50 to 2000 normal liters H₂ per liter oxygenated hydrocarbon feed. Preferably, the oxygenated hydrocarbon feed comprises at least one or more of vegetable oil(s), animal fat(s), and/or microbial oil(s). Hydrodeoxygenation is preferably conducted as described in prior art publications, such as FI100248B, EP1741768A1, EP2155838B1, or FI129220 B1.

The optional hydrocarbon feed fractionation is typically conducted in a fractionation unit different from the fractionation unit(s) used to recover the liquid transportation fuel component(s) and/or to separate the recycle stream. The optional hydrocarbon feed fractionation may be regarded separate from the fractionation of the present process, which fractionation of the present process refers to or comprises fractionation to recover the liquid transportation fuel component(s) and/or to separate the recycle stream.

Preferably, the HDO catalyst is a sulphided catalyst comprising at least one or more metals from Group VIII of the Periodic Table, and/or from Group VIB of the Periodic Table, preferably at least one or more of Ni, Mo, W, and/or Co, even more preferably at least one or more of Ni, and/or Co and Mo, and/or W, such as NiMo, CoMo, NiCoMo, NiW, NiMoW. These catalysts are efficient, readily available, and tolerate typical impurities of fatty feedstocks well. If using a catalyst having hydrodewaxing properties, such as a catalyst containing NiW, in the hydrodeoxygenation as the hydrodeoxygenation catalyst or as a co-catalyst, HDO effluent with somewhat elevated isoparaffin content may be attained.

Preferably, in the first mode of the present process, the reactor A feed consists essentially of the hydrocarbon feed, and optionally one or more streams from the process. In the context of the present disclosure, molecular hydrogen (H₂) fed to the reactor(s) for hydroprocessing is excluded from the definition of the reactor feed(s), i.e. it is not consider herein part of the reactor A feed or reactor B eed.

Preferably, in the first mode of the process according the first aspect, the reactor B feed consists essentially of the degassed hydroprocessing effluent A, and optionally one or more further streams from the process and/or a portion of the hydrocarbon feed. Preferably, in the first mode of the process according to the second aspect, the reactor B feed consists essentially of the recycle stream, and optionally one or more further streams from the process and/or a portion of the hydrocarbon feed.

Preferably, in the second mode of the process according to the first aspect and in the second mode of the process according to the second aspect, the reactor B feed consists essentially of the hydrocarbon feed, and optionally one or more streams from the process.

Preferably, in the second mode of the process according to the first aspect, the reactor A feed consists essentially of the degassed hydroprocessing effluent B, and optionally one or more further streams from the process and/or a portion of the hydrocarbon feed. Preferably, in the second mode of the process according to the second aspect, the reactor A feed consists essentially of the recycle stream, and optionally one or more further streams from the process and/or a portion of the hydrocarbon feed.

Said one or more (further) stream from the process may for example be a side cut and/or recycle stream from the fractionation, and/or a portion of the degassed hydroprocessing effluent A and/or B, depending on the process mode and aspect. Preferably, in the process according to the first and/or the second aspects, the feed to the first reactor in a process mode (reactor A in the first mode and reactor B in the second mode) comprises at least 0.4 w-ppm nitrogen, expressed as elemental nitrogen (ASTM D4629-17), of the total weight of the reactor feed, or at least 0.6 w-ppm, or at least 1.0 w-ppm, or even at least 1.5 w-ppm or at least 2.0 w-ppm nitrogen.

In certain embodiments the hydroprocessing in reactor A and in reactor B are selected, independently from each other, from at least one or more of hydroisomerisation, hydrocracking, hydrodearomatisation and/or hydropolishing; preferably the hydroprocessing in reactor A and in reactor B is hydroisomerisation, or the hydroprocessing in reactor A and reactor B is hydrocracking, or the hydroprocessing in reactor A is hydroisomerisation and the hydroprocessing in reactor B is hydrocracking, or the hydroprocessing in reactor A is hydrocracking and the hydroprocessing in reactor B is hydroisomerisation; more preferably the hydroprocessing in reactor A and reactor B is hydroisomerisation. In other words, preferably the hydroprocessing in reactor A and in reactor B are selected, independently from each other, from at least one or more of hydroisomerisation and/or hydrocracking.

Similarly, in certain embodiments of the process according to the third aspect, the hydroprocessing in reactor A is selected from at least one or more of hydroisomerisation, hydrocracking, hydrodearomatisation and/or hydropolishing, preferably the hydroprocessing in reactor A is hydroisomerisation or hydrocracking, more preferably the hydroprocessing in reactor A is hydroisomerisation. The hydroprocessing in reactor A in the process according to the third aspect may be as herein described for reactor A and/or reactor B.

When the hydroprocessing in reactor A and in reactor B are of same type, the change in quality and/or yield of the recovered liquid transportation fuel component(s) after switching may be of lower magnitude (compared to embodiments where the hydroprocessing in reactor A and reactor B are of different type) or even unnoticeable. When the hydroprocessing in reactor A and reactor B is hydrocracking, enhanced aviation and gasoline fuel component yields may be obtained, even towards the end of the hydrotreatment catalyst lifetime. When the hydroprocessing in reactor A and reactor B is hydroisomerisation, enhanced aviation and diesel fuel component quality and yield may be obtained, even towards the end of the hydrotreatment catalyst lifetime. When the hydroprocessing in reactor A is hydroisomerisation and the hydroprocessing in reactor B is hydrocracking, or vice versa, enhanced aviation and diesel fuel component quality and enhanced aviation and gasoline fuel component yields may be obtained, even towards the end of the hydrotreatment catalyst lifetime.

In certain preferred embodiments, the hydroprocessing in reactor A and/or reactor B is hydroisomerisation. Preferably, the hydroprocessing in reactor A and/or reactor B is hydroisomerisation conducted at a temperature within a range from 200 °C to 500 °C, preferably from 230 °C to 500 °C, more preferably from 250 °C to 450 °C, even more preferably from 280 °C to 400 °C, a pressure within a range from 1 MPa to 10 MPa, preferably from 2 MPa to 8 MPa or from 3 MPa to 10 MPa, a H₂ partial pressure at the inlet of reactor within a range from 1 MPa to 10 MPa, preferably from 2 MPa to 8 MPa, a weight hourly space velocity within a range from 0.1 to 10, preferably from 0.2 to 8, more preferably from 0.4 to 6 kg reactor feed per kg catalyst per hour, and a H₂ to reactor feed ratio within a range from 10 to 2000, preferably from 50 to 1000 normal liters H₂ per liter reactor feed.

According to certain embodiments, the hydroprocessing in reactor A and/or reactor B is hydroisomerisation conducted at a temperature within a range from 200 °C to 500 °C, a pressure within a range from 1 MPa to 10 MPa, a H₂ partial pressure at the inlet of reactor within a range from 1 MPa to 10 MPa, a weight hourly space velocity within a range from 0.1 to 10 kg reactor feed per kg catalyst per hour, and a H₂ to reactor feed ratio within a range from 10 to 2000 normal liters H₂ per liter reactor feed. These conditions are particularly beneficial for hydroisomerisation.

According to certain particularly preferred embodiments, the hydroprocessing in reactor A and/or reactor B is hydroisomerisation conducted at a temperature within a range from 230 °C to 500 °C, a pressure within a range from 2 to 8 MPa, a H₂ partial pressure at the inlet of reactor within a range from 2 to 8 MPa, a weight hourly space velocity within a range from 0.2 to 8 kg reactor feed per kg catalyst per hour, and a H₂ to reactor feed ratio within a range from 50 to 1000 normal liters H₂ per liter reactor feed. These conditions are particularly beneficial for hydroisomerisation.

Degree of isomerisation of the hydroprocessing effluent may be improved by increasing severity of the HI e.g. by at least one or more of: decreasing WHSV, increasing temperature, and/or increasing pressure. When using fresh hydrotreatment catalyst, high severity HI conditions may be reached at lower temperature, and/or lower pressure, and/or using higher WHSV (compared to later during the run, when the hydrotreatment catalyst has been deactivated at least to some extent). Towards the end of the hydrotreatment catalyst lifetime higher temperature, and/or higher pressure, and/or lower WHSV may be needed to reach even medium severity HI. In the present context, HI that yields liquid effluents having, as wt-% of paraffins in the liquid effluent, total i-paraffins content within a range from 50 wt-% to 85 wt-% and multiple-branched i-paraffins content at most 25 wt-%, or total i-paraffins content within a range from 85 wt-% to 95 wt-% and multiple-branched i-paraffins content within a range from 25 wt-% to 55 wt-%, or total i-paraffins content at least 95 wt-% and multiple-branched i-paraffins content more than 55 wt-%, is generally regarded as HI of low severity or medium severity or high severity, respectively, although these content ranges are merely for illustrating the order of magnitude, and may overlap to some extent, and vary from case to case.

Preferably, when the hydroprocessing in reactor A and/or reactor B is hydroisomerisation the respective reactor is operated so that the ratio of the wt-% amount of isoparaffins of the total weight of paraffins in the respective hydroprocessing effluent to the wt-% amount of isoparaffins of the total weight of paraffins in the respective reactor feed is at least 2, or at least 4, or at least 6, or at least 8, or at least 10, or at least 12, and/or so that the ratio of the wt-% amount of multiple-branched isoparaffins of the total weight of paraffins in the respective hydroprocessing effluent to the wt-% amount of multiple-branched isoparaffins of the total weight of paraffins in the respective reactor feed is at least 2, or at least 4, or at least 6, or at least 8, or at least 10, or at least 12, and/or so that the (total) respective hydroprocessing effluent comprises C1 to C4 hydrocarbons less than 20 wt-%, or less than 10 wt-%, preferably less than 5 wt-%, more preferably less than 3 wt-% of the total weight of the respective hydroprocessing effluent. These may be achieved especially when operating the respective reactor within the HI operating conditions, and/or using the HI catalyst(s) and/or the hydrocarbon feed as defined herein.

When hydroprocessing effluent (or portion of hydroprocessing effuent) A and/or B, depending on the process mode and aspect, is to be fed to subsequent hydroprocessing, i.e. brought into contact with a subsequent hydroprocessing catalyst, at least compounds that are gaseous at NTP are separated or removed from the respective hydroprocessing effluent(s). This can be done e.g. as a gas-liquid separation, which can be conducted as an integral step within the respective reactor or in a separate gas-liquid separation unit, or as part of the fractionation. Particularly in connection with the process according to the second aspect, separation of at least compounds that are gaseous at NTP may be regarded as part of the fractionation.

Degassing removes for example gaseous impurities from the hydroprocessing effluent(s). This may protect the subsequent reactor from impurities in the hydrocarbon feed and extend the lifetime of the catalyst in the subsequent reactor. Preferably, separation or removal of at least compounds that are gaseous at NTP includes separating or removing NH₃ from hydroprocessing effluent A and/or B. The degassed hydroprocessing effluent A and/or B may contain minor amounts or trace amounts of compounds that are gaseous at NTP, as the separation does not necessarily achieve complete removal of all compounds gaseous at NTP. Degassing also increases hydrogen partial pressure in the subsequent reactor, and the operating conditions in the subsequent reactor are easier to optimize when the reactor feed is less heterogeneous.

When using highly paraffinic hydrocarbon feed, the hydroprocessing effluent A and/or B may comprise at least 60 wt-%, preferably at least 70 wt-%, more preferably at least 80 wt-%, even more preferably at least 90 wt-% or at least 95 wt-% paraffins of the total weight of the hydroprocessing effluent A and/or B. In certain embodiments, the hydroprocessing effluent A and/or B may even consist essentially of paraffins.

Typically, when the hydroprocessing in reactor A and/or reactor B is hydroisomerisation, the respective hydroprocessing effluent may comprise at least 50 wt-%, preferably at least 60 wt-%, more preferably at least 70 wt-%, even more preferably at least 80 wt-% isoparaffins of the total weight of paraffins in the respective hydroprocessing effluent, and optionally multiple-branched isoparaffins at least 5 wt-%, more preferably at least 10 wt-%, even more preferably at least 15 wt-%, or at least 20 wt-% of the total weight of paraffins in the respective hydroprocessing effluent. Typically, the respective hydroprocessing effluent may comprise at most 75 wt-%, or at most 70 wt-%, or at most 60 wt-%, such as at most 50 wt-%, or at most 40 wt-%, sometimes at most 30 wt-%, or even at most 20 wt-% multiple-branched isoparaffins of the total weight of paraffins in the respective hydroprocessing effluent when the hydroprocessing in reactor A and/or B is hydroisomerisation. Typically, the respective hydroprocessing effluent may have a cloud point less than 0 °C, preferably less than -5 °C, more preferably less than -8 °C, even more preferably less than -10 °C, or less than -15 °C (ASTM D 5771-17) when the hydroprocessing in reactor A and/or reactor B is hydroisomerisation.

Presence of multiple-branched isoparaffins in the first obtained hydroprocessing effluent in a process mode may be considered beneficial for example when the subsequent hydroprocessing is hydrocracking as it may contribute beneficially to the degree of effective cracking in the hydroprocessing in the subsequent reactor. When a desired degree of effective cracking, particularly to C8 to C14 hydrocarbons but also to lighter non-gaseous hydrocarbons, is achieved in the hydroprocessing in the subsequent reactor at milder operating conditions, excessive cracking may be avoided, and formation of gaseous hydrocarbons reduced. Without being bound to any theory, it is believed that a multiple-branched isoparaffin is more likely to form upon cracking in the hydroprocessing in the subsequent reactor two branched paraffin molecules instead of one branched and one n-paraffin, hence increasing the isoparaffin content of the second obtained hydroprocessing effluent. An increased content of multiple-branched isoparaffins in the hydroprocessing effluent A and/or B may be considered beneficial in that it may improve the cold properties of the recovered liquid transportation fuel component(s), especially the cold properties of aviation fuel component and/or diesel fuel component, and/or RON of gasoline fuel component.

In certain embodiments, the hydroprocessing in reactor A and/or reactor B is hydrocracking. Preferably, the hydroprocessing in reactor A and/or reactor B is hydrocracking conducted at a temperature within a range from 200 °C to 450 °C, preferably from 220 °C to 430 °C, more preferably from 280 °C to 350 °C, a pressure within a range from 0.4 MPa to 8 MPa, preferably from 1 MPa to 7 MPa, more preferably from 2.5 MPa to 7 MPa, a H₂ partial pressure at the inlet of the reactor within a range from 0.4 MPa to 8 MPa, preferably from 1 MPa to 7 MPa, more preferably from 2.5 MPa to 7 MPa, a weight hourly space velocity within a range from 0.1 to 10, preferably from 0.2 to 8, more preferably from 0.4 to 6, even more preferably from 0.5 to 1.5 kg reactor feed per kg catalyst per hour, and a H₂ to reactor feed ratio within a range from 10 to 2000, preferably from 50 to 1000 normal liters H₂ per liter reactor feed.

According to certain embodiments, the hydroprocessing in reactor A and/or reactor B is hydrocracking conducted at a temperature within a range from 220 °C to 430 °C, a pressure within a range from 1 MPa to 7 MPa, a weight hourly space velocity within a range from 0.2 to 8 kg reactor feed per kg catalyst per hour, and a H₂ to reactor feed ratio within a range from 50 to 1000 normal liters H₂ per liter reactor B feed. In certain preferred embodiments, the hydroprocessing in reactor A and/or reactor B is hydrocracking conducted at a temperature within a range from 280 to 350 °C, a pressure within a range from 2.5 to 7 MPa, a H₂ partial pressure at the inlet of reactor within a range from 2.5 to 7 MPa, a weight hourly space velocity within a range from 0.4 to 6 kg reactor feed per kg catalyst per hour, and a H₂ to reactor B feed ratio within a range from 50 to 1000 normal liters H₂ per liter reactor feed. These conditions are particularly beneficial for hydrocracking.

When operating the hydroprocessing at lower temperatures and sufficiently high pressures, aromatizing side reactions may be better suppressed or even dearomatisation enhanced.

Preferably, when the hydroprocessing in reactor A and/or reactor B is hydrocracking the respective reactor is operated so that the ratio of the wt-% amount of isoparaffins of the total weight of paraffins in the respective hydroprocessing effluent to the wt-% amount of isoparaffins of the total weight of paraffins in the respective reactor feed is at least 0.5, or at least 0.7, or at least 0.8, or at least 0.9, or at least 1.0; and/or so that the ratio of the C8-C14 hydrocarbon content in the respective hydroprocessing effluent to the C8-C14 hydrocarbon content in the respective reactor feed is at least 1.1, preferably at least 1.3, more preferably at least 1.5, even more preferably at least 1.8 or at least 2.0.

In certain preferred embodiments, when the hydroprocessing in reactor A and/or reactor B is hydrocracking the respective reactor is operated so that the ratio of wt-% amount of C1-C4 hydrocarbons formed during hydrocracking in the (total) respective hydroprocessing effluent to the wt-% amount of C1-C4 hydrocarbons in the (total) respective reactor feed is within a range from 1.1 to 5.0, preferably from 1.1 to 4.0, more preferably from 1.2 to 3.0; and/or so that the (total) respective hydroprocessing effluent comprises C1-C4 hydrocarbons less than 20 wt-%, or less than 10 wt-%, or less than 5 wt-% of the total weight of the respective hydroprocessing effluent.

These may be achieved especially when using the reactor feeds as specified in the foregoing, and/or operating the reactor within the hydrocracking operating conditions specified in the foregoing, and/or in the presence of a HC catalyst as defined hereinafter, particularly in the presence of a non-sulphided bifunctional HC catalyst as defined hereinafter, preferably a non-sulphided bifunctional HC catalyst comprising at least one or more Group VIII noble metals, more preferably Pt and/or Pd.

When using highly paraffinic hydrocarbon feed, in embodiments where the hydroprocessing in reactor A and/or reactor B is hydrocracking, the respective hydroprocessing effluent may comprise at least 60 wt-%, preferably at least 70 wt-%, more preferably at least 80 wt-%, even more preferably at least 90 wt-% or at least 95 wt-% paraffins of the total weight of the respective hydroprocessing effluent, or the hydroprocessing effluent may even consist essentially of paraffins.

A preferred feed to the fractionation in steps iii) and/or III) may comprise at least 50 wt-%, preferably at least 60 wt-%, more preferably at least 70 wt-%, even more preferably at least 80 wt-% isoparaffins of the total weight of the feed to the fractionation, and/or at least 3 wt-%, or at least 5 wt-%, or at least 10 wt-%, or at least 15 wt-%, or at least 20 wt-% multiple-branched isoparaffins of the total weight of the feed to the fractionation. Such feeds to the fractionation may be achieved for example when the hydroprocessing in reactor A is HI and the hydroprocessing in reactor B is HC, or when the hydroprocessing in reactor A is HC and the hydroprocessing in reactor B is HI.

Preferably, reactor A is operated at a higher temperature than reactor B, when comparing the temperatures at the reactor inlets. The compared temperatures are the temperatures at the inlet of the reactor A and at the inlet of the reactor B, respectively, at a given time point, including short time intervals. In other words, the comparison is not made in this context between the highest temperatures during the whole run from starting the process to stopping it. Particularly preferably, reactor A is operated at a higher temperature than reactor B and reactor A and reactor B are operated within the operating condition ranges specified herein for the hydroprocessing(s) in question.

By operating reactor A at a higher temperature than reactor B, and especially within the operating condition ranges specified herein, deactivation of the hydrotreatment catalyst A may be better compensated. This is beneficial as hydrotreatment catalyst A is subjected to higher impurity level, particularly to higher nitrogen impurity level, in the first mode, and may remain more deactivated also during the second mode, compared to the hydrotreatment catalyst B. Additionally, operating reactor A at a higher temperature in the second mode may enhance liberation of the reversibly bound impurities from the hydrotreatment catalyst A.

The hydroprocessing in reactor A and/or reactor B may in certain embodiments be hydrodearomatisation. This may be beneficial for example when the hydrocarbon feed has elevated aromatics content, and/or when aromatics are formed during hydroprocessing and/or if ultra-low aromatics content is desired for all or some of the recovered liquid transportation fuel components or for other product fractions possibly recovered from the process. Examples of such other product fractions include product fractions intended for use in cosmetics, pharmaceuticals, and/or other applications involving close contact to humans. In embodiments where the hydroprocessing in reactor A and/or reactor B is HDA, the level of aromatic hydrocarbons can be reduced for example to less than 5000 w-ppm, or less than 300 w-ppm, preferably to less than 100 w-ppm, or less than 50 w-ppm, preferably as determined according to UOP 495-03.

The HDA is preferably conducted at a temperature within a range from 80 °C to 350 °C, a pressure within a range from 3 MPa to 16 MPa, a liquid hourly space velocity within a range from 0.2 to 8 hr⁻¹, and H₂ to reactor feed ratio within a range from 50 to 2000 normal liters H₂ per liter reactor feed.

The hydroprocessing in reactor A and/or reactor B may in certain embodiments be hydropolishing. This may be beneficial for example when the hydrocarbon feed contains at least one or more of organic oxygen impurities, organic sulphur impurities, olefins and/or metal impurities in addition to the nitrogen impurities, and/or if ultra-low contents or even elimination of nitrogen, oxygen, sulphur, olefins and/or metals is desired e.g. for all or some of the recovered liquid transportation fuel components or for other product fractions possibly recovered from the process. In embodiments where the hydroprocessing in reactor A and/or B is hydropolishing, the level of these impurities may in the respective hydroprocessing effluent be reduced for example to less than 50% of their content before the hydropolishing, or even eliminated completely i.e. below the detection limit of the respective analysis method.

The hydropolishing is preferably conducted at a temperature within a range from about 150 °C to 400 °C, a pressure within a range from 0.5 MPa to 15 MPa, a liquid hourly space velocity within a range from 0.5 to 3 hr⁻¹, and H₂ to reactor feed ratio within a range from 50 to 2000 normal liters H₂ per liter reactor feed.

Particularly when run according to the second aspect, the present process may comprise subjecting the hydroprocessing effluent A (in the first mode) and/or hydroprocessing effluent B (in the second mode) to fractionation to separate from the hydroprocessing effluent(s) at least a recycle stream having a T5 temperature (5 vol-% recovered, EN ISO 3405-2019) of 270 °C or higher. The total feed subjected to the fractionation in the second aspect is initially the hydroprocessing effluent A (in the first mode) or the hydroprocessing effluent B (in the second mode), optionally after being subjected to gas-liquid separation integrally within reactor A or B, and as the process is kept running the total feed subjected to the fractionation comprises the hydroprocessing effluent A and the hydroprocessing effluent B, said effluents being preferably combined.

Also process variants or embodiments according to the first and the third aspect benefit from separating from the fractionation a recycle stream as disclosed herein, while running the process in the first mode or in the second mode, or both in the first and the second mode. This may be particularly beneficial when running the process in the second mode, as separating the recycle stream and hydroprocessing it again helps to further compensate the hydrotreatment catalyst deactivation at least to some extent. In embodiments of the first and the third aspects, where the reactor A and/or B feed comprises the recycle stream or a portion thereof, the amount of such portion or stream may vary but is generally just a minor portion of the respective reactor feed. In embodiments according to the first and the third aspect, the reactor A and/or B feed may comprise, based on the total weight of the respective reactor feed, less than 50 wt-%, preferably less than 30 wt-%, more preferably less than 10 wt-% recycle stream. In other words, the recycle stream or a portion thereof may constitute less than 50 wt-%, preferably less than 30 wt-%, more preferably less than 10 wt-% of the total weight of the respective reactor feed in embodiments according to the first and the third aspect.

Preferably, the recycle stream has a T5 temperature of 275 °C or higher. In certain preferred embodiments, the recycle stream has a T5 temperature within a range from 270 °C to less than 300°C, preferably within a range from 270 °C to less than 295 °C, more preferably within a range from 270 °C to less than 290 °C; and/or an initial boiling point (IBP, EN ISO 3405-2019) less than 290 °C, preferably less than 288 °C, more preferably less than 285°C, or less than 280°C. The recycle stream may have the FBP of the total feed subjected to fractionation, i.e. the recycle stream may comprise the heavy bottom of the total feed subjected to fractionation.

The T5 temperature of the recycle stream is preferably selected so that at least a portion of the C16 n-paraffins present in the total feed subjected to the fractionation, if any, are recovered in the recycle stream. Recovering C16 n-paraffins in the recycle stream may be attained by suitably selecting the T5 temperature and/or IBP of the recycle stream. C16 n-paraffins have a boiling point of 287 °C (at normal pressure), but as fractionations, such as distillation separations, are not perfectly sharp, C16 n-paraffins may be recovered over a temperature range around that boiling point. Preferably, the recycle stream comprises C16 n-paraffins.

Recycling at least some of C16 n-paraffins to reactor A and/or B to be subjected to hydroprocessing again is beneficial as C16 n-paraffins have a relatively high melting point. Hence, the presence of C16 n-paraffins in any significant amounts for example in the recovered aviation fuel component might even render the aviation fuel component unusable in aviation fuel compositions, due to poor cold properties, especially poor freezing point, and/or poor, i.e. high, kinematic viscosity at subzero temperatures such as -20 °C or -40 °C. In any event, C16 n-paraffins in the recovered aviation fuel component impairs its cold properties i.e. increases one or more of cloud point, freezing point, pour point, cold filter plugging point, and/or subzero kinematic viscosity, compared to aviation fuel components with less or without C16 n-paraffin content. Preferably, the recycle stream comprises at least 20 wt-% or at least 30 wt-%, more preferably at least 40 wt-% or at least 50 wt-% of the C16 n-paraffins in the total feed subjected to fractionation. The amount of n-paraffins, including C16 n-paraffins, in the total feed subjected to the fractionation may increase upon deactivation of the hydrotreatment catalyst(s), and may also be formed by cracking in reactor A and/or B.

In certain particularly preferred embodiments, the recycle stream comprises at least 85 wt-%, preferably at least 90 wt-%, more preferably at least 95 wt-% C16+ paraffins of the total weight of paraffins in the recycle stream. Subjecting C16+ paraffins to hydroprocessing again in reactor A and/or B is beneficial in that it may increase yield and/or improve quality of aviation fuel component. C16+ n-paraffins (n-paraffins having a carbon number of at least C16) have poor cold properties while C18+ n-paraffins (n-paraffins having a carbon number of at least C18) generally boil outside the aviation fuel boiling range.

Typically, the recycle stream comprises at least 60 wt-%, preferably at least 70 wt-%, more preferably at least 80 wt-%, even more preferably at least 90 wt-% or at least 95 wt-% paraffins of the total weight of the recycle stream, or the recycle stream may even consist essentially of paraffins. This may be achieved especially when using highly paraffinic hydrocarbon feed. In certain particularly preferred embodiments, the recycle stream comprises at least 50 wt-%, preferably at least 60 wt-%, more preferably at least 70 wt-%, or at least 80 wt-% and/or up to 100 wt-%, or at most 98 wt-% isoparaffins of the total weight of paraffins in the recycle stream, and/or at least 5 wt-%, or at least 10 wt-%, preferably at least 15 wt-%, or at least 20 wt-% multiple-branched isoparaffins of the total weight of paraffins in the recycle stream. Typically, the recycle stream comprises at most 95 wt-% or at most 90 wt-%, or at most 80 wt-%, or at most 70 wt-%, such as at most 60 wt-%, or at most 50 wt-%, or at most 40 wt-%, sometimes at most 30 wt-%, or even at most 20 wt-% multiple-branched isoparaffins of the total weight of paraffins in the recycle stream, typically ranging from 5 wt-% to 95 wt-%, preferably from 5 wt-% to 80 wt-%, or from 5 wt-% to 70 wt-%, such as from 10 wt-% to 70 wt-%, or from 15 wt-% to 65 wt-% multiple-branched isoparaffins of the total weight of paraffins in the recycle stream. Typically, the recycle stream has a cloud point less than 0°C, preferably less than -5 °C, more preferably less than -10°C, even more preferably less than -20 °C, further preferably less than -30 °C (ASTM D 5771-17).

This kind of recycle streams have a beneficial composition in view of degree of efficient cracking and/or isomerization degree forming more hydrocarbons boiling in the aviation fuel range, and also in gasoline boiling range, as generally paraffins are more prone to crack and isomerise than cyclic hydrocarbons, longer paraffins are more prone to crack and have room for more branches than shorter paraffins, and isoparaffins are foreseen more prone to crack than n-paraffins, and relative homogeneity of the recycle stream composition can make optimising the subsequent hydroprocessing conditions easier. Additionally, these embodiments facilitate easy separation by mere splitting of e.g. diesel fuel component, marine fuel component, base oil component, and/or transformer oil component from the recycle stream, just to name a few examples of components that may be separated from such recycle streams and recovered as product streams. In such embodiments, a portion of the recycle stream is fed to hydroprocessing in reactor A and/or B as described in the foregoing and another portion of the recycle stream is recovered, preferably the recycle stream is split into a portion that is fed to hydroprocessing in reactor A and/or B and a portion that is recovered.

Both the hydrotreatment catalyst A and the hydrotreatment catalyst B may be arranged in one or more catalyst beds within the respective reactors. The hydrotreatment catalyst A and the hydrotreatment catalyst B may be arranged in at least one or more fixed bed(s), respectively. The process according to the third aspect does typically not comprise a reactor B or hydrotreament catalyst B, but otherwise the hydrotreatment catalyst A in reactor A of the process according to the third aspect may be as described herein and arranged as described herein. Preferably, the hydrotreatment catalyst A in reactor A and the hydrotreatment catalyst B in reactor B are bifunctional hydrotreatment catalysts, more preferably non-sulphided bifunctional hydrotreatment catalysts. In certain preferred embodiments, the hydrotreatment catalyst A and hydrotreatment catalyst B are selected, independently from each other, from any conventionally used bifunctional HI catalysts and any conventionally used bifunctional HC catalysts.

HI catalysts are selective for isomerisation i.e. capable of converting at least a certain amount of n-paraffins to i-paraffins, especially to mono-branched i-paraffins, and/or converting mono-branched i-paraffins to multiple-branched i-paraffins, such as di-branched, and/or tri-branched i-paraffins, even i-paraffins comprising more than three branches. HC catalysts are capable of cracking hydrocarbon molecules. In the present processes particularly desired are HC catalysts capable of effective cracking i.e. cracking that yields non-gaseous (NTP) cracking products, especially as expressed herein as the ratio of the C8 to C14 hydrocarbon content in a reactor effluent to the C8 to C14 hydrocarbon content in the respective reactor feed.

Bifunctional hydrotreatment catalysts, such as bifunctional HI and HC catalysts, comprise metal sites for catalysing (de)hydrogenation reactions and acid sites for catalysing isomerisation and cracking reactions. Bifunctional hydrotreatment catalysts, such as bifunctional HI catalysts and bifunctional HC catalysts, are well known in the field of oil refining and in the field of renewable fuel production. Bifunctional hydrotreatment catalysts are preferred because they have excellent catalytic performance providing synergistic effects between the metal sites and acid sites. Bifunctional hydrotreatment catalysts are also beneficial as access and diffusion of molecules to catalytic sites may be controlled by suitably selecting porosity characteristics of the catalyst, especially pore size, pore dimension, and/or pore interconnectivity in the catalyst.

In certain particularly preferred embodiments, the hydrotreatment catalyst A and the hydrotreatment catalyst B are selected, independently from each other, from non-sulphided bifunctional hydrotreatment catalysts comprising at least one or more noble metals of Group VIII of the Periodic Table, more preferably Pt and/or Pd, and at least one or more acidic porous materials, and wherein the reactor A feed and the reactor B feed each comprises less than 50 wt-ppm, preferably less than 30 wt-ppm, more preferably less than 10 wt-ppm sulphur of the total respective feed (ppm by weight, calculated as elemental S), as determined according to ISO 20846-2019.

The sulphur content may be determined according to ISO 20846-2019 from liquids or according to ASTM-D6667 from gaseous fractions.

Non-sulphided bifunctional catalysts are preferred because they do not require sulphidation during operation for maintaining their activity, and hence the sulphur content of various process streams may be kept low and less efficient H₂S separation and recovery is needed from various process streams. Especially non-sulphided bifunctional catalysts comprising noble-metals may be active at lower temperatures, and show higher selectivity for isomerisation reactions, compared to sulphided catalysts, but are sensitive to deactivation by H₂S.

Preferably, the reactor A feed and the reactor B feed each comprises sulphur less than 50 wt-ppm, preferably less than 30 wt-ppm, more preferably less than 10 wt-ppm (ppm by weight, calculated as elemental S), as determined according to ISO 20846-2019.

Similarly, the reactor A feed of the process according to the third aspect may be a low sulphur feed as described in the foregoing.

A very low sulphur content of the stream entering reactor A is beneficial as sulphur impurities are known to adsorb essentially irreversibly to high coverage in most catalytic processes involving metal catalysts, causing multiple problems. In such embodiments, there is more freedom to choose the catalyst, and liquid transportation fuel component(s) with ultra-low sulphur content are obtainable. Furthermore, since less H₂S will be present, also less corrosion is foreseen in the long-run, and potentially even less stringent corrosion resistance requirements could be applicable for some of the equipment materials.

Various different kinds of bifunctional hydrotreatment catalysts, such as HI and HC catalysts, are commercially available, for example with different metals or metal combinations, different metal loadings, different acid strengths, and/or total acidities, and/or different porosities.

Bifunctional hydrotreatment catalysts, including bifunctional HC catalysts and bifunctional HI catalysts, have similarities in the sense that they contain metal sites that are capable of catalysing (de)hydrogenation of n/i-paraffins to corresponding n/i-olefins, and acid sites that are capable of catalysing protonation of the n/i-olefins to n/i-carbocations, isomerisation of n-carbocations, or i-carbocations further, and/or cracking of n/i-carbocations into lighter n/i-olefin and lighter n/i-carbocation, and deprotonation of n/i-carbocations to n/i-olefins. Hydrogenation of the various n/i-olefins is catalysed again by the metal sites of these bifunctional catalysts to form n/i-paraffins. Whether e.g. isomerisation or cracking reactions prevail at given operating conditions and given feed composition, can be influenced especially by the characteristics of the bifunctional catalyst to be contacted with the feed. Such characteristics of the bifunctional catalyst include, for example, total acidity of the catalyst, number of Brønsted acid sites, strength and/or density of the acid sites, and content of the metal(s) in the catalyst.

Preferably, the hydrotreatment catalyst A and the hydrotreatment catalyst B comprise, independently from each other, at least one or more metals selected from Group VIII of the Periodic Table, preferably from noble metals of Group VIII, more preferably from Pt and/or Pd. Noble metals are preferred as they may provide higher selectivity towards the desired reactions under the operating conditions, and are highly active at lower operating temperatures, compared to catalysts comprising only non-noble metals. High activity at lower temperatures provides a wider temperature range within which temperature may be adjusted, typically increased, during operation. Gradual catalyst deactivation occurring when the process is operated for longer time periods may be compensated to a certain extent by increasing temperature in the reactor.

Preferably, the hydrotreatment catalyst A and the hydrotreatment catalyst B comprise, independently from each other, at least one or more porous acidic materials having microporous, mesoporous, or hierarchical (micro-mesoporous) structure. Various zeolite-type materials such as SAPOs and zeolites are available providing desired acidity and porosity characteristics.

According to certain preferred embodiments, the hydroprocessing in reactor A and/or reactor B is hydroisomerisation, and the hydrotreatment catalyst A and/or the hydrotreatment catalyst B is selected (when the hydroprocessing in the respective reactor is HI), independently from each other, from bifunctional hydroisomerisation catalysts, preferably from non-sulphided bifunctional hydroisomerisation catalysts, comprising
at least one or more metals selected from Group VIII of the Periodic Table, preferably from noble metals of Group VIII, more preferably from Pt and/or Pd; and
at least one or more acidic porous materials selected from zeolites and/or zeolite-type materials, wherein preferably at least one or more of the zeolites and/or zeolite-type materials has a framework type selected from AEL, ATO, AFO, MRE, MTT, MTW, TON, MRT, MOR, FER, and/or MWW, preferably at least one or more acidic porous materials selected from SAPO-11, SAPO-31, SAPO-41, ZSM-22, ZSM-23, ZSM-48, NU-10, ZBM-30, IZM-2, EU-2, and/or mordenite, more preferably at least one or more acidic porous materials selected from SAPO-11, SAPO-41, ZSM-23, and/or ZSM-48; and
optionally at least one or more of alumina, silica, amorphous silica-alumina, titanium alumina, titania, and/or zirconia.

This catalyst selection has been found to provide higher isomerisation selectivity that further contributes to achieving even higher amounts of isoparaffins, particularly multiple-branched isoparaffins, highly beneficial for the properties of the liquid transportation fuel components. The mentioned SAPOs and zeolites are commercially available with acidity and porosity characteristics that allow isomerisation, including multiple-branching, of n-paraffins, even of long-chained n-paraffins, such as C16+ paraffins.

According to certain embodiments, the hydroprocessing in reactor A and/or reactor B is hydrocracking, and the hydrotreatment catalyst A and/or the hydrotreatment catalyst B is selected (when the hydroprocessing in the respective reactor is HC), independently from each other, from bifunctional hydrocracking catalysts, preferably from non-sulphided bifunctional hydrocracking catalysts, comprising
at least one or more metals selected from Group VIII of the Periodic Table, Mo, Co, and/or W, preferably from Ni, Mo, Co, W, Pt, and/or Pd, more preferably from Pt and/or Pd; and
at least one or more acidic porous materials selected from zeolites, zeolite-type materials, and/or amorphous silica-alumina, wherein preferably at least one or more of the zeolites or zeolite-type materials has a framework type selected from MFI, BEA, FAU, MOR, FER, AEL, AFI, ATO, AFO, MRE, MTT, MTW, TON, and/or MRT, preferably at least one or more acidic porous materials selected from SAPO-5, SAPO-11, SAPO-31, SAPO-41, ZSM-22, ZSM-23, ZSM-43, ZSM-48, IZM-2, mordenite, beta-zeolites, Y-type zeolites, and/or amorphous silica-alumina, more preferably at least one or more acidic porous material selected from SAPO-5, SAPO-11, ZSM-23, beta-zeolites, Y-type zeolites, and/or amorphous silica-alumina; and
optionally at least one or more of alumina, silica, titanium alumina, titania, and/or zirconia.

This catalyst selection has been found highly beneficial because they have in addition to cracking activity also some isomerisation activity, and may be especially efficient in effective cracking. As further advantage, bifunctional HC catalysts comprising at least one or more metals selected from Group VIII noble metals, preferably from Pt and/or Pd, have been found to provide at relatively low temperatures a high activity compared to HC catalysts comprising non-noble metals, and thus even better control of thermal cracking. At low temperatures, the thermodynamic equilibrium tends to shift towards dearomatisation, thus reducing aromatics formation by side reactions. Providing the reactors with a bifunctional HC catalyst may achieve good isoparaffin content (wt-% isoparaffins of the total weight of paraffins) in the reactor effluent, even close to isoparaffin content obtainable with a bifunctional HI catalyst.

In the present process, a hydrocarbon feed is subjected to hydroprocessing in the presence of a hydrotreatment catalyst A, and optionally hydroprocessing effluent A or a recycle stream is subjected to hydroprocessing in the presence of a hydrotreatment catalyst B. The hydrotreatment catalyst A and the hydrotreatment catalyst B may have similar or same components. According to a preferred embodiment, the hydrotreatment catalyst A and the hydrotreatment catalyst B are different from each other. The hydrotreatment catalyst A and the hydrotreatment catalyst B may differ from each other for example by selection of at least one or more of a catalyst component, acidity, and/or metal loading, just to name a few, but it is also possible to use same catalyst as the hydrotreatment catalyst A in reactor A and as the hydrotreatment catalyst B in reactor B. Not merely the catalyst but also the operating conditions and the composition of the feed contribute to which reactions prevail. When contacting the hydrotreatment effluent A or the recycle stream with the hydrotreatment catalyst B under the hydroprocessing conditions in reactor B (first mode of the process according to the first and the second aspects, respectively), the hydroprocessing effluent B can be expected to have different composition and properties compared to the composition and properties of the hydroprocessing effluent A, obtained by contacting the hydrocarbon feed with the hydrotreatment catalyst A under the hydroprocessing conditions in reactor A, even if the catalysts were the same.'

In certain embodiments, the hydrotreatment catalyst A in reactor A and the hydrotreatment catalyst B in reactor B have different acidity-related characteristics. The acidity-related characteristics of acidic porous materials, such as zeolite, may include nature, number, and distribution, according to relative strength, of acid sites, and may be determined by well-known methods, for example by adsorption-desorption methods where release of adsorbed base substance like ammonia or pyridine at higher temperature indicates presence of strong acid sites. As one example of usable adsorption-desorption methods, a temperature programmed desorption of ammonia can be mentioned, for examples as performed in accordance with the procedure described in Niwa et al (Niwa, M., Katada, N. Measurements of acidic property of zeolites by temperature programmed desorption of ammonia. Catalysis Surveys from Asia 1, 215-226 (1997)). Yet another well-known method for determining the acidic property of zeolites, including strength of acidity, is ¹H-NMR method, for examples as performed in accordance with the procedure described in Heeribout et al (Heeribout L., Semmer V., Batamack P., Dorémieux-Morin C., Fraissard J. Brønsted acid strength of zeolites studied by 1H NMR: scaling, influence of defects. Microporous and Mesoporous Materials, Volume 21, Issues 4-6, May 1998, Pages 565-570).

In certain embodiments, particularly when the hydroprocessing in reactor A is HI and the hydroprocessing in reactor B is HC, the bifunctional hydrotreatment catalyst B of reactor B has a higher number of Brønsted acid sites compared to the bifunctional hydrotreatment catalyst A of reactor A, as determined by NH₃-TPD. In certain embodiments, particularly when the hydroprocessing in reactor A is HI and the hydroprocessing in reactor B is HC, the bifunctional hydrotreatment catalyst B has a higher total number of acid sites compared to the bifunctional hydrotreatment catalyst A, as determined by NH₃-TPD.

The hydrotreatment catalysts A and B may be in ready-to-use state as such or they may be as treated in any customary way to adjust their properties, such as selectivity and/or activity, before or during start-up by subjecting to reduction, sulphidation, and/or passivation for example with a nitrogen containing compound, such as an amine or ammonia, so as to obtain the ready-to-use fresh or regenerated hydrotreatment catalyst A and/or B. As used herein, hydrotreatment catalyst A, hydrotreatment catalyst B, fresh catalyst, and regenerated catalyst generally refer to the catalyst in its ready-to-use state.

In certain embodiments, the hydrotreatment catalyst A in reactor A and the hydrotreatment catalyst B in reactor B comprise a hydrocracking catalyst and a hydroisomerisation catalyst arranged in series, particularly in at least one or more separate beds. In these embodiments it is preferred to use bifunctional HC and HI catalysts, more preferably non-sulphided bifunctional HC and HI catalysts.

In embodiments where the hydroprocessing in reactor A and/or B is hydrodearomatisation, the respective hydrotreatment catalyst may be any catalyst conventionally used for hydrodearomatisation. Preferably non-sulphided catalysts, more preferably non-sulphided bifunctional catalysts, comprising noble metals are used as these are active in HDA already at lower temperatures, which is beneficial as at low temperatures thermodynamic equilibrium tends to shift towards dearomatisation. Additionally, in embodiments wherein both reactor A and B are operated using non-sulphided noble-metal catalysts, no sulphur needs to be added for keeping the catalyst active, and less or no effort is needed for separating sulphur containing compounds, especially H₂S, from gaseous fractions of the reactor effluents, and the recovered liquid transportation fuel component(s), and optional other product fractions, are obtainable with both ultra-low aromatics and sulphur content.

In embodiments where the hydroprocessing in reactor A and/or B is hydropolishing, the respective hydrotreatment catalyst may be any catalyst conventionally used for hydropolishing. Preferably non-sulphided catalysts, more preferably non-sulphided bifunctional catalysts, comprising noble metals are used as these are active already at lower temperatures, thus providing widest window for increasing temperature to compensate catalyst deactivation during continued operation.

The present process comprises a fractionation in which at least one or more liquid transportation fuel component(s) are recovered and the recycle stream may be separated. In embodiments or aspects where the recycle stream is separated, the amount of the separated recycle stream and the amount of recycle stream fed to reactor A and/or B may vary within broad ranges. Further liquid streams or cuts, such as side cut(s), may optionally be separated and/or recovered from the fractionation, and optionally recycled back to the process.

Preferably, in the process of the second aspect, the weight-ratio of the hydroprocessing effluent B to the hydroprocessing effluent A, subjected to fractionation, is from 1:10 to 10:1, such as from 1:5 to 5:1. When running i), ii), iii) i.e. in the first mode of the process, ratios towards the lower limits may be preferred when processing hydrocarbon feeds boiling at lower temperatures, and ratios towards the upper limits may be preferred when processing hydrocarbon feeds boiling at higher temperatures, such as feeds comprising paraffins heavier than C18.

In certain embodiments, the reactor A feed and/or the reactor B feed may comprise a side cut separated from the fractionation of the present process. Said side cut is preferably included in the reactor A feed and/or in the reactor B feed as a minor constituent. The side cut is preferably a fraction rich in C14-18 hydrocarbons, more preferably rich in C15-C17 hydrocarbons. The side cut is preferably a relatively narrow fraction to allow precise modification of the boiling point distribution of the total feed to the fractionation. Incorporating such side cut in in the reactor A feed and/or in the reactor B feed may improve quality of the recovered liquid transportation fuel components or of at least some of the recovered liquid transportation fuel components, particularly of aviation fuel component. The side cut may have an elevated isoparaffin content. Hence, inclusion of the side cut in in the reactor A feed and/or in the reactor B feed may increase the isoparaffin content of the respective feed. Additionally, hydroprocessing typically has an impurity-removing effect on the processed stream especially when followed by a gas-liquid separation. Hence, incorporating even a small amount of a side cut to the reactor A feed and/or the reactor B feed may reduce or dilute the impurity content of the respective feed, slowing down deactivation of the hydrotreatment catalyst in the respective reactor.

The fractionation of the present process may comprise any conventionally used fractionation technology. Preferably, the fractionation comprises distillation, such as atmospheric distillation or vacuum distillation. The fractionation may be preceded by a gas-liquid separation e.g. as described hereinafter.

The fractionation may be carried out in a fractionation system comprising one or more fractionation units. For example, gases and light naphtha may be separated in a pre-fractionation unit, while liquid transportation fuel component(s) and a recycle stream may be recovered and separated from a main distillation unit downstream of the pre-fractionation unit. In an alternative example, a single fractionation unit may be used.

In the process according to the first aspect the hydroprocessing effluent from the reactor arranged first in each mode (the reactor to A in the first mode and reactor B in the second mode) is subjected to separation of compounds gaseous at NTP e.g. by gas-liquid separation, before feeding the degassed hydroprocessing effluent to the reactor arranged second in each mode (reactor B in the first mode and reactor A in the second mode), while the hydroprocessing effluent from the reactor arranged second in each mode maybe subjected to to separation of compounds gaseous at NTP e.g. by gas-liquid separation within the fractionation. In the process according to the second and the third aspects the hydroprocessing effluent(s) may be subjected to separation of compounds gaseous at NTP e.g. by gas-liquid separation within the fractionation. In all aspects and modes of operation, separation of compounds gaseous at NTP may be conducted for example as gas-liquid separations as an integral step within the respective reactors, or in separate gas-liquid separation units that may follow the respective reactors.

Typically, the gas-liquid separation is conducted at a temperature within a range from 0 °C to 500 °C, such as from 15°C to 300°C, or from 15 °C to 150 °C, preferably from 15 °C to 65 °C, such as from 20 °C to 60 °C, and preferably at the same pressure as that of the reactor wherefrom the effluent originates. Typically, the pressure during the gas-liquid separation(s) may be within a range from 0.1 MPa to 20 MPa, preferably from 1 MPa to 10 MPa, or from 3 MPa to 7 MPa.

According to certain embodiments of the present process, different liquid transportation fuel components and further products may be recovered from the fractionation depending e.g. on the type of hydroprocessing in reactors A and B (or reactor A in the process according to the third aspect), operating conditions chosen, composition of the used hydrocarbon feed, prevailing mode of operation (first or second), then-current performance of hydrotreatment catalyst A and B (or hydrotreatment catalyst A in in the process of the third aspect), and/or existing or foreseen market demand. In this context by different liquid transportation fuel components and further products it is meant that from time to time different selection of product fractions can be recovered, and/or that from time to time the recovered product fractions can have different chemical composition and/or properties.

The liquid transportation fuel component(s) and optional further product(s) separated and/or recovered from the fractionation may for example include gasoline fuel component(s) boiling within a range from about 25 °C to about 200 °C, aviation fuel component(s) boiling within a range from about 100 °C to about 300 °C, such from about 150 °C to about 300 °C, a recycle stream having a T5 temperature of at least 270°C, diesel fuel component(s) boiling within a range from about 160 °C to about 380 °C, and/or marine fuel component(s) boiling within a range from about 180 °C to about 600 °C, such as from about 180 °C to about 400 °C (boiling within the ranges as determined according to EN ISO 3405-2019). In certain embodiments, gasoline fuel component(s) boiling within the range from about 25 °C to about 200 °C, aviation fuel component(s) boiling within the range from about 100 °C to about 300 °C, such as from about 150 °C to about 300 °C, and a recycle stream having a T5 temperature of at least 270°C are first recovered and/or separated from the fractionation, and diesel fuel component(s) boiling within the range from about 160 °C to about 380 °C and/or marine fuel component(s) boiling within the range from about 180 °C to about 600 °C (boiling within the ranges as determined according to EN ISO 3405-2019), are recovered from the separated recycle stream. Further products may be recovered from the fractionation, such as split or further separated from the gasoline fuel component, aviation fuel component, diesel fuel component, marine fuel component and/or the recycle stream. Examples of such further products include components for solvents, electrotechnical fluids, and base oils.

With the present process it is possible to produce a low viscosity aviation fuel component, having a lower freezing point, and especially a lower kinematic viscosity at -20 °C, compared to an aviation fuel component having similar IBP and FBP, and recovered at same run-time but produced by conventional HDO of fatty feedstock followed by HI, i.e. without switching from a first mode to a second mode, as in the present process.

Hence, in preferred embodiments of the present process, in steps iii) and III) at least one of the liquid transportation fuel components recovered from the fractionation is an aviation fuel component having density at 15 °C within a range from 730 to 772 kg/m³ (EN ISO 12185-1996), T10 temperature at most 205 °C (EN ISO 3405-2019), final boiling point at most 300 °C (EN ISO 3405-2019), flash point at least 38°C (IP 170-2013, Abel closed-cup method), and freezing point at most -40 °C (IP 529-2016). In these embodiments the recovered aviation fuel component is of high quality, and can be incorporated in aviation fuel compositions in elevated amounts. Typically, the recovered aviation fuel component has T10 and T90 temperatures, as determined according to EN ISO 3405-2019, within a range from 120 °C to 295 °C, preferably within a range from 130 °C to 295°C.

The present process enables recovery of at least an aviation fuel component in surprisingly high yields, even towards the end of the hydrotreatment catalyst A lifetime. In certain preferred embodiments of the present process, the aviation fuel component is recovered in a yield of at least 30 wt-%, preferably at least 40 w%, more preferably at least 50 w%, such as from 30 wt-% to 90 wt-% of the total weight of the hydrocarbon feed. In certain preferred embodiments, the aviation fuel component has a difference between T90 and T10 temperatures, as determined according to EN ISO 3405-2019, at least 70 °C, preferably at least 75 °C, more preferably at least 80 °C, even more preferably at least 85 °C, typically at most 180°C, such as within a range from 80 °C to 150°C, preferably from 80 °C to 130°C. In these embodiments the aviation fuel component may be recovered with improved yields, while reaching the desired cold properties, density, and flash point characteristics. Poor quality of the total feed fed to the fractionation would necessitate limiting the FBP heavily in order to meet the cold property requirements, which would limit also the T90 temperature, and make the T90-T10 difference narrower. However, here the present process enables providing excellent characteristics even towards the end of the hydrotreatment catalyst A lifetime, such as high isomerisation degree, particularly high multiple-branched i-paraffin content, and modified distillation characteristics as the amounts of the carbon numbers may be more evenly distributed, particularly in the C6-C18 range.

Typically in the present process, in steps iii) and III) at least one or more of an aviation fuel component, a diesel fuel component, a gasoline fuel component, and/or a marine fuel component is recovered from the fractionation, preferably at least an aviation fuel component, more preferably at least an aviation fuel component and a diesel fuel component, or at least an aviation fuel component and a gasoline fuel component, even more preferably at least an aviation fuel component, a diesel fuel component, and a gasoline fuel component, are recovered from the fractionation. Generally, in embodiments or aspect involving separating and recycling a recycle stream, it is preferred to recover from the fractionation at least periodically at least one heavy product such as a diesel fuel component, marine fuel component, and/or a base oil component. In this way, the heaviest components can be removed from the recycle loop.

In certain preferred embodiments, the recovered liquid transportation fuel components have a biogenic carbon content (EN 16640 (2017)) of at least 50 wt-%, preferably at least 70 wt-%, more preferably at least 90 wt-%, further preferably at least 95 wt-%, or even about 100 wt-%, based on the total weight of carbon (TC) in the respective recovered liquid transportation fuel component. The biogenic carbon content in the recovered liquid transportation fuel component(s) is mainly influenced by the biogenic carbon content in the hydrocarbon feed, in certain preferred embodiments by the oxygenated hydrocarbon feed subjected to HDO. However, any amounts of e.g. fossil hydrocarbon diluent fed to the HDO reactor may affect the biogenic carbon content of the recovered liquid transportation fuel component(s).

The liquid transportation fuel component(s) recovered in the present process may have improved (increased) isoparaffin content, particularly multiple-branched isoparaffin content, increased content of C8-C14 hydrocarbons, and/or reduced aromatics content compared to corresponding component(s) obtained by conventional HDO of fatty feedstock followed by HI. An aviation fuel component recovered from the present process may comprise C6 to C18 i-paraffins at least 85 wt-%, preferably at least 87 wt-%, more preferably at least 90 wt-%, even more preferably at least 92 wt-% of the total aviation fuel component weight; and/or C6-C18 multiple-branched i-paraffins at least 58 wt-%, preferably at least 60 wt-%, more preferably at least 62 wt-% of the total aviation fuel component weight. A diesel fuel component recovered from the present process may comprise C15-C22 i-paraffins at least 70 wt-%, preferably at least 75 wt-%, more preferably at least 80 wt-%, even more preferably at least 90 wt-% of the diesel fuel component weight; and/or C15-C22 multiple-branched i-paraffins at least 60 wt-%, preferably at least 63 wt-%, more preferably at least 65 wt-% of the diesel fuel component weight. A gasoline fuel component recovered from the present process may comprise at least 50 wt-%, preferably at least 55 wt-%, more preferably at least 60 wt-%, even more preferably at least 65 wt-% C4-C9 i-paraffins of the gasoline fuel component weight; and/or at least 5 wt-%, preferably at least 6 wt-%, more preferably at least 7 wt-%, even more preferably at least 10 wt-%, or at least 11 wt-% C6-C9 multiple-branched i-paraffins of the gasoline fuel component weight.

Even if each of the gasoline fuel component, aviation fuel component, and diesel fuel component may be recovered at the same time in the present process, they may be recovered as reasonably wide fractions. When the yield of the aviation fuel component is optimized, the diesel fuel component may be recovered as a narrower cut, or not at all.

Products recovered from the present process have excellent characteristics. The recovered liquid transportation fuel component(s) are suitable for use as blending components in fuel compositions, and may even be used, when suitably additized, as fuels as such, i.e. as unblended components. The recovered liquid transportation fuel component(s), and optional further product(s), are suitable for a wide range of various other uses, such as in feedstock(s) for industrial conversion processes, preferably in thermal cracking feedstock(s), such as in steam cracking feedstock(s), and/or in catalytic cracking feedstock(s), in transformer oil(s), in heat-transfer medium or media, in switchgear oil(s), in shock absorber oil(s), in insulating oil(s), in hydraulic fluid(s), in gear oil(s), in transmission fluid(s), in degreasing composition(s), in penetrating oil(s), in anticorrosion composition(s), in multipurpose oil(s), in metal working fluid(s), in rolling oil(s) especially for aluminum, in cutting oil(s), in drilling fluid(s), in solvent(s), in lubricant(s), in extender oil(s), in carrier(s), in dispersant composition(s), in demulsifier(s), in extractant(s), in paint composition(s), in coating fluid(s) or paste(s), in adhesive(s), in resin(s), in varnish(es), in printing paste(s) or ink(s), in detergent(s), in cleaner(s), in plasticizing oil(s), in turbine oil(s), in hydrophobization composition(s), in agriculture, in crop protection fluid(s), in construction, in concrete demoulding formulation(s), in electronic(s), in medical appliance(s), in composition(s) for car, electrical, textile, packaging, paper, cosmetic and/or pharmaceutical industry, and/or in manufacture of intermediate(s) therefor. The relatively high isomerisation degree and elevated share of shorter carbon chains obtainable by the present process are foreseen to improve fluidity, pumping and mixing characteristics, and blendability of the recovered components and/or fractions. These are generally desired and beneficial properties for a wide range of uses, particularly involving spraying, injecting and/or admixing with other ingredients.

### Schematic presentation of the process

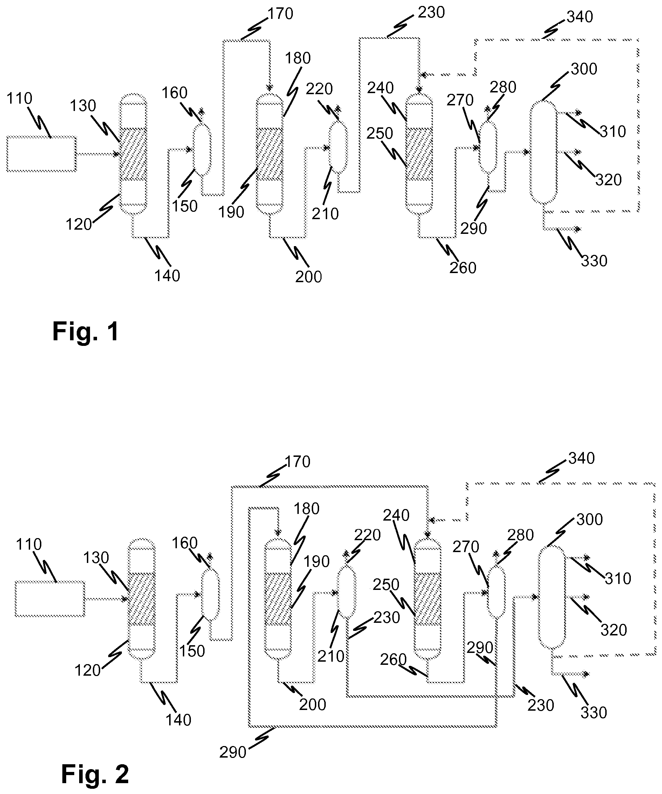

Fig. 1 schematically shows a process according to an embodiment of the first aspect of the invention before switching from i), ii), iii) to I), II), III) i.e. running according to the steps i), ii), iii) (first mode). In Fig. 1, oxygenated hydrocarbon feed 110 is fed to a HDO reactor 120 in which it is subjected to hydrodeoxygenation in the presence of a HDO catalyst 130 to obtain a hydrodeoxygenation effluent (HDO effluent) 140, and the obtained HDO effluent 140 is subjected to gas-liquid separation 150 to separate from the HDO effluent at least compounds that are gaseous at NTP 160 to obtain a degassed HDO effluent 170 , which is in this embodiment the herein defined paraffinic hydrocarbon feed. The degassed HDO effluent 170 is in Fig. 1 then fed to a reactor A 180 in which the degassed HDO effluent 170 is subjected to hydroprocessing in the presence of a hydrotreatment catalyst A 190 to obtain a hydroprocessing effluent A 200, and the obtained hydroprocessing effluent A 200 is subjected to gas-liquid separation 210 to separate from the hydroprocessing effluent A 200 at least compounds that are gaseous at NTP 220 to obtain a degassed hydroprocessing effluent A 230. In Fig. 1, the degassed hydroprocessing effluent A 230 is fed to a reactor B 240 in which it is subjected to hydroprocessing in the presence of a hydrotreatment catalyst B 250 to obtain a hydroprocessing effluent B 260. The hydroprocessing effluent B 260 is in Fig. 1 subjected to gas-liquid separation 270 to separate from the hydroprocessing effluent B 260 at least compounds that are gaseous at NTP 280 to obtain a degassed hydroprocessing effluent B 290. In Fig. 1, the degassed hydroprocessing effluent B 290 is fed to a distillation unit 300 that may comprise a single column, or prefractionation and main distillation columns, from which several streams or cuts may be obtained. From the distillation in Fig. 1, a gasoline fuel component 310, an aviation fuel component 320, and a diesel fuel component 330 are recovered. Additionally, a recycle stream 340, preferably having a T5 boiling point of 270 °C or higher, may optionally be separated, in which case a fuel component, such as the diesel fuel component 330, may be recovered by separating as a portion therefrom. In Fig. 1, the recycle stream 340 may be fed to the reactor B 240 to hydroprocessing as a co-feed with the degassed hydroprocessing effluent A 230.

Fig. 2 schematically shows a process according to an embodiment of the first aspect of the invention after having switched from i), ii), iii) to I), II), III) i.e. running according to the steps I), II), III) (second mode). In Fig. 2, the process may proceed as schematically shown in Fig. 1 to obtain a degassed HDO effluent 170 which is in this embodiment the herein defined paraffinic hydrocarbon feed. In Fig. 2 the degassed HDO effluent 170 is now fed to the reactor B 240 in which the degassed HDO effluent 170 is subjected to hydroprocessing in the presence of the hydrotreatment catalyst B 250 to obtain a hydroprocessing effluent B 260, and the obtained hydroprocessing effluent B 260 is subjected to gas-liquid separation 270 to separate from the hydroprocessing effluent B 260 at least compounds that are gaseous at NTP 280 to obtain a degassed hydroprocessing effluent B 290. In Fig. 2, the degassed hydroprocessing effluent B 290 is fed to the reactor A 180 in which it is subjected to hydroprocessing in the presence of the hydrotreatment catalyst A 190 to obtain a hydroprocessing effluent A 200. The hydroprocessing effluent A 200 is in Fig. 2 subjected to gas-liquid separation 210 to separate from the hydroprocessing effluent A 200 at least compounds that are gaseous at NTP 220 to obtain a degassed hydroprocessing effluent A 230. In Fig. 2, the degassed hydroprocessing effluent A 230 is fed to the distillation unit 300, where it may be fractionated into several streams or cuts. From the distillation in Fig. 2, a gasoline fuel component 310, an aviation fuel component 320, and a diesel fuel component 330 are recovered. Additionally, a recycle stream 340, preferably having a T5 boiling point of 270 °C or higher, may optionally be separated, in which case a fuel component, such as the diesel fuel component 330, may be recovered by separating as a portion therefrom. In Fig. 2, the recycle stream 340 may be fed to the reactor B 240 to hydroprocessing as a co-feed with the degassed HDO effluent 170.

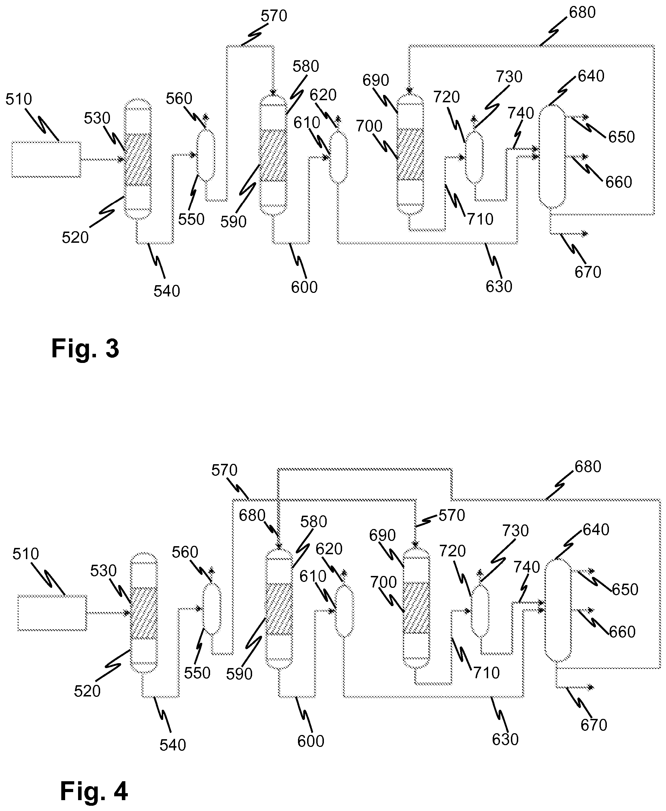

Fig. 3 schematically shows a process according to an embodiment of the second aspect of the invention before switching from i), ii), iii) to I), II), III), i.e. running according to the steps i), ii), iii) (first mode). In Fig. 3, oxygenated hydrocarbon feed 510 is fed to a HDO reactor 520 in which it is subjected to hydrodeoxygenation in the presence of a HDO catalyst 530 to obtain a hydrodeoxygenation effluent (HDO effluent) 540, and the obtained HDO effluent 540 is subjected to gas-liquid separation 550 to separate from the HDO effluent at least compounds that are gaseous at NTP 560 to obtain a degassed HDO effluent 570 which is in this embodiment the herein defined paraffinic hydrocarbon feed. The degassed HDO effluent 570 is in Fig. 3 then fed to a reactor A 580 in which the degassed HDO effluent 570 is subjected to hydroprocessing in the presence of a hydrotreatment catalyst A 590 to obtain a hydroprocessing effluent A 600, and the obtained hydroprocessing effluent A 600 is subjected to gas-liquid separation 610 to separate from the hydroprocessing effluent A 600 at least compounds that are gaseous at NTP 620 to obtain a degassed hydroprocessing effluent A 630. In Fig. 3, the degassed hydroprocessing effluent A 630 is fed to a distillation unit 640 that may comprise a single column, or prefractionation and main distillation columns, from which several streams or cuts may be obtained. From the distillation in Fig. 3, a gasoline fuel component 650, an aviation fuel component 660, and/or a diesel fuel component 670 are recovered, and further, a recycle stream 680, preferably having a T5 boiling point of 270 °C or higher, is separated. The recycle stream 680 is in Fig. 3 fed to a reactor B 690 in which it is subjected to hydroprocessing in the presence of a hydrotreatment catalyst B 700 to obtain a hydroprocessing effluent B 710. In Fig. 3, the hydroprocessing effluent B 710 is subjected to gas-liquid separation 720 to separate from the hydroprocessing effluent B 710 at least compounds that are gaseous at NTP 730 to obtain a degassed hydroprocessing effluent B 740. The degassed hydroprocessing effluent B 740 is in Fig. 3 then fed as a co-feed with the degassed hydroprocessing effluent A 630 to the distillation unit 640, from which several streams or cuts may be obtained as described above.

Fig. 4 schematically shows a process according to an embodiment of the second aspect of the invention after switching from i), ii), iii) to I), II), III) i.e. running according to the steps I), II), III) (second mode). In Fig. 4, the process may proceed as schematically shown in Fig. 3 to obtain a degassed HDO effluent 570 which is in this embodiment the herein defined paraffinic hydrocarbon feed. In Fig. 4 the degassed HDO effluent 570 is now fed to the reactor B 690 in which the degassed HDO effluent 570 is subjected to hydroprocessing in the presence of the hydrotreatment catalyst B 700 to obtain a hydroprocessing effluent B 710, and the obtained hydroprocessing effluent B 710 is subjected to gas-liquid separation 720 to separate from the hydroprocessing effluent B 710 at least compounds that are gaseous at NTP 730 to obtain a degassed hydroprocessing effluent B 740. In Fig. 4, the degassed hydroprocessing effluent B 740 is fed to the distillation unit 640, from which several streams or cuts may be obtained. From the distillation in Fig. 4, a gasoline fuel component 650, an aviation fuel component 660, and/or a diesel fuel component 670 are recovered, and further, a recycle stream 680, preferably having a T5 boiling point of 270 °C or higher, is separated. The recycle stream 680 is in Fig. 4 fed to the reactor A 580 in which it is subjected to hydroprocessing in the presence of the hydrotreatment catalyst A 590 to obtain a hydroprocessing effluent A 600. In Fig. 4, the hydroprocessing effluent A 600 is subjected to gas-liquid separation 610 to separate from the hydroprocessing effluent A 600 at least compounds that are gaseous at NTP 620 to obtain a degassed hydroprocessing effluent A 630, and the degassed hydroprocessing effluent A 630 is then fed as a co-feed with the degassed hydroprocessing effluent B 740 to the distillation unit 640, from which several streams or cuts may be obtained as described above.

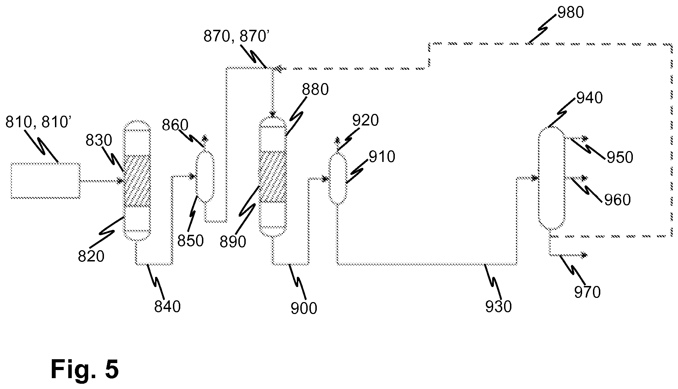

Fig. 5 schematically shows a process according to an embodiment of the third aspect of the invention. In Fig. 5, before switching from i), ii), iii) to I), II), III), i.e. when running according to the steps i), ii), iii) (first mode), oxygenated hydrocarbon feed 810 is fed to a HDO reactor 820 in which it is subjected to hydrodeoxygenation in the presence of a HDO catalyst 830 to obtain a hydrodeoxygenation effluent (HDO effluent) 840, and the obtained HDO effluent 840 is subjected to gas-liquid separation 850 to separate from the HDO effluent at least compounds that are gaseous at NTP 860 to obtain a degassed HDO effluent 870, which is in this embodiment the herein defined paraffinic hydrocarbon feed that comprises nitrogen impurities, and providing the degassed HDO effluent 870 as a reactor A feed. The degassed HDO effluent 870 is in Fig. 5 then fed to a reactor A 880 in which the degassed HDO effluent 870 is subjected to hydroprocessing in the presence of a hydrotreatment catalyst A 890 to obtain a hydroprocessing effluent A 900. In Fig. 4, the obtained hydroprocessing effluent A 900 is subjected to gas-liquid separation 910 to separate from the hydroprocessing effluent A 900 at least compounds that are gaseous at NTP 920 to obtain a degassed hydroprocessing effluent A 930. In Fig. 5, the degassed hydroprocessing effluent A 930 is fed to a distillation unit 940 that may comprise a single column, or prefractionation and main distillation columns, from which several streams or cuts may be obtained. From the distillation in Fig. 5, a gasoline fuel component 950, an aviation fuel component 960, and/or a diesel fuel component 970 are recovered. Additionally, a recycle stream 980 may optionally be separated and fed to the reactor A 880 to hydroprocessing as a co-feed with the degassed HDO effluent 870. Preferably the recycle stream 980 has a T5 boiling point of 270 °C or higher, in which case the diesel fuel component 970 may be recovered by separating as a portion therefrom. In Fig. 5, after switching from i), ii), iii) to I), II), III), i.e. when running according to the steps I), II), III) (second mode), the process may proceed as schematically shown in Fig. 5 and as explained above for the first mode, except that this time a reactor A feed comprising essentially no nitrogen impurities (ASTM D4629-17) 870' is fed to the reactor A 880 and subjected to hydroprocessing in the presence of the hydrotreatment catalyst A 890. The reactor A feed comprising essentially no nitrogen impurities (ASTM D4629-17) 870' may be obtained for example by selecting an oxygenated hydrocarbon feed 810' having a lower content of nitrogen impurities compared to the oxygenated hydrocarbon feed 810 used in the first mode and/or by diluting a degassed HDO effluent 870 with a suitable amount of a recycle stream 980 separated from the fractionation 940 so as to achieve the targeted level of nitrogen impurities. While separating a recycle stream 980 is an option also in the above-described first mode of the third aspect, in the second mode it is a preferred option, as it provides a simple way to perfect the level of nitrogen impurities in the feed fed to the reactor A. Preferably the recycle stream 980 has a T5 boiling point of 270 °C or higher, and in case a diesel fuel component 970 is recovered it can be separated as a portion from such recycle stream, and the heavy hydrocarbon molecules present in such recycle stream may be repeatedly subjected to hydroprocessing in reactor A in the presence of the hydrotreatment catalyst A thereby compensating at least to some extent the deactivation level of the hydrotreatment catalyst A.

Without limitation to the embodiments presented in the Figures, in certain preferred embodiments of the present process where at least an aviation fuel component is recovered from the fractionation, the monitored parameters indicative of deactivation of the hydroteatment catalyst A, when run in the first mode, include two or more of the following: temperature e.g. as monitored at the inlet of the reactor A, WHSV in the reactor A, temperature difference over the reactor A or over a bed of hydrotreatment catalyst A, cloud point and/or pour point of the degassed hydroprocessing effluent A, cloud point and/or pour point of the recycle stream or the diesel fuel component, freezing point and/or one or more distillation characteristics of the aviation fuel component, the corresponding received values of which are compared with predetermined values. When the monitored parameter is the temperature e.g. as monitored at the inlet of the reactor A, the predetermined value may be e.g. max. 450°C; for WHSV in the reactor A the predetermined value may be e.g. 6 kg hydrocarbon feed per kg catalyst per hour; for the cloud point of the degassed hydrotreatmment effluent A the predetermined value may be e.g. max. -5°C; for the cloud point of the recycle stream or the diesel fuel component the predetermined value may be e.g. max. -15°C (ASTM D 5771-2017); for the freezing point of the aviation fuel component the predetermined value may be e.g. max. -40°C (IP 529-201); for the distillation characteristics of the aviation fuel component the predetermined value may be e.g. for T10 max. 205°C or for T90-T10 difference e.g. min. 22 °C (EN ISO 3405-2019); for the distillation characteristics of the diesel fuel component the predetermined value may be e.g. T95 max. 360°C (EN ISO 3405-2019). When the values received for the monitored parameters reach the corresponding predetermined values, the process is switched to and run in the second mode. After switching, the monitored parameters and the predetermined values may be the same as in the first mode, or different parameters may be monitored, and/or different predetermined values selected, depending e.g. on whether same products and qualities thereof are still targeted.

The process as described herein can be controlled by state of the art means, particularly by computer implemented means. In certain exemplary embodiments, the present process further comprises causing a control apparatus to perform at least the steps of comparing the received value(s) with predetermined value(s); and switching from i), ii), iii) to I), II), III). In certain exemplary embodiments, the present process is controlled by a computer program product, as defined as the fourth aspect of the invention, comprising instructions which, when executed by a processor of a control apparatus in a system for producing at least one liquid transportation fuel component, cause the control apparatus to at least compare the received values with predetermined values, and when the received values reach the predetermined values to switch from i), ii), iii) to I), II), III) of the process according to any of the aspects, and optionally to switch from I), II), III) back to i), ii), iii) of the process according to any of the aspects. The control apparatus executing the instructions of the computer program product may be for example a general-purpose computer or some other electronic data processing apparatus comprising a processor and a (non-transient) memory. The instructions of the computer program product may be stored in the memory for execution by the processor to cause the operation of the control apparatus.

Various embodiments have been presented. It should be appreciated that in this document, words comprise, include, and contain are each used as open-ended expressions with no intended exclusivity.

The foregoing description has provided by way of non-limiting examples of particular implementations and embodiments a full and informative description of the best mode presently contemplated by the inventors for carrying out the invention. As such, the foregoing description shall be considered as merely illustrative of the principles of the present invention, and not in limitation thereof. Hence, the scope of the invention is only restricted by the appended patent claims.

## Claims

1. A process for producing at least one liquid transportation fuel component, the process comprising:
providing a hydrocarbon feed comprising nitrogen impurities;
i) subjecting a reactor A feed comprising the hydrocarbon feed to hydroprocessing in a reactor A in the presence of a hydrotreatment catalyst A to obtain a hydroprocessing effluent A, and separating from the hydroprocessing effluent A at least compounds gaseous at NTP to obtain a degassed hydroprocessing effluent A;
ii) subjecting a reactor B feed comprising the degassed hydroprocessing effluent A to hydroprocessing in a reactor B in the presence of a hydrotreatment catalyst B to obtain a hydroprocessing effluent B;
iii) feeding the hydroprocessing effluent B, optionally after separating from the hydroprocessing effluent B at least compounds gaseous at NTP, to fractionation, and recovering from the fractionation at least one or more liquid transportation fuel component(s); and
monitoring parameters indicative of deactivation of the hydrotreatment catalyst A to receive values;
comparing the received values with predetermined values; and
when the received values reach the predetermined values, switching from i), ii), iii) to:
I) subjecting a reactor B feed comprising the hydrocarbon feed to hydroprocessing in the reactor B in the presence of the hydrotreatment catalyst B to obtain a hydroprocessing effluent B, and separating from the hydroprocessing effluent B at least compounds gaseous at NTP to obtain a degassed hydroprocessing effluent B;
II) subjecting a reactor A feed comprising the degassed hydroprocessing effluent B to hydroprocessing in the reactor A in the presence of the hydrotreatment catalyst A to obtain a hydroprocessing effluent A;
III) feeding the hydroprocessing effluent A, optionally after separating from the hydroprocessing effluent A at least compounds gaseous at NTP, to fractionation, and recovering from the fractionation at least one or more liquid transportation fuel component(s).

2. A process for producing at least one liquid transportation fuel component, the process comprising:
providing a hydrocarbon feed comprising nitrogen impurities;
i) subjecting a reactor A feed comprising the hydrocarbon feed to hydroprocessing in a reactor A in the presence of a hydrotreatment catalyst A to obtain a hydroprocessing effluent A, subjecting the hydroprocessing effluent A to fractionation and separating from the fractionation at least a recycle stream having a T5 temperature (5 vol-% recovered, EN ISO 3405-2019) of 270 °C or higher, and optionally comprising C16 n-paraffins;
ii) subjecting a reactor B feed comprising the recycle stream to hydroprocessing in a reactor B in the presence of a hydrotreatment catalyst B to obtain a hydroprocessing effluent B;
iii) feeding the hydroprocessing effluent B to the fractionation as a co-feed with the hydroprocessing effluent A, and recovering from the fractionation at least one or more liquid transportation fuel component(s); and
monitoring parameters indicative of deactivation of the hydrotreatment catalyst A to receive values;
comparing the received values with predetermined values; and
when the received values reach the predetermined values, switching from i), ii), iii) to:
I) subjecting a reactor B feed comprising the hydrocarbon feed to hydroprocessing in the reactor B in the presence of the hydrotreatment catalyst B to obtain a hydroprocessing effluent B, subjecting the hydroprocessing effluent B to fractionation and separating from the fractionation at least a recycle stream having a T5 temperature (5 vol-% recovered, EN ISO 3405-2019) of 270 °C or higher, and optionally comprising C16 n-paraffins;
II) subjecting a reactor A feed comprising the recycle stream to hydroprocessing in the reactor A in the presence of the hydrotreatment catalyst A to obtain a hydroprocessing effluent A;
III) feeding the hydroprocessing effluent A to the fractionation as a co-feed with the hydroprocessing effluent B, and recovering from the fractionation at least one or more liquid transportation fuel component(s).

3. The process according to claim 1 or 2, wherein the hydrocarbon feed comprises at least 0.4 w-ppm nitrogen, expressed as elemental nitrogen (ASTM D4629-17), of the total weight of the hydrocarbon feed, or at least 0.6 w-ppm, or at least 1.0 w-ppm, or even at least 1.5 w-ppm or at least 2.0 w-ppm nitrogen, and optionally at most 1.0 wt-%, preferably at most 0.8 wt-%, more preferably at most 0.5 wt-% oxygen, expressed as elemental oxygen (ASTM D5622-2017), of the total weight of the hydrocarbon feed.

4. The process according to any one of the preceding claims, wherein the parameters indicative of deactivation of the hydrotreatment catalyst A comprise at least two or more of:
a. content of nitrogen impurities in the reactor A feed or in the hydrocarbon feed, and optionally content of at least one or more additional impurity selected from S, O, P, Si, CI, Fe, alkali metals, alkaline earth metals, and/or coke-forming compounds in the reactor A feed or in the hydrocarbon feed;
b. content of NH₃ and optionally content of H₂S in the gaseous phase of the hydroprocessing effluent A;
c. physico-chemical characteristic(s) of the degassed hydroprocessing effluent A, preferably at least one or more of a cloud point, freezing point, pour point, cold filter plugging point, kinematic viscosity, density, and/or a distillation characteristic;
d. compositional characteristic(s) of the degassed hydroprocessing effluent A, preferably at least one or more of content of isoparaffins, content of C8-C14 hydrocarbons, content of multiple-branched isoparaffins, and/or content of C1-C4 hydrocarbons in the degassed hydroprocessing effluent A;
e. yield of at least one or more of the recovered liquid transportation fuel component(s) and/or the separated recycle stream, preferably yield of an aviation fuel component;
f. physico-chemical characteristic(s) of at least one or more of the recovered liquid transportation fuel component(s) and/or of the separated recycle stream, preferably at least one or more of a cloud point, freezing point, pour point, cold filter plugging point, kinematic viscosity, density, research octane number (RON), cetane number, and/or a distillation characteristic;
g. compositional characteristic(s) of at least one or more of the recovered liquid transportation fuel component(s) and/or the recycle stream, preferably content of isoparaffins and/or content of multiple-branched isoparaffins in one or more of the recovered liquid transportation fuel component(s) and/or of the separated recycle stream;
h. temperature difference over the reactor A, or over one or more catalyst bed therein; and/or
i. operating condition(s) in the reactor A selected from temperature, pressure, weight hourly space velocity (WHSV), H₂ to reactor A feed ratio, and/or H₂ partial pressure at the inlet of the reactor A.

5. The process according to any one of the preceding claims, wherein the hydroprocessing in reactor A and in reactor B are selected, independently from each other, from at least one or more of hydroisomerisation, hydrocracking, hydrodearomatisation and/or hydropolishing, preferably the hydroprocessing in reactor A and in reactor B is hydroisomerisation, or the hydroprocessing in reactor A and reactor B is hydrocracking, or the hydroprocessing in reactor A is hydroisomerisation and the hydroprocessing in reactor B is hydrocracking, or the hydroprocessing in reactor A is hydrocracking and the hydroprocessing in reactor B is hydroisomerisation; more preferably the hydroprocessing in reactor A and reactor B is hydroisomerisation.

6. The process according to claim 1 or any one of claims 3-5 when depending on claim 1, wherein the switching comprises, when running i), ii), iii), feeding a gradually decreasing portion of the hydrocarbon feed and a gradually increasing portion of degassed hydroprocessing effluent B as part of the reactor A feed to reactor A and at the same time a gradually increasing portion of the hydrocarbon feed and a gradually decreasing portion of the degassed hydroprocessing effluent A as part of the reactor B feed to reactor B, until the process is run according to I), II), III); or
the process according to claim 2 or any one of claims 3-5 when depending on claim 2, wherein the switching comprises, when running i), ii), iii), feeding a gradually decreasing portion of the hydrocarbon feed and a gradually increasing portion of the recycle stream as part of the reactor A feed to reactor A and at the same time a gradually increasing portion of the hydrocarbon feed and a gradually decreasing portion of the recycle stream as part of the reactor B feed to reactor B, until the process is run according to I), II, III).

7. The process according to any one of the preceding claims,
wherein the hydroprocessing in reactor A and/or reactor B is hydroisomerisation conducted at a temperature within a range from 200 °C to 500 °C, preferably from 230 °C to 500 °C, more preferably from 250 °C to 450 °C, even more preferably from 280 °C to 400 °C, a pressure within a range from 1 MPa to 10 MPa, preferably from 2 MPa to 8 MPa or from 3 MPa to 10 MPa, a H₂ partial pressure at the inlet of the reactor within a range from 1 MPa to 10 MPa, preferably from 2 MPa to 8 MPa, a weight hourly space velocity within a range from 0.1 to 10, preferably from 0.2 to 8, more preferably from 0.4 to 6 kg reactor feed per kg catalyst per hour, and a H₂ to reactor feed ratio within a range from 10 to 2000, preferably from 50 to 1000 normal liters H₂ per liter reactor feed; and/or
wherein the hydroprocessing in reactor A and/or reactor B is hydrocracking conducted at a temperature within a range from 200 °C to 450 °C, preferably from 220 °C to 430 °C, more preferably from 280 °C to 350 °C, a pressure within a range from 0.4 MPa to 8 MPa, preferably from 1 MPa to 7 MPa, more preferably from 2. 5 MPa to 7 MPa, a H₂ partial pressure at the inlet of the reactor within a range from 0.4 MPa to 8 MPa, preferably from 1 MPa to 7 MPa, more preferably from 2.5 MPa to 7 MPa, a weight hourly space velocity within a range from 0.1 to 10, preferably from 0.2 to 8, more preferably from 0.4 to 6, even more preferably from 0.5 to 1.5 kg reactor feed per kg catalyst per hour, and a H₂ to reactor feed ratio within a range from 10 to 2000, preferably from 50 to 1000 normal liters H₂ per liter reactor feed; and/or wherein reactor A is operated at a higher temperature than reactor B.

8. The process according to any one of the preceding claims, wherein the hydrotreatment catalyst A and/or the hydrotreatment catalyst B is selected, independently from each other, from non-sulphided bifunctional hydrotreatment catalysts comprising at least one or more noble metals of Group VIII of the Periodic Table, more preferably Pt and/or Pd, and at least one or more acidic porous materials, and wherein the reactor A feed and the reactor B feed each comprises less than 50 wt-ppm, preferably less than 30 wt-ppm, more preferably less than 10 wt-ppm sulphur of the total respective feed (ppm by weight, calculated as elemental S), as determined according to ISO 20846-2019;
and/or wherein the hydrotreatment catalyst A and the hydrotreatment catalyst B are different from each other.

9. The process according to any one of the preceding claims,
wherein the hydroprocessing in reactor A and/or reactor B is hydrocracking, and the respective hydrotreatment catalyst(s) is selected, independently from the other, from bifunctional hydrocracking catalysts, preferably from non-sulphided bifunctional hydrocracking catalysts, comprising
at least one or more metals selected from Group VIII of the Periodic Table, Mo, Co, and/or W, preferably from Ni, Mo, Co, W, Pt, and/or Pd, more preferably from Pt and/or Pd; and
at least one or more acidic porous materials selected from zeolites, zeolite-type materials, and/or amorphous silica-alumina, wherein preferably at least one or more of the zeolites or zeolite-type materials has a framework type selected from MFI, BEA, FAU, MOR, FER, AEL, AFI, ATO, AFO, MRE, MTT, MTW, TON, and/or MRT, preferably at least one or more acidic porous materials selected from SAPO-5, SAPO-11, SAPO-31, SAPO-41, ZSM-22, ZSM-23, ZSM-43, ZSM-48, IZM-2, mordenite, beta-zeolites, Y-type zeolites, and/or amorphous silica-alumina, more preferably at least one or more acidic porous material selected from SAPO-5, SAPO-11, ZSM-23, beta-zeolites, Y-type zeolites, and/or amorphous silica-alumina; and
optionally at least one or more of alumina, silica, titanium alumina, titania, and/or zirconia; and/or
wherein the hydroprocessing in reactor A and/or reactor B is hydroisomerisation, and the respective hydrotreatment catalyst(s) is selected, independently from each other, from bifunctional hydroisomerisation catalysts, preferably from non-sulphided bifunctional hydroisomerisation catalysts, comprising
at least one or more metals selected from Group VIII of the Periodic Table, preferably from noble metals of Group VIII, more preferably from Pt and/or Pd; and
at least one or more acidic porous materials selected from zeolites and/or zeolite-type materials, wherein preferably at least one or more of the zeolites and/or zeolite-type materials has a framework type selected from AEL, ATO, AFO, MRE, MTT, MTW, TON, MRT, MOR, FER, and/or MWW, preferably at least one or more acidic porous materials selected from SAPO-11, SAPO-31, SAPO-41, ZSM-22, ZSM-23, ZSM-48, NU-10, ZBM-30, IZM-2, EU-2, and/or mordenite, more preferably at least one or more acidic porous materials selected from SAPO-11, SAPO-41, ZSM-23, and/or ZSM-48; and
optionally at least one or more of alumina, silica, amorphous silica-alumina, titanium alumina, titania, and/or zirconia.

10. The process according to any one of the preceding claims, wherein the hydrocarbon feed comprises at least 90 wt-%, preferably at least 95 wt-%, more preferably at least 98 wt-%, even more preferably at least 99 wt-% hydrocarbons of the total weight of the hydrocarbon feed, and/or at least 60 wt-%, preferably at least 70 wt-%, more preferably at least 80 wt-%, even more preferably at least 90 wt-% paraffins of the total weight of the hydrocarbon feed; and/or at most 30 wt-%, preferably at most 25 wt-%, more preferably at most 20 wt-%, even more preferably at most 15 wt-% isoparaffins of the total weight of paraffins in the hydrocarbon feed; and/or at least 70 wt-%, preferably at least 80 wt-%, more preferably at least 90 wt-%, even more preferably at least 95 wt-% C12-C30 hydrocarbons of the total weight of the hydrocarbon feed; and/or at least 70 wt-%, preferably at least 80 wt-%, more preferably at least 90 wt-%, even more preferably at least 95 wt-% C14-C22 hydrocarbons of the total weight of the hydrocarbon feed and/or
wherein the biogenic carbon content (EN 16640 (2017)) of the hydrocarbon feed is at least 50 wt-%, preferably at least 70 wt-%, more preferably at least 90 wt-%, even more preferably at least 95 wt-%, or about 100 wt-% based on the total weight of carbon (TC) in the hydrocarbon feed; and/or
wherein the step of providing the hydrocarbon feed comprises
subjecting an oxygenated hydrocarbon feed to catalytic hydrotreatment, preferably to catalytic hydrodeoxygenation, to obtain a hydrotreatment effluent, wherein the oxygenated hydrocarbon feed preferably comprises at least one or more of vegetable oils, animal fats, and/or microbial oils, and/or
subjecting a syngas to a Fischer-Tropsch (FT) conversion to obtain a FT effluent,
subjecting the hydrotreatment effluent and/or the FT effluent to a gas-liquid separation, and optionally to a hydrocarbon feed fractionation to provide the hydrocarbon feed.

11. The process according to any one of the preceding claims,
wherein in steps iii) and III) at least one or more of an aviation fuel component, a diesel fuel component, a gasoline fuel component, and/or a marine fuel component are recovered from the fractionation, preferably at least an aviation fuel component, more preferably at least an aviation fuel component and a diesel fuel component, or at least an aviation fuel component and a gasoline fuel component; and/or
wherein in steps iii) and III) at least an aviation fuel component having density at 15 °C within a range from 730 kg/m³ to 772 kg/m³ (EN ISO 12185-1996), T10 temperature at most 205 °C (EN ISO 3405-2019), final boiling point at most 300 °C (EN ISO 3405-2019), flash point at least 38 °C (IP 170-2013, Abel closed-cup method), and freezing point at most -40 °C (IP 529-2016) is recovered from the fractionation.

12. A process for producing at least one liquid transportation fuel component, the process comprising:
i) providing a reactor A feed comprising a hydrocarbon feed comprising nitrogen impurities, wherein the reactor A feed comprises at least 0.4 w-ppm nitrogen, expressed as elemental nitrogen (ASTM D4629-17), of the total weight of the reactor A feed, or at least 0.6 w-ppm, or at least 1.0 w-ppm, or even at least 1.5 w-ppm or at least 2.0 w-ppm nitrogen;
ii) subjecting the reactor A feed to hydroprocessing in a reactor A in the presence of a hydrotreatment catalyst A to obtain a hydroprocessing effluent A, and optionally separating from the hydroprocessing effluent A at least compounds gaseous at NTP to obtain a degassed hydroprocessing effluent A;
iii) feeding the hydroprocessing effluent A or the degassed hydroprocessing effluent A to fractionation, and recovering from the fractionation at least one or more liquid transportation fuel component(s); and
monitoring parameters indicative of deactivation of the hydrotreatment catalyst A to receive values;
comparing the received values with predetermined values; and
when the received values reach the predetermined values, switching from i), ii), iii) to:
I) providing a reactor A feed comprising a hydrocarbon feed, wherein the reactor A feed comprises essentially no nitrogen impurities;
II) subjecting the reactor A feed to hydroprocessing in the reactor A in the presence of the hydrotreatment catalyst A to obtain a hydroprocessing effluent A, and optionally separating from the hydroprocessing effluent A at least compounds gaseous at NTP to obtain a degassed hydroprocessing effluent A;
III) feeding the hydroprocessing effluent A or the degassed hydroprocessing effluent A to fractionation, and recovering from the fractionation at least one or more liquid transportation fuel component(s).

13. The process according to claim 12, wherein the reactor A feed in step I) comprises at most 0.3 w-ppm, preferably less than 0.3 w-ppm nitrogen expressed as elemental nitrogen (ASTM D4629-17), based on the total weight of the reactor A feed,
and/or wherein
the hydrocarbon feed in step i) and/or step I) is as further defined in claim 10; and/or
the step of providing the hydrocarbon feed in step i) and/or step I) is as further defined in claim 10; and/or
the parameters indicative of deactivation of the hydrotreatment catalyst A are as further defined in claim 4; and/or
the hydroprocessing in reactor A is as further defined in claim 5; and/or
the hydroprocessing in reactor A is conducted as further defined in claim 7; and/or
the hydroprocessing in reactor A and the hydrotreatment catalyst A are as further defined in claim 9; and/or
the recovered liquid transportation fuel component(s) are as further defined in claim 11.

14. The process according to any one of the preceding claims, wherein the process further comprises when running I), II), III), monitoring parameters indicative of reversal of the deactivation of the hydrotreatment catalyst A to receive values, wherein the parameters indicative of reversal of the deactivation of the hydrotreatment catalyst A preferably comprise at least two or more parameters selected from a. to i. as defined in claim 4; comparing the received values with predetermined values; and optionally when the received values reach the predetermined values, switching from I), II), III) back to i), ii), iii).

15. A computer program product comprising instructions which, when executed by a processor of a control apparatus in a system for producing at least one liquid transportation fuel component, cause in a process according to any one of claims 1 to 14 the control apparatus to compare the received values with predetermined values, and when the received values reach the predetermined values to switch from i), ii), iii) to I), II), III), and optionally to switch from I), II), III) back to i), ii), iii).

## Patentansprüche

1. Verfahren zur Herstellung zumindest einer flüssigen Transportkraftstoffkomponente (bzw. Verkehrskraftstoffkomponente), wobei das Verfahren umfasst:
Bereitstellen eines Kohlenwasserstoffeinsatzmaterials, das Stickstoffverunreinigungen umfasst;
i) Unterziehen eines das Kohlenwasserstoffeinsatzmaterial umfassenden Einsatzmaterials für Reaktor A einem Hydroprocessing (bzw. einer Hydrobehandlung) in einem Reaktor A in Gegenwart eines Hydrotreatment- (bzw. Hydrier-) Katalysators A, um ein Hydroprocessing-Ausströmmaterial A zu erhalten, und Abtrennen zumindest von bei NTP (Normaltemperatur und -druck) gasförmigen Verbindungen von dem Hydroprocessing-Ausströmmaterial A, um ein entgastes Hydroprocessing-Ausströmmaterial A zu erhalten;
ii) Unterziehen eines das entgaste Hydroprocessing-Ausströmmaterial A umfassenden Einsatzmaterials für Reaktor B einem Hydroprocessing in einem Reaktor B in Gegenwart eines Hydrotreatment-Katalysators B, um ein Hydroprocessing-Ausströmmaterial B zu erhalten;
iii) Einspeisen des Hydroprocessing-Ausströmmaterials B, optional nach Abtrennung zumindest von bei NTP gasförmigen Verbindungen aus dem Hydroprocessing-Ausströmmaterial B, in eine Fraktionierung und Gewinnung von zumindest einer oder mehreren flüssigen Transportkraftstoffkomponente(n) aus der Fraktionierung; und
Überwachen von Parametern, die auf eine Deaktivierung des Hydrotreatment-Katalysators A hinweisen, um Werte zu erhalten;
Vergleichen der erhaltenen Werte mit vorbestimmten Werten; und
wenn die erhaltenen Werte die vorbestimmten Werte erreichen, Umschalten von i), ii), iii) auf:
I) Unterziehen eines das Kohlenwasserstoffeinsatzmaterial umfassenden Einsatzmaterials für Reaktor B einem Hydroprocessing in dem Reaktor B in Gegenwart des Hydrotreatment-Katalysators B, um ein Hydroprocessing-Ausströmmaterial B zu erhalten, und Abtrennen zumindest von bei NTP gasförmigen Verbindungen von dem Hydroprocessing-Ausströmmaterial B, um ein entgastes Hydroprocessing-Ausströmmaterial B zu erhalten;
II) Unterziehen eines das entgaste Hydroprocessing-Ausströmmaterial B umfassenden Einsatzmaterials für Reaktor A einem Hydroprocessing in dem Reaktor A in Gegenwart des Hydrotreatment-Katalysators A, um ein Hydroprocessing-Ausströmmaterial A zu erhalten;
III) Einspeisen des Hydroprocessing-Ausströmmaterials A, optional nach Abtrennung zumindest von bei NTP gasförmigen Verbindungen aus dem Hydroprocessing-Ausströmmaterial A, in eine Fraktionierung und Gewinnung von zumindest einer oder mehreren flüssigen Transportkraftstoffkomponente(n) aus der Fraktionierung.

2. Verfahren zur Herstellung zumindest einer flüssigen Transportkraftstoffkomponente, wobei das Verfahren umfasst:
Bereitstellen eines Kohlenwasserstoffeinsatzmaterials, das Stickstoffverunreinigungen umfasst;
i) Unterziehen eines das Kohlenwasserstoff-Einsatzmaterial umfassenden Einsatzmaterials für Reaktor A einem Hydroprocessing in einem Reaktor A in Gegenwart eines Hydrotreatment-Katalysators A, um ein Hydroprocessing-Ausströmmaterial A zu erhalten, Unterziehen des Hydroprocessing-Ausströmmaterials A einer Fraktionierung und Abtrennen zumindest eines Rückführstroms, der eine T5-Temperatur (5 Vol.-% aufgefangen, EN ISO 3405-2019) von 270 °C oder höher aufweist und optional C16 n-Paraffine umfasst, aus der Fraktionierung;
ii) Unterziehen eines den Rückführungsstrom umfassenden Einsatzmaterials für Reaktor B, einem Hydroprocessing in einem Reaktor B in Gegenwart eines Hydrotreatment-Katalysators B, um ein Hydroprocessing-Ausströmmaterial B zu erhalten;
iii) Einspeisen des Hydroprocessing-Ausströmmaterials B in die Fraktionierung als Co-Einsatzmaterial mit dem Hydroprocessing-Ausströmmaterial A und Gewinnung von zumindest einer oder mehreren flüssigen Transportkraftstoffkomponente(n) aus der Fraktionierung; und
Überwachen von Parametern, die auf die Deaktivierung des Hydrotreatment-Katalysators A hinweisen, um Werte zu erhalten;
Vergleichen der erhaltenen Werte mit vorbestimmten Werten; und
wenn die erhaltenen Werte die vorbestimmten Werte erreichen, Umschalten von i), ii), iii) auf:
I) Unterziehen eines das Kohlenwasserstoff-Einsatzmaterial umfassenden Einsatzmaterials für Reaktor B, einem Hydroprocessing in dem Reaktor B in Gegenwart des Hydrotreatment-Katalysators B, um ein Hydroprocessing-Ausströmmaterial B zu erhalten, Unterziehen des Hydroprocessing-Ausströmmaterials B einer Fraktionierung und Abtrennen zumindest eines Rückführstroms, der eine T5-Temperatur (5 Vol.-% aufgefangen, EN ISO 3405-2019) von 270 °C oder höher aufweist und optional C16 n-Paraffine umfasst, aus der Fraktionierung;
II) Unterziehen eines den Rückführungsstrom umfassenden Einsatzmaterials für Reaktor A einem Hydroprocessing in dem Reaktor A in Gegenwart des Hydrotreatment-Katalysators A, um ein Hydroprocessing-Ausströmmaterial A zu erhalten;
III) Einspeisen des Hydroprocessing-Ausströmmaterials A in die Fraktionierung als Co-Einsatzmaterial mit dem Hydroprocessing-Ausströmmaterial B und Gewinnung von zumindest einer oder mehreren flüssigen Transportkraftstoffkomponente(n) aus der Fraktionierung.

3. Verfahren nach Anspruch 1 oder 2, wobei das Kohlenwasserstoffeinsatzmaterial zumindest 0,4 Gew.-ppm Stickstoff, ausgedrückt als elementarer Stickstoff (ASTM D4629-17), bezogen auf das Gesamtgewicht des Kohlenwasserstoffeinsatzmaterials umfasst, oder zumindest 0,6 Gew.-ppm oder zumindest 1,0 Gew.-ppm oder sogar zumindest 1,5 Gew.-ppm oder zumindest 2,0 Gew.-ppm Stickstoff und optional höchstens 1,0 Gew.-%, vorzugsweise höchstens 0,8 Gew.-%, noch bevorzugter höchstens 0,5 Gew.-% Sauerstoff, ausgedrückt als elementarer Sauerstoff (ASTM D5622-2017), bezogen auf das Gesamtgewicht des Kohlenwasserstoffeinsatzmaterials umfasst.

4. Verfahren nach einem der vorhergehenden Ansprüche, wobei die Parameter, die auf eine Deaktivierung des Hydrotreatment-Katalysators A hinweisen, zumindest zwei oder mehr der umfassen aus:
a. Gehalt an Stickstoffverunreinigungen in dem Einsatzmaterial für Reaktor A oder in dem Kohlenwasserstoff-Einsatzmaterial und optional Gehalt an zumindest einer oder mehreren zusätzlichen Verunreinigungen, ausgewählt aus S, O, P, Si, Cl, Fe, Alkalimetallen, Erdalkalimetallen und/oder koksbildenden Verbindungen, in dem Einsatzmaterial für Reaktor A oder in dem Kohlenwasserstoff-Einsatzmaterial;
b. Gehalt an NH₃ und optional Gehalt an H₂S in der Gasphase des Hydroprocessing-Ausströmmaterials A;
c. physikalisch-chemische Charakteristik(a) des entgasten Hydroprocessing-Ausströmmaterials A, vorzugsweise zumindest eines oder mehrere aus Trübungspunkt, Gefrierpunkt, Fließpunkt (Pourpoint), KaltFilterverstopfungspunkt, kinematische Viskosität, Dichte und/oder Destillationseigenschaft;
d. Zusammensetzungscharakteristik(a) des entgasten Hydroprocessing-Ausströmmaterials A, vorzugsweise zumindest eines oder mehrereaus Gehalt an Isoparaffinen, Gehalt an C8-C14-Kohlenwasserstoffen, Gehalt an mehrfach verzweigten Isoparaffinen und/oder Gehalt an C1-C4-Kohlenwasserstoffen in dem entgasten Hydroprocessing-Ausströmmaterial A;
e. Ausbeute von zumindest einer oder mehreren der gewonnenen flüssigen Transportkraftstoffkomponente(n) und/oder des abgetrennten Rückführstroms, vorzugsweise Ausbeute einer Flugkraftstoffkomponente;
f. physikalisch-chemische Charakteristik(a) zumindest einer oder mehrerer der gewonnenen flüssigen Transportkraftstoffkomponente(n) und/oder des abgetrennten Rückführstroms, vorzugsweise zumindest eines oder mehrere aus Trübungspunkt, Gefrierpunkt, Fließpunkt, Kaltfilterverstopfungspunkt, kinematische Viskosität, Dichte, Research-Oktanzahl (ROZ), Cetanzahl und/oder Destillationseigenschaften;
g. Zusammensetzungscharakteristik(a) zumindest einer oder mehreren der gewonnenen flüssigen Transportkraftstoffkomponente(n) und/oder des Rückführstroms, vorzugsweise Gehalt an Isoparaffinen und/oder Gehalt an mehrfach verzweigten Isoparaffinen in einer oder mehreren der gewonnenen flüssigen Kraftstoff-Komponente(n) und/oder des abgetrennten Rückführstroms;
h. Temperaturunterschied über den Reaktor A oder über eines oder mehrere der darin befindlichen Katalysatorbetten; und/oder
i. Betriebsbedingung(en) in dem Reaktor A, ausgewählt aus Temperatur, Druck, stündlicher Gewichts-Raum-Geschwindigkeit (WHSV), H₂-Einspeiseverhältnis in den Reaktor A und/oder H₂-Partialdruck am Einlass des Reaktors A.

5. Verfahren nach einem der vorhergehenden Ansprüche, wobei die Hydroprocessings in Reaktor A und in Reaktor B unabhängig voneinander aus zumindest einem oder mehreren aus Hydroisomerisierung, Hydrocracken, Hydrodearomatisierung und/oder Hydropolishing ausgewählt sind, wobei es sich bei dem Hydroprocessing in Reaktor A und in Reaktor B vorzugsweise um Hydroisomerisierung handelt, oder das Hydroprocessing in Reaktor A und Reaktor B Hydrocracken ist, oder das Hydroprocessing in Reaktor A Hydroisomerisierung ist und das Hydroprocessing in Reaktor B Hydrocracken ist, oder das Hydroprocessing in Reaktor A Hydrocracken ist und das Hydroprocessing in Reaktor B Hydroisomerisierung ist; noch bevorzugter ist das Hydroprocessing in Reaktor A und Reaktor B Hydroisomerisierung.

6. Verfahren nach Anspruch 1 oder nach einem der Ansprüche 3 bis 5, wenn sie von Anspruch 1 abhängen, wobei das Umschalten umfasst, während i), ii), iii) ablaufen, das Einspeisen eines allmählich abnehmenden Anteils des Kohlenwasserstoffeinsatzmaterials und eines allmählich zunehmenden Anteils des entgasten Hydroprocessing-Ausströmmaterials B als Teil des Einsatzmaterials für Reaktor A in Reaktor A und gleichzeitig eines allmählich zunehmenden Anteils des Kohlenwasserstoffeinsatzmaterials und eines allmählich abnehmenden Anteils des entgasten Hydroprocessing-Ausströmmaterials A als Teil des Einsatzmaterials für Reaktor B in Reaktor B, bis das Verfahren nach I), II), III) abläuft; oder
Verfahren nach Anspruch 2 oder nach einem der Ansprüche 3 bis 5, wenn sie von Anspruch 2 abhängen, wobei das Umschalten umfasst, während i), ii), iii) ablaufen, das Einspeisen eines allmählich abnehmenden Anteils des Kohlenwasserstoffeinsatzmaterials und eines allmählich zunehmenden Anteils des Rückführungsstroms als Teil des Einsatzmaterials für Reaktor A in Reaktor A und gleichzeitig eines allmählich zunehmenden Anteils des Kohlenwasserstoffeinsatzmaterials und eines allmählich abnehmenden Anteils des Rückführungsstroms als Teil des Einsatzmaterials für Reaktor B in Reaktor B, bis das Verfahren nach I), II, III) abläuft.

7. Verfahren nach einem der vorhergehenden Ansprüche, wobei das Hydroprocessing in Reaktor A und/oder Reaktor B eine Hydroisomerisierung ist, die bei einer Temperatur im Bereich von 200 °C bis 500 °C, vorzugsweise von 230 °C bis 500 °C, noch bevorzugter von 250 °C bis 450 °C, noch bevorzugter von 280 °C bis 400 °C, einem Druck in einem Bereich von 1 MPa bis 10 MPa, vorzugsweise von 2 MPa bis 8 MPa oder von 3 MPa bis 10 MPa, einem H₂-Partialdruck am Einlass des Reaktors in einem Bereich von 1 MPa bis 10 MPa, vorzugsweise von 2 MPa bis 8 MPa, einer stündlichen Gewichts-Raum-Geschwindigkeit in einem Bereich von 0,1 bis 10, vorzugsweise von 0,2 bis 8, besonders bevorzugt von 0,4 bis 6 kg Reaktoreinsatzmaterial pro kg Katalysator pro Stunde, und einem Verhältnis von H₂ zu Reaktoreinsatzmaterial im Bereich von 10 bis 2000, vorzugsweise von 50 bis 1000 Normlitern H₂ pro Liter Reaktoreinsatzmaterial durchgeführt wird; und/oder
wobei das Hydroprocessing in Reaktor A und/oder Reaktor B Hydrocracken ist, das bei einer Temperatur im Bereich von 200 °C bis 450 °C, vorzugsweise von 220 °C bis 430 °C, besonders bevorzugt von 280 °C bis 350 °C, einem Druck im Bereich von 0,4 MPa bis 8 MPa, vorzugsweise von 1 MPa bis 7 MPa, besonders bevorzugt von 2,5 MPa bis 7 MPa, einem H₂-Partialdruck am Einlass des Reaktors im Bereich von 0,4 MPa bis 8 MPa, vorzugsweise von 1 MPa bis 7 MPa, noch bevorzugter von 2,5 MPa bis 7 MPa, eine stündliche Gewichts-Raumgeschwindigkeit in einem Bereich von 0,1 bis 10, vorzugsweise von 0,2 bis 8, noch bevorzugter von 0,4 bis 6, noch bevorzugter von 0,5 bis 1,5 kg Reaktoreinsatzmaterial pro kg Katalysator pro Stunde, und einem Verhältnis von H₂ zu Reaktoreinsatzmaterial in einem Bereich von 10 bis 2000, vorzugsweise von 50 bis 1000 Normalliter H₂ pro Liter Reaktoreinsatzmaterial durchgeführt wird; und/oder
wobei Reaktor A bei einer höheren Temperatur als Reaktor B betrieben wird.

8. Verfahren nach einem der vorhergehenden Ansprüche, wobei der Hydrotreatment-Katalysator A und/oder der Hydrotreatment-Katalysator B unabhängig voneinander ausgewählt ist aus nicht-sulfidierten bifunktionellen Hydrotreatment-Katalysatoren, umfassend zumindest ein oder mehrere Edelmetalle der Gruppe VIII des Periodensystems, besonders bevorzugt Pt und/oder Pd, und zumindest ein oder mehrere saure poröse Materialien, und wobei das Einsatzmaterial für Reaktor A und das Einsatzmaterials für Reaktor B jeweils weniger als 50 Gew.-ppm, vorzugsweise weniger als 30 Gew.-ppm, besonders bevorzugt weniger als 10 Gew.-ppm Schwefel bezogen auf das jeweiligen Gesamteinsatzmaterial (ppm nach Gewicht, berechnet als elementarer S), wie nach ISO 20846-2019 bestimmt, umfasst; und/oder
wobei der Hydrotreatment-Katalysator A und der Hydrotreatment-Katalysator B voneinander verschieden sind.

9. Verfahren nach einem der vorhergehenden Ansprüche, wobei das Hydroprocessing in dem Reaktor A und/oder dem Reaktor B Hydrocracken ist und der (die) jeweilige(n) Hydrotreatment-Katalysator(en) unabhängig voneinander aus bifunktionellen Hydrocracking-Katalysatoren ausgewählt ist (sind), vorzugsweise aus nicht-sulfidierten bifunktionellen Hydrocracking-Katalysatoren, umfassend
zumindest eines oder mehrere Metalle, ausgewählt aus der Gruppe VIII des Periodensystems, Mo, Co und/oder W, vorzugsweise aus Ni, Mo, Co, W, Pt und/oder Pd, besonders bevorzugt aus Pt und/oder Pd; und
zumindest eines oder mehrere saure poröse Materialien ausgewählt aus Zeolithen, zeolith-artigen Materialien und/oder amorphem Siliciumoxid-Aluminiumoxid, wobei vorzugsweise zumindest eines oder mehrere der Zeolithe oder zeolith-artigen Materialien einen Gerüsttyp aufweisen, der ausgewählt ist aus MFI, BEA, FAU, MOR, FER, AEL, AFI, ATO, AFO, MRE, MTT, MTW, TON und/oder MRT, vorzugsweise zumindest eines oder mehrere saure poröse Materialien ausgewählt aus SAPO-5, SAPO-11, SAPO-31, SAPO-41, ZSM-22, ZSM-23, ZSM-43, ZSM-48, IZM-2, Mordenit, Beta-Zeolithen, Zeolithen vom Y-Typ, und/oder amorphem Siliciumoxid-Aluminiumoxid, vorzugsweise zumindest eines oder mehrere saure poröse Materialien ausgewählt aus SAPO-5, SAPO-11, ZSM-23, Beta-Zeolithen, Zeolithen vom Y-Typ und/oder amorphem Siliciumoxid-Aluminiumoxid; und
optional zumindest eines oder mehrere aus Aluminiumoxid, Siliciumoxid, Titanaluminiumoxid, Titandioxid und/oder Zirkoniumoxid; und/oder
wobei das Hydroprocessing in dem Reaktor A und/oder dem Reaktor B eine Hydroisomerisierung ist und der (die) jeweilige(n) Hydrotreatment-Katalysator(en) unabhängig voneinander ausgewählt ist (sind) aus bifunktionellen Hydroisomerisierungskatalysatoren, vorzugsweise aus nicht-sulfidierten bifunktionellen Hydroisomerisierungskatalysatoren, umfassend
zumindest eines oder mehrere Metalle ausgewählt aus der Gruppe VIII des Periodensystems, vorzugsweise aus Edelmetallen der Gruppe VIII, besonders bevorzugt aus Pt und/oder Pd; und
zumindest eines oder mehrere saure poröse Materialien ausgewählt aus Zeolithen und/oder zeolith-artigen Materialien, wobei vorzugsweise zumindest eines oder mehrere der Zeolithe und/oder zeolith-artigen Materialien einen Gerüsttyp aufweisen ausgewählt aus AEL, ATO, AFO, MRE, MTT, MTW, TON, MRT, MOR, FER und/oder MWW, vorzugsweise zumindest eines oder mehrere saure poröse Materialien ausgewählt aus SAPO-11, SAPO-31, SAPO-41, ZSM-22, ZSM-23, ZSM-48, NU-10, ZBM-30, IZM-2, EU-2 und/oder Mordenit, weiter vorzugsweise zumindest eines oder mehrere saure poröse Materialien ausgewählt aus SAPO-11, SAPO-41, ZSM-23 und/oder ZSM-48; und
optional zumindest eines oder mehrere aus Aluminiumoxid, Siliciumoxid, amorphem Siliciumoxid-Aluminiumoxid, Titanaluminiumoxid, Titandioxid und/oder Zirkoniumoxid.

10. Verfahren nach einem der vorhergehenden Ansprüche, wobei das Kohlenwasserstoffeinsatzmaterial zumindest 90 Gew.-%, vorzugsweise zumindest 95 Gew.-%, stärker bevorzugt zumindest 98 Gew.-%, noch stärker bevorzugt zumindest 99 Gew.-% Kohlenwasserstoffe, bezogen auf das Gesamtgewicht des Kohlenwasserstoffeinsatzmaterials, und/oder zumindest 60 Gew.-%, vorzugsweise zumindest 70 Gew.-%, stärker bevorzugt zumindest 80 Gew.-%, noch stärker bevorzugt zumindest 90 Gew.-% Paraffine, bezogen auf das Gesamtgewicht des Kohlenwasserstoffeinsatzmaterials; und/oder höchstens 30 Gew.-%, vorzugsweise höchstens 25 Gew.-%, noch bevorzugter höchstens 20 Gew.-%, noch bevorzugter höchstens 15 Gew.-% Isoparaffine, bezogen auf das Gesamtgewicht des Kohlenwasserstoffeinsatzmaterials; und/oder zumindest 70 Gew.-%, vorzugsweise zumindest 80 Gew.-%, stärker bevorzugt zumindest 90 Gew.-%, noch stärker bevorzugt zumindest 95 Gew.-% C12-C30-Kohlenwasserstoffe, bezogen auf das Gesamtgewicht des Kohlenwasserstoffeinsatzmaterials; und/oder zumindest 70 Gew.-%, vorzugsweise zumindest 80 Gew.-%, stärker bevorzugt zumindest 90 Gew.-%, noch stärker bevorzugt zumindest 95 Gew.-% C14-C22-Kohlenwasserstoffe, bezogen auf das Gesamtgewicht des Kohlenwasserstoffeinsatzmaterial, umfasst; und/oder
wobei der biogene Kohlenstoffgehalt (EN 16640 (2017)) des Kohlenwasserstoffeinsatzmaterials zumindest 50 Gew.-%, vorzugsweise zumindest 70 Gew.-%, noch bevorzugter zumindest 90 Gew.-%, noch bevorzugter zumindest 95 Gew.-% oder etwa 100 Gew.-%, bezogen auf das Gesamtgewicht an Kohlenstoff (TC) in dem Kohlenwasserstoffeinsatzmaterial, beträgt; und/oder
wobei der Schritt der Bereitstellung des Kohlenwasserstoffeinsatzmaterials umfasst
Unterziehen eines sauerstoffhaltigen KohlenwasserstoffEinsatzmaterials einem katalytischen Hydrotreatment, vorzugsweise einer katalytischen Hydrodeoxygenierung, um ein Hydrotreatment-Ausströmmaterial zu erhalten, wobei das sauerstoffhaltige Kohlenwasserstoffeinsatzmaterial vorzugsweise zumindest eines oder mehrere aus pflanzlichen Ölen, tierischen Fetten und/oder mikrobiellen Ölen umfasst, und/oder
Unterziehen eines Synthesegases einer Fischer-Tropsch (FT)-Umwandlung, um ein FT-Ausströmmaterial zu erhalten,
Unterziehen des Hydrotreatment-Ausströmmaterials und/oder des FT-Ausströmmaterials einer Gas-Flüssigkeits-Trennung und optional einer Kohlenwasserstoffeinsatzmaterial-Fraktionierung, um das Kohlenwasserstoffeinsatzmaterial bereitzustellen.

11. Verfahren nach einem der vorhergehenden Ansprüche,
wobei in den Schritten iii) und III) zumindest eines oder mehrere aus einer Flugkraftstoffkomponente, einer Dieselkraftstoffkomponente, einer Ottokraftstoffkomponente und/oder einer Schiffskraftstoffkomponente aus der Fraktionierung gewonnen werden, vorzugsweise zumindest eine Flugkraftstoffkomponente, noch bevorzugter zumindest eine Flugkraftstoffkomponente und eine Dieselkraftstoffkomponente, oder zumindest eine Flugkraftstoffkomponente und eine Ottokraftstoffkomponente; und/oder
wobei in den Schritten iii) und III) zumindest eine Flugkraftstoffkomponente mit einer Dichte bei 15 °C in einem Bereich von 730 kg/m³ bis 772 kg/m³ (EN ISO 12185-1996), einer T10-Temperatur von höchstens 205 °C (EN ISO 3405-2019), einem Siedeendpunkt von höchstens 300 °C (EN ISO 3405-2019), einem Flammpunkt von zumindest 38 °C (IP 170-2013, Abel-Verfahren mit geschlossenem Tiegel) und einem Gefrierpunkt von höchstens -40 °C (IP 529-2016) aus der Fraktionierung gewonnen wird.

12. Verfahren zur Herstellung zumindest einer flüssigen Transportkraftstoffkomponente, wobei das Verfahren umfasst:
i) Bereitstellen eines ein Kohlenwasserstoffeinsatzmaterial umfassenden Einsatzmaterials für Reaktor A, das Stickstoffverunreinigungen enthält, wobei das Einsatzmaterial für Reaktor A zumindest 0,4 Gew.-ppm Stickstoff, ausgedrückt als elementarer Stickstoff (ASTM D4629-17), bezogen auf das Gesamtgewicht des Einsatzmaterials für Reaktor A, oder zumindest 0,6 Gew.-ppm oder zumindest 1,0 Gew.-ppm oder sogar zumindest 1,5 Gew.-ppm oder zumindest 2,0 Gew.-ppm Stickstoff umfasst; ii) Unterziehen des Einsatzmaterials für Reaktor A einem Hydroprocessing in einem Reaktor A in Gegenwart eines Hydrotreatment-Katalysators A, um ein Hydroprocessing-Ausströmmaterial A zu erhalten, und optional Abtrennen zumindest von bei NTP gasförmigen Verbindungen von dem Hydroprocessing-Ausströmmaterial A, um ein entgastes Hydroprocessing-Ausströmmaterial A zu erhalten;
iii) Einspeisen des Hydroprocessing-Ausströmmaterials A oder des entgasten Hydroprocessing-Ausströmmaterials A in eine Fraktionierung und Gewinnen von zumindest einer oder mehreren flüssigen Transportkraftstoffkomponente(n) aus der Fraktionierung; und
Überwachen von Parametern, die auf die Deaktivierung des Hydrotreatment-Katalysators A hinweisen, um Werte zu erhalten;
Vergleichen der erhaltenen Werte mit vorbestimmten Werten; und
wenn die erhaltenen Werte die vorbestimmten Werte erreichen, Umschalten von i), ii), iii) auf:
I) Bereitstellen eines ein Kohlenwasserstoffeinsatzmaterial umfassendes Einsatzmaterials für Reaktor A, wobei das Einsatzmaterial für Reaktor A im Wesentlichen keine Stickstoffverunreinigungen umfasst;
II) Unterziehen des Einsatzmaterials für Reaktor A einem Hydroprocessing in dem Reaktor A in Gegenwart des Hydrotreatment-Katalysators A, um ein Hydroprocessing-Ausströmmaterial A zu erhalten, und optional Abtrennen zumindest von bei NTP gasförmigen Verbindungen von dem Hydroprocessing-Ausströmmaterial A, um ein entgastes Hydroprocessing-Ausströmmaterial A zu erhalten;
III) Einspeisen des Hydroprocessing-Ausströmmaterials A oder des entgasten Hydroprocessing-Ausströmmaterials A eine Fraktionierung und Gewinnen von zumindest einer oder mehreren flüssigen Transportkraftstoffkomponente(n) aus der Fraktionierung.

13. Verfahren nach Anspruch 12, wobei das Einsatzmaterial für Reaktor A in Schritt I) höchstens 0,3 Gew.-ppm, vorzugsweise weniger als 0,3 Gew.-ppm Stickstoff, ausgedrückt als elementarer Stickstoff (ASTM D4629-17), bezogen auf das Gesamtgewicht des Einsatzmaterials für Reaktor A, umfasst, und/oder wobei
das Kohlenwasserstoffeinsatzmaterial in Schritt i) und/oder Schritt I) wie in Anspruch 10 weiter definiert ist; und/oder
der Schritt der Bereitstellung des Kohlenwasserstoffeinsatzmaterials in Schritt i) und/oder Schritt I) wie in Anspruch 10 weiter definiert ist; und/oder
die Parameter, die eine Deaktivierung des Hydrotreatment-Katalysators A anzeigen, wie in Anspruch 4 weiter definiert sind; und/oder
das Hydroprocessing in Reaktor A wie in Anspruch 5 weiter definiert ist; und/oder
das Hydroprocessing in Reaktor A wie in Anspruch 7 weiter definiert durchgeführt wird; und/oder
das Hydroprocessing in Reaktor A und der Hydrotreatment-Katalysator A wie in Anspruch 9 weiter definiert sind; und/oder
die gewonnene(n) flüssige(n) Transportkraftstoffkomponente(n) wie in Anspruch 11 weiter definiert sind.

14. Verfahren nach einem der vorhergehenden Ansprüche, wobei das Verfahren ferner, während I), II), III) ablaufen, das Überwachen von Parametern umfasst, die eine Umkehrung der Deaktivierung des Hydrotreatment-Katalysators A anzeigen, um Werte zu erhalten, wobei die Parameter, die eine Umkehrung der Deaktivierung des Hydrotreatment-Katalysators A anzeigen, vorzugsweise zumindest zwei oder mehr Parameter umfassen ausgewählt aus a. bis i. wie in Anspruch 4 definiert; Vergleichen der erhaltenen Werte mit vorbestimmten Werten; und optional, wenn die erhaltenen Werte die vorbestimmten Werte erreichen, Umschalten von I), II), III) zurück zu i), ii), iii).

15. Computerprogrammprodukt, das Anweisungen umfasst, die, wenn sie von einem Prozessor eines Steuerapparats in einem System zur Herstellung zumindest einer flüssigen Kraftstoffkomponente ausgeführt werden, in einem Verfahren nach einem der Ansprüche 1 bis 14 das Steuergerät veranlassen, die erhaltenen Werte mit vorbestimmten Werten zu vergleichen und, wenn die erhaltenen Werte die vorbestimmten Werte erreichen, von i), ii), iii) auf I), II), III) umzuschalten und optional von I), II), III) zurück auf i), ii), iii) umzuschalten.

## Revendications

1. Procédé de production d'au moins un composant liquide de carburant de transport, le procédé comprenant les étapes de :
fournir une charge d'hydrocarbures comprenant des impuretés azotées ;
i) soumettre une charge de réacteur A comprenant la charge d'hydrocarbures à un hydrotraitement dans un réacteur A en présence d'un catalyseur d'hydrotraitement A pour obtenir un effluent d'hydrotraitement A, et séparer de l'effluent d'hydrotraitement A au moins des composés gazeux à des conditions normales de température et de pression (NTP) pour obtenir un effluent d'hydrotraitement A dégazé ;
ii) soumettre une charge de réacteur B comprenant l'effluent d'hydrotraitement A dégazé à un hydrotraitement dans un réacteur B en présence d'un catalyseur d'hydrotraitement B pour obtenir un effluent d'hydrotraitement B ;
iii) soumettre l'effluent d'hydrotraitement B, éventuellement après avoir séparé de l'effluent d'hydrotraitement B au moins des composés gazeux à des conditions normales de température et de pression (NTP), dans un fractionnement, et récupérer à partir du fractionnement au moins un ou plusieurs composant(s) liquide(s) de carburant de transport ; et
surveiller des paramètres indicatifs d'une désactivation du catalyseur d'hydrotraitement A pour obtenir des valeurs ;
comparer les valeurs obtenues à des valeurs prédéterminées ; et
lorsque les valeurs obtenues atteignent les valeurs prédéterminées, commuter des étapes i), ii), iii) aux étapes de :
I) soumettre une charge de réacteur B comprenant la charge d'hydrocarbures à un hydrotraitement dans le réacteur B en présence du catalyseur d'hydrotraitement B pour obtenir un effluent d'hydrotraitement B, et séparer de l'effluent d'hydrotraitement B au moins les composés gazeux à des conditions normales de température et de pression (NTP) pour obtenir un effluent d'hydrotraitement B dégazé ;
II) soumettre une charge de réacteur A comprenant l'effluent d'hydrotraitement B dégazé à un hydrotraitement dans le réacteur A en présence du catalyseur d'hydrotraitement A pour obtenir un effluent d'hydrotraitement A ;
III) soumettre l'effluent d'hydrotraitement A, éventuellement après avoir séparé de l'effluent d'hydrotraitement A au moins des composés gazeux à des conditions normales de température et de pression (NTP), à un fractionnement, et récupérer à l'issue du fractionnement au moins un ou plusieurs composant(s) liquide(s) de carburant de transport.

2. Procédé de production d'au moins un composant liquide de carburant de transport, le procédé comprenant les étapes de :
fournir une charge d'hydrocarbures comprenant des impuretés azotées ;
i) soumettre une charge de réacteur A comprenant la charge d'hydrocarbures à un hydrotraitement dans un réacteur A en présence d'un catalyseur d'hydrotraitement A pour obtenir un effluent d'hydrotraitement A, soumettre l'effluent d'hydrotraitement A à un fractionnement et séparer du fractionnement au moins un flux de recyclage ayant une température T5 (5 % en volume récupéré, EN ISO 3405-2019) de 270 °C ou plus, et comprenant éventuellement des n-paraffines en C16 ;
ii) soumettre une charge de réacteur B comprenant le courant de recyclage à un hydrotraitement dans un réacteur B en présence d'un catalyseur d'hydrotraitement B pour obtenir un effluent d'hydrotraitement B ;
iii) soumettre l'effluent d'hydrotraitement B au fractionnement en tant que co-charge avec l'effluent d'hydrotraitement A, et récupérer à partir du fractionnement au moins un ou plusieurs composant(s) liquide(s) de carburant de transport ; et
surveiller des paramètres indicatifs d'une désactivation du catalyseur d'hydrotraitement A pour obtenir des valeurs ;
comparer les valeurs obtenues à des valeurs prédéterminées ; et
lorsque les valeurs obtenues atteignent des valeurs prédéterminées, commuter des étapes i), ii), iii) aux étapes de :
I) soumettre une charge de réacteur B comprenant la charge d'hydrocarbures à un hydrotraitement dans le réacteur B en présence du catalyseur d'hydrotraitement B pour obtenir un effluent d'hydrotraitement B, soumettre l'effluent d'hydrotraitement B à un fractionnement et séparer du fractionnement au moins un courant de recyclage ayant une température T5 (5% en volume récupéré, EN ISO 3405-2019) de 270 °C ou plus, et comprenant éventuellement des n-paraffines en C16 ;
II) soumettre une charge de réacteur A comprenant le courant de recyclage à un hydrotraitement dans le réacteur A en présence du catalyseur d'hydrotraitement A pour obtenir un effluent d'hydrotraitement A ;
III) soumettre l'effluent d'hydrotraitement A au fractionnement en tant que co-charge avec l'effluent d'hydrotraitement B, et récupérer du fractionnement au moins un ou plusieurs composant(s) liquide(s) de carburant de transport.

3. Procédé selon la revendication 1 ou 2, dans lequel la charge d'hydrocarbures comprend au moins 0,4 ppm en poids d'azote, exprimé en azote élémentaire (ASTM D4629-17), par rapport au poids total de la charge d'hydrocarbures, ou au moins 0,6 ppm en poids, ou au moins 1,0 ppm en poids, ou encore au moins 1,5 ppm en poids ou au moins 2,0 ppm en poids d'azote, et éventuellement au plus 1,0% en poids, de préférence au plus 0,8% en poids, plus préférablement au plus 0,5% en poids d'oxygène, exprimé en oxygène élémentaire (ASTM D5622-2017), par rapport au poids total de la charge d'hydrocarbures.

4. Procédé selon l'une quelconque des revendications précédentes, dans lequel les paramètres indicatifs d'une désactivation du catalyseur d'hydrotraitement A comprennent au moins deux ou plus des éléments suivants :
a. une teneur en impuretés azotées dans la charge de réacteur A ou dans la charge d'hydrocarbures, et éventuellement une teneur en au moins une ou plus impureté supplémentaire choisie parmi S, O, P, Si, Cl, Fe, les métaux alcalins, les métaux alcalino-terreux et/ou les composés cokéfiables dans la charge de réacteur A ou dans la charge d'hydrocarbures ;
b. une teneur en NH₃ et, éventuellement, une teneur en H₂S dans la phase gazeuse de l'effluent d'hydrotraitement A ;
c. une ou des caractéristique(s) physico-chimique(s) de l'effluent d'hydrotraitement A dégazé, de préférence au moins un ou plusieurs parmi un point de trouble, un point de congélation, un point d'écoulement, un point de colmatage à froid, une viscosité cinématique, une densité et/ou une caractéristique de distillation ;
d. une ou des caractéristique(s) de composition de l'effluent d'hydrotraitement A dégazé, de préférence au moins une ou plus parmi une teneur en isoparaffines, une teneur en hydrocarbures en C8 à C14, une teneur en isoparaffines à ramification multiple et/ou une teneur en hydrocarbures en C1 à C4 dans l'effluent d'hydrotraitement A dégazé ;
e. un rendement d'au moins un ou plus d'un ou de composant(s) liquide(s) de carburant de transport récupérés et/ou du courant de recyclage séparé, de préférence un rendement d'un composant de carburant d'aviation ;
f. une ou des caractéristique(s) physico-chimique(s) d'au moins un ou plus du ou des composant(s) liquide(s) de de carburant transport récupéré(s) et/ou du courant de recyclage séparé, de préférence au moins un ou plusieurs parmi un point de trouble, un point de congélation, un point d'écoulement, un point de colmatage de filtre à froid, une viscosité cinématique, une densité, un indice d'octane recherche (RON), un indice de cétane et/ou une caractéristique de distillation ;
g. une ou des caractéristique(s) de composition d'au moins un ou plus du ou des composant(s) liquides(s) de carburant de transport récupéré(s) et/ou du courant de recyclage, de préférence une teneur en isoparaffines et/ou une teneur en isoparaffines à ramifications multiples dans un ou plus du ou des composant(s) liquide(s) de carburant de transport récupéré(s) et/ou du courant de recyclage séparé ;
h. une différence de température au-dessus du réacteur A, ou au-dessus d'au moins un ou plus lits catalytiques à l'intérieur de celui-ci ; et/ou
i. une ou des condition(s) de fonctionnement dans le réacteur A choisie(s) parmi une température, une pression, une vitesse spatiale horaire en poids (WHSV), un rapport H₂/charge de réacteur A, et/ou une pression partielle de H₂ à l'entrée du réacteur A.

5. Procédé selon l'une quelconque des revendications précédentes, dans lequel les hydrotraitements dans le réacteur A et dans le réacteur B sont choisis, indépendamment l'un de l'autre, parmi au moins un ou plus des traitements suivants : hydroisomérisation, hydrocraquage, d'hydrodésaromatisation et/ou d'hydropolissage, préférablement l'hydrotraitement dans le réacteur A et dans le réacteur B est une hydroisomérisation, ou l'hydrotraitement dans le réacteur A et le réacteur B est un hydrocraquage, ou l'hydrotraitement dans le réacteur A est une hydroisomérisation et le traitement par hydrotraitement dans le réacteur B est un hydrocraquage, ou le traitement par hydrotraitement dans le réacteur A est un hydrocraquage et le traitement par hydrotraitement dans le réacteur B est une hydroisomérisation ; plus préférablement, le traitement par hydrotraitement dans le réacteur A et le réacteur B est une hydroisomérisation.

6. Procédé selon la revendication 1 ou l'une quelconque des revendications 3 à 5 lorsque celles-ci dépendent de la revendication 1, dans lequel la commutation comprend, lors de la mise en œuvre des étapes i), ii), iii), alimenter le réacteur A avec une proportion progressivement décroissante de la charge d'hydrocarbures et une proportion progressivement croissante de l'effluent d'hydrotraitement B dégazé en tant que partie de la charge de réacteur A, et, en même temps, alimenter le réacteur B avec une proportion progressivement croissante de la charge d'hydrocarbures et une proportion progressivement décroissante de l'effluent d'hydrotraitement A dégazé, en tant que partie de la charge de réacteur B, jusqu'à ce que le procédé soit mis en œuvre selon les étapes I), II) et III) ; ou
le procédé selon la revendication 2 ou l'une quelconque des revendications 3 à 5, lorsque celles-ci dépend de la revendication 2, dans lequel la commutation comprend, lors de la mise en œuvre des étapes i), ii) et iii), alimenter le réacteur A avec une proportion progressivement décroissante de la charge d'hydrocarbures et une portion progressivement croissante du courant de recyclage en tant que partie de la charge de réacteur A, et, en même temps, alimenter le réacteur B avec une proportion progressivement croissante de la charge d'hydrocarbures et une portion progressivement décroissante du courant de recyclage en tant que partie de la charge de réacteur B, jusqu'à ce que le procédé soit mis en œuvre selon les étapes I), II) et III).

7. Procédé selon l'une quelconque des revendications précédentes,
dans lequel le traitement par hydrotraitement dans le réacteur A et/ou le réacteur B est une hydroisomérisation effectuée à une température comprise dans une plage allant de 200°C à 500°C, préférablement de 230°C à 500°C, plus préférablement de 250°C à 450°C, et encore plus préférablement de 280°C à 400°C, à une pression comprise dans une plage allant de 1 MPa à 10 MPa, préférablement de 2 MPa à 8 MPa ou de 3 MPa à 10 MPa, à une pression partielle d'H₂ à l'entrée du réacteur dans une plage allant de 1 MPa à 10 MPa, préférablement de 2 MPa à 8 MPa, à une vitesse spatiale horaire en poids comprise dans une plage allant de 0,1 à 10, préférablement de 0,2 à 8, plus préférablement de 0,4 à 6 kg de charge de réacteur par kg de catalyseur par heure, et à un rapport H₂/charge de réacteur compris dans une plage allant de 10 à 2000, de préférence de 50 à 1000 litres normaux de H₂ par litre de charge d réacteur; et/ou
dans lequel le traitement par hydrotraitement dans le réacteur A et/ou le réacteur B est un hydrocraquage effectué à une température comprise dans une plage allant de 200°C à 450°C, préférablement de 220°C à 430°C, plus préférablement de 280°C à 350°C, à une pression comprise dans une plage allant de 0,4 MPa à 8 MPa, préférablement de 1 MPa à 7 MPa, plus préférablement de 2,5 MPa à 7 MPa, à une pression partielle de H₂ à l'entrée du réacteur comprise dans une plage allant de 0,4 MPa à 8 MPa, préférablement de 1 MPa à 7 MPa, plus préférablement de 2,5 MPa à 7 MPa, une vitesse spatiale horaire en poids comprise dans une plage allant de 0,1 à 10, préférablement de 0,2 à 8, plus préférablement de 0,4 à 6, encore plus préférablement de 0,5 à 1,5 kg de charge de réacteur par kg de catalyseur et par heure, et à un rapport H₂/charge de réacteur compris dans une plage allant de 10 à 2 000, préférablement de 50 à 1 000 litres normaux de H₂ par litre de charge de réacteur; et/ou dans lequel le réacteur A fonctionne à une température supérieure à celle du réacteur B.

8. Procédé selon l'une quelconque des revendications précédentes, dans lequel le catalyseur d'hydrotraitement A et/ou le catalyseur d'hydrotraitement B sont choisis, indépendamment l'un de l'autre, parmi des catalyseurs d'hydrotraitement bifonctionnels non sulfurés comprenant au moins un ou plusieurs métaux nobles du groupe VIII du tableau périodique, préférablement Pt et/ou Pd, et au moins un ou plusieurs matériaux poreux acides, et dans lequel la charge de réacteur A et la charge de réacteur B contiennent chacun moins de 50 ppm en poids, préférablement moins de 30 ppm en poids, et plus préférablement moins de 10 ppm en poids de soufre par rapport à la charge totale respective (ppm en poids, calculés en tant que soufre élémentaire), tel que déterminé conformément à la norme ISO 20846-2019 ;
et/ou dans lequel le catalyseur d'hydrotraitement A et le catalyseur d'hydrotraitement B sont différents l'un de l'autre.

9. Procédé selon l'une quelconque des revendications précédentes,
dans lequel l'hydrotraitement dans le réacteur A et/ou le réacteur B est un hydrocraquage, et le ou les catalyseur(s) d'hydrotraitement respectif(s) sont choisis, indépendamment l'un de l'autre, parmi des catalyseurs d'hydrocraquage bifonctionnels, préférablement parmi les catalyseurs d'hydrocraquage bifonctionnels non sulfurés, comprenant
au moins un ou plusieurs métaux choisis parmi le groupe VIII du tableau périodique, Mo, Co et/ou W, préférablement choisis parmi Ni, Mo, Co, W, Pt et/ou Pd, plus préférablement parmi Pt et/ou Pd ; et
au moins un ou plusieurs matériaux poreux acides choisis parmi les zéolites, les matériaux de type zéolite et/ou la silice-alumine amorphe, dans lesquels, préférablement, au moins un ou plusieurs des zéolites ou des matériaux de type zéolite ont un type de structure choisi parmi MFI, BEA, FAU, MOR, FER, AEL, AFI, ATO, AFO, MRE, MTT, MTW, TON et/ou MRT, préférablement au moins un ou plusieurs des matériaux poreux acides choisis parmi le SAPO-5, le SAPO-11, le SAPO-31, le SAPO-41, le ZSM-22, le ZSM-23, le ZSM-43, le ZSM-48, l'IZM-2, la mordénite, les zéolites bêta, les zéolites de type Y et/ou la silice-alumine amorphe, plus préférablement au moins un ou plusieurs matériaux poreux acides choisis parmi le SAPO-5, le SAPO-11, le ZSM-23, les zéolites bêta, les zéolites de type Y et/ou la silice-alumine amorphe ; et
éventuellement au moins un ou plusieurs des composés suivants : alumine, silice, alumine-titane, dioxyde de titane et/ou dioxyde de zirconium ; et/ou
dans lequel l'hydrotraitement dans le réacteur A et/ou le réacteur B est une hydroisomérisation, et le ou les catalyseur(s) d'hydrotraitement respectif(s) sont choisis, indépendamment l'un de l'autre, parmi des catalyseurs d'hydroisomérisation bifonctionnels, préférablement parmi des catalyseurs d'hydroisomérisation bifonctionnels non sulfurés, comprenant
au moins un ou plusieurs métaux choisis parmi le groupe VIII du tableau périodique, préférablement parmi les métaux nobles du groupe VIII, et plus préférablement parmi le Pt et/ou le Pd ; et
au moins un ou plusieurs matériaux poreux acides choisis parmi les zéolites et/ou les matériaux de type zéolite, dans lesquels, préférablement, au moins un ou plusieurs des zéolites et/ou des matériaux de type zéolite présente une structure choisie parmi AEL, ATO, AFO, MRE, MTT, MTW, TON, MRT, MOR, FER et/ou MWW, préférablement au moins un ou plusieurs matériaux poreux acides choisis parmi le SAPO-11, le SAPO-31, le SAPO-41, le ZSM-22, le ZSM-23, le ZSM-48, le NU-10, le ZBM-30, l'IZM-2, l'EU-2, et/ou la mordénite, plus préférablement au moins un ou plusieurs matériaux poreux acides choisis parmi le SAPO-11, le SAPO-41, le ZSM-23 et/ou le ZSM-48 ; et
éventuellement au moins un ou plusieurs des composés suivants : alumine, silice, silice-alumine amorphe, alumine de titane, dioxyde de titane et/ou dioxyde de zirconium.

10. Procédé selon l'une quelconque des revendications précédentes, dans lequel la charge d'hydrocarbures comprend au moins 90% en poids, préférablement au moins 95% en poids, plus préférablement au moins 98% en poids, et encore plus préférablement au moins 99% en poids d'hydrocarbures par rapport au poids total de la charge d'hydrocarbures, et/ou au moins 60% en poids, préférablement au moins 70% en poids, plus préférablement au moins 80% en poids, et encore plus préférablement au moins 90% en poids de paraffines par rapport au poids total de la charge d'hydrocarbures ; et/ou au plus 30% en poids, préférablement au plus 25% en poids, plus préférablement au plus 20% en poids, et encore plus préférablement au plus 15% en poids d'isoparaffines par rapport au poids total des paraffines dans la charge d'hydrocarbures ; et/ou au moins 70% en poids, préférablement au moins 80% en poids, plus préférablement au moins 90% en poids, encore plus préférablement au moins 95% en poids en hydrocarbures en C12 à C30 par rapport au poids total de la charge d'hydrocarbures ; et/ou au moins 70% en poids, préférablement au moins 80% en poids, plus préférablement au moins 90% en poids, et encore plus préférablement au moins 95% en poids d'hydrocarbures en C14 à C22 par rapport au poids total de la charge d'hydrocarbures ; et/ou
dans lequel la teneur en carbone biogénique (EN 16640 (2017)) de la charge d'hydrocarbures est d'au moins 50% en poids, préférablement d'au moins70 % en poids, plus préférablement d'au moins 90% en poids, encore plus préférablement d'au moins 95% en poids, ou environ 100% en poids par rapport au poids total de carbone (TC) dans la charge d'hydrocarbures ; et/ou
dans lequel l'étape de fournir la charge d'hydrocarbures comprend les étapes de
soumettre une charge d'hydrocarbures oxygénés à un hydrotraitement catalytique, préférablement à une hydrodésoxygénation catalytique, pour obtenir un effluent d'hydrotraitement, dans lequel la charge d'hydrocarbures oxygénés comprend préférablement au moins une ou plusieurs des substances suivantes : huiles végétales, graisses animales et/ou huiles microbiennes, et/ou
soumettre un gaz de synthèse à une conversion Fischer-Tropsch (FT) pour obtenir un effluent FT,
soumettre l'effluent d'hydrotraitement et/ou l'effluent FT à une séparation gaz-liquide, et, éventuellement, à un fractionnement de la charge d'hydrocarbures afin d'obtenir la charge d'hydrocarbures.

11. Procédé selon l'une quelconque des revendications précédentes,
dans lequel, aux étapes iii) et III), au moins un ou plusieurs composants parmi un composant de carburant d'aviation, un composant de carburant diesel, un composant de carburant essence et/ou un composant de carburant marin sont récupérés à partir du fractionnement, préférablement au moins un composant de carburant d'aviation, plus préférablement au moins un composant de carburant d'aviation et un composant de carburant diesel, ou au moins un composant de carburant d'aviation et un composant de carburant essence ; et/ou
dans lequel, aux étapes iii) et III), au moins un composant de carburant d'aviation présentant une densité à 15°C comprise and une plage allant de 730 kg/m³ à 772 kg/m³ (EN ISO 12185-1996), une température T10 d'au plus 205°C (EN ISO 3405-2019), un point d'ébullition final d'au plus 300°C (EN ISO 3405-2019), un point d'éclair d'au moins 38°C (IP 170-2013, méthode Abel en coupelle fermée), et point d'au plus -40 °C (IP 529-2016) est récupéré du fractionnement.

12. Procédé de production d'au moins un composant liquide de carburant de transport, le procédé comprenant les étapes de :
i) fournir une charge de réacteur A comprenant une charge d'hydrocarbures comprenant des impuretés azotées, dans lequel la charge de réacteur A contient au moins 0,4 ppm en poids d'azote, exprimé en azote élémentaire (ASTM D4629-17), par rapport au poids total de la charge de réacteur A, ou au moins 0,6 ppm en poids, ou au moins 1,0 ppm en poids, ou encore au moins 1,5 ppm en poids ou au moins 2,0 ppm en poids d'azote ;
ii) soumettre la charge de réacteur A à un hydrotraitement dans un réacteur A en présence d'un catalyseur d'hydrotraitement A pour obtenir un effluent d'hydrotraitement A, et éventuellement séparer de l'effluent d'hydrotraitement A au moins des composés gazeux à des conditions normales de température et de pression (NTP) pour obtenir un effluent d'hydrotraitement A dégazé ;
iii) soumettre l'effluent d'hydrotraitement A ou l'effluent d'hydrotraitement A dégazé à un fractionnement, et récupérer du fractionnement au moins un ou plusieurs composant(s) liquide(s) de carburant de transport ; et
surveiller des paramètres indicatifs de la désactivation du catalyseur d'hydrotraitement A pour obtenir des valeurs ;
comparer les valeurs obtenues à des valeurs prédéterminées ; et
lorsque les valeurs obtenues atteignent les valeurs prédéterminées, commuter des étapes i), ii), iii) aux étapes de :
I) fournir une charge d'alimentation du réacteur A comprenant une charge d'hydrocarbures, dans laquelle la charge d'alimentation du réacteur A ne contient pratiquement pas d'impuretés azotées ;
II) soumettre la charge de réacteur A à un hydrotraitement dans le réacteur A en présence du catalyseur d'hydrotraitement A pour obtenir un effluent d'hydrotraitement A, et éventuellement séparer de l'effluent d'hydrotraitement A au moins des composés gazeux à conditions normales de température et de pression (NTP) pour obtenir un effluent d'hydrotraitement A dégazé ;
III) soumettre l'effluent d'hydrotraitement A ou de l'effluent d'hydrotraitement A dégazé à un fractionnement, et récupérer du fractionnement au moins un ou plusieurs composant(s) liquide(s) de carburant de transport.

13. Procédé selon la revendication 12, dans lequel la charge de réacteur A à l'étape I) comprend au plus 0,3 ppm en poids, préférablement moins de 0,3 ppm en poids d'azote exprimé en azote élémentaire (ASTM D4629-17), par rapport au poids total de la charge de réacteur A,
et/ou dans lequel
la charge d'hydrocarbures de l'étape i) et/ou de l'étape I) est telle que définie plus en détail dans la revendication 10 ; et/ou l'étape de fournir la charge d'hydrocarbures de l'étape i) et/ou de l'étape I) est telle que définie plus en détail dans la revendication 10 ; et/ou
les paramètres indicatifs de la désactivation du catalyseur d'hydrotraitement A sont tels que définis plus en détail définis dans la revendication 4 ; et/ou
l'hydrotraitement dans le réacteur A est tel que défini plus en détail dans la revendication 5 ; et/ou
l'hydrotraitement dans le réacteur A est effectué comme précisé plus en détail dans la revendication 7 ; et/ou
l'hydrotraitement dans le réacteur A et le catalyseur d'hydrotraitement A sont tels que définis plus en détail dans la revendication 9 ; et/ou
le ou les composant(s) liquide(s) de carburant de transport récupéré(s) est/sont tel(s) que défini(s) plus en détail dans la revendication 11.

14. Procédé selon l'une quelconque des revendications précédentes, dans lequel le procédé comprend en outre, lors de l'exécution des étapes I), II) et III), une étape de surveiller des paramètres indicatifs d'une inversion de la désactivation du catalyseur d'hydrotraitement A pour obtenir des valeurs, dans lequel les paramètres indicatifs d'une inversion de la désactivation du catalyseur d'hydrotraitement A comprennent préférablement au moins deux paramètres ou plus choisis parmi a. à i. tels que définis dans la revendication 4 ; une étape de comparer des valeurs obtenues à des valeurs prédéterminées ; et, éventuellement, lorsque les valeurs obtenues atteignent les valeurs prédéterminées, revenir des étapes I), II), III) aux étapes i), ii), iii).

15. Produit logiciel comprenant des instructions qui, lorsqu'elles sont exécutées par un processeur d'un dispositif de commande dans un système destiné à produire au moins un composant liquide de carburant, amène, dans un procédé selon l'une quelconque des revendications 1 à 14, le dispositif de commande à comparer les valeurs obtenues avec des valeurs prédéterminées, et lorsque les valeurs obtenues atteignent les valeurs prédéterminées, à commuter des étapes i), ii), iii) aux étapes I), II), III), et éventuellement à revenir des étapes I), II), III) aux étapes i), ii), iii).
